# EUROPEAN PATENT APPLICATION

(11) **EP 2 327 710 A1**
(43) Date of publication of application: **01.06.2011**
(21) Application number: 11000587.3
(22) Date of filing: 11.02.2005
(51) Int. Cl.: C07H 21/04, A61K 48/00, C12N 15/113

(54) **Anti-microRNA oligonucleotide molecules**

(30) Priority: 13.02.2004 US 778908; 13.05.2004 US 845057
(62) Divisional of application: 05723073.2
(71) Applicant: THE ROCKEFELLER UNIVERSITY, New York NY 11021-6399 (US)
(72) Inventor: Tuschl, Thomas H., New York, NY 10021 (US); Landthaler, Markus, New York, NY 10028 (US); Meister, Gunter, New York, NY 10021 (US); Pfeffer, Sebastian, New York, NY 10044 (US)
(74) Representative: Grünecker, Kinkeldey, Stockmair & Schwanhäusser Anwaltssozietät

(57) **Abstract**

The invention relates to isolated anti-microRNA molecules. In another embodiment, the invention relates to an isolated microRNA molecule. In yet another embodiment, the invention provides a method for inhibiting microRNP activity in a cell.

SEQUENCE LISTING

<110> The Rockefeller University

<120> Anti-MicroRNA Oligonucleotide Molecules

<130> EP44959IHV163pau

<140> not yet assigned
<141> 2005-02-11

<150> EP 05 723 073.2
<151> 2005-02-11

<150> PCT/US05/04714
<151> 2005-02-11

<150> US 10/845,057
<151> 2004-05-13

<150> US 10/778,908
<151> 2004-02-13

<160> 623

<170> PatentIn version 3.1

<210> 1
<211> 22
<212> RNA
<213> Homo sapiens

<400> 1
aacccguaga uccgaacuug ug 22

<210> 2
<211> 22
<212> RNA
<213> Homo sapiens

<400> 2
agcagcauug uacagggcua ug 22

<210> 3
<211> 22
<212> RNA
<213> Homo sapiens

<400> 3
ucaaaugcuc agacuccugu gg 22

<210> 4
<211> 22
<212> RNA
<213> Homo sapiens

<400> 4
aaaagugcuu acagugcagg ua 22

<210> 5
<211> 22
<212> RNA
<213> Homo sapiens

<400> 5
uaaagugcug acagugcaga ua 22

<210> 6
<211> 22
<212> RNA

<213>  Homo sapiens

<400>  6
agcagcauug uacagggcua uc                                              22


<210>  7
<211>  22
<212>  RNA
<213>  Homo sapiens

<400>  7
uacccuguag aaccgaauuu gu                                              22


<210>  8
<211>  22
<212>  RNA
<213>  Homo sapiens

<400>  8
ucacagugaa ccggucucuu uc                                              22


<210>  9
<211>  22
<212>  RNA
<213>  Homo sapiens

<400>  9
cagugcaaug augaaagggc au                                              22


<210>  10
<211>  22
<212>  RNA
<213>  Homo sapiens

<400>  10
uaccacaggg uagaaccacg ga                                              22


<210>  11
<211>  22
<212>  RNA
<213>  Homo sapiens

<400>  11
cccauaaagu agaaagcacu ac                                              22


<210>  12
<211>  22
<212>  RNA
<213>  Homo sapiens

<400>  12
ucgaggagcu cacagucuag ua                                              22


<210>  13
<211>  22
<212>  RNA
<213>  Homo sapiens

<400>  13
uuaaugcuaa ucgugauagg gg                                              22


<210>  14
<211>  22
<212>  RNA
<213>  Homo sapiens

<400>  14

aacauucaac gcugucggug ag                                                22

<210>  15
<211>  22
<212>  RNA
<213>  Homo sapiens

<400>  15
aacauucauu gcugucggug gg                                                22

<210>  16
<211>  22
<212>  RNA
<213>  Homo sapiens

<400>  16
aacauucaac cugucgguga gu                                                22

<210>  17
<211>  22
<212>  RNA
<213>  Homo sapiens

<400>  17
uuuggcaaug guagaacuca ca                                                22

<210>  18
<211>  22
<212>  RNA
<213>  Homo sapiens

<400>  18
uauggcacug guagaauuca cu                                                22

<210>  19
<211>  22
<212>  RNA
<213>  Homo sapiens

<400>  19
uggacggaga acugauaagg gu                                                22

<210>  20
<211>  22
<212>  RNA
<213>  Homo sapiens

<400>  20
uggagagaaa ggcaguuccu ga                                                22

<210>  21
<211>  22
<212>  RNA
<213>  Homo sapiens

<400>  21
caaagaauuc uccuuuuggg cu                                                22

<210>  22
<211>  22
<212>  RNA
<213>  Homo sapiens

<400>  22
ucgugucuug uguugcagcc gg                                                22

<210>  23
<211>  22
<212>  RNA
<213>  Homo sapiens

<400>  23
   cucccacaug caggguuugc ag                                                22


<210>  24
<211>  22
<212>  RNA
<213>  Homo sapiens

<400>  24
caucccuugc augguggagg gu                                               22


<210>  25
<211>  22
<212>  RNA
<213>  Homo sapiens

<400>  25
gugccuacug agcugauauc ag                                               22


<210>  26
<211>  22
<212>  RNA
<213>  Homo sapiens

<400>  26
ugauauguuu gauauauuag gu                                               22


<210>  27
<211>  22
<212>  RNA
<213>  Homo sapiens

<400>  27
caacggaauc ccaaaagcag cu                                               22


<210>  28
<211>  22
<212>  RNA
<213>  Homo sapiens

<400>  28
cugaccuaug aauugacagc ca                                               22


<210>  29
<211>  22
<212>  RNA
<213>  Homo sapiens

<400>  29
   aacuggccua caaaguccca gu                                              22


<210>  30
<211>  22
<212>  RNA
<213>  Homo sapiens

<400>  30
ugggucuuug cgggcaagau ga                                               22


<210>  31
<211>  22
<212>  RNA

<213>  Homo sapiens

<400>  31
uguaacagca acuccaugug ga                                                22


<210>  32
<211>  22
<212>  RNA
<213>  Homo sapiens

<400>  32
uagcagcaca gaaauauugg ca                                                22


<210>  33
<211>  22
<212>  RNA
<213>  Homo sapiens

<400>  33
uagguaguuu cauguuguug gg                                                22


<210>  34
<211>  22
<212>  RNA
<213>  Homo sapiens

<400>  34
uucaccaccu ucuccaccca gc                                                22


<210>  35
<211>  22
<212>  RNA
<213>  Homo sapiens

<400>  35
gguccagagg ggagauaggu uc                                                22


<210>  36
<211>  22
<212>  RNA
<213>  Homo sapiens

<400>  36
acaguagucu gcacauuggu ua                                                22


<210>  37
<211>  22
<212>  RNA
<213>  Homo sapiens

<400>  37
cccaguguuc agacuaccug uu                                                22


<210>  38
<211>  22
<212>  RNA
<213>  Homo sapiens

<400>  38
cccaguguuu agacuaucug uu                                                22


<210>  39
<211>  22
<212>  RNA
<213>  Homo sapiens

<400>  39

uaacacuguc ugguaacgau gu 22

<210> 40
<211> 22
<212> RNA
<213> Homo sapiens

<400> 40
cucuaauacu gccugguaau ga 22

<210> 41
<211> 22
<212> RNA
<213> Homo sapiens

<400> 41
aauacugccg gguaaugaug ga 22

<210> 42
<211> 22
<212> RNA
<213> Homo sapiens

<400> 42
gugaaauguu uaggaccacu ag 22

<210> 43
<211> 22
<212> RNA
<213> Homo sapiens

<400> 43
uucccuuugu cauccuaugc cu 22

<210> 44
<211> 22
<212> RNA
<213> Homo sapiens

<400> 44
uccuucauuc caccggaguc ug 22

<210> 45
<211> 22
<212> RNA
<213> Homo sapiens

<400> 45
uggaauguaa ggaagugugu gg 22

<210> 46
<211> 22
<212> RNA
<213> Homo sapiens

<400> 46
auaagacgag caaaaagcuu gu 22

<210> 47
<211> 22
<212> RNA
<213> Homo sapiens

<400> 47
cugugcgugu gacagcggcu ga 22

<210> 48
<211> 22
<212> RNA
<213> Homo sapiens

<400> 48
uucccuuugu cauccuucgc cu 22


<210> 49
<211> 22
<212> RNA
<213> Homo sapiens

<400> 49
uaacagucuc cagucacggc ca 22


<210> 50
<211> 22
<212> RNA
<213> Homo sapiens

<400> 50
accaucgacc guugauugua cc 22


<210> 51
<211> 22
<212> RNA
<213> Homo sapiens

<400> 51
acagcaggca cagacaggca gu 22


<210> 52
<211> 22
<212> RNA
<213> Homo sapiens

<400> 52
augaccuaug aauugacaga ca 22


<210> 53
<211> 22
<212> RNA
<213> Homo sapiens

<400> 53
uaaucucagc uggcaacugu ga 22


<210> 54
<211> 22
<212> RNA
<213> Homo sapiens

<400> 54
uacugcauca ggaacugauu gg 22


<210> 55
<211> 22
<212> RNA
<213> Homo sapiens

<400> 55
uugugcuuga ucuaaccaug ug 22


<210> 56
<211> 22
<212> RNA

<213>  Homo sapiens

<400>  56
ugauugucca aacgcaauuc uu                                                22


<210>  57
<211>  22
<212>  RNA
<213>  Homo sapiens

<400>  57
ccacaccgua ucugacacuu ug                                                22


<210>  58
<211>  22
<212>  RNA
<213>  Homo sapiens

<400>  58
agcuacauug ucugcugggu uu                                                22


<210>  59
<211>  22
<212>  RNA
<213>  Homo sapiens

<400>  59
agcuacaucu ggcuacuggg uc                                                22


<210>  60
<211>  22
<212>  RNA
<213>  Homo sapiens

<400>  60
ugucaguuug ucaaauaccc ca                                                22


<210>  61
<211>  22
<212>  RNA
<213>  Homo sapiens

<400>  61
caagucacua gugguuccgu uu                                                22


<210>  62
<211>  22
<212>  RNA
<213>  Homo sapiens

<400>  62
aaggagcuca cagucuauug ag                                                22


<210>  63
<211>  22
<212>  RNA
<213>  Homo sapiens

<400>  63
cucaaacugu gggggcacuu uc                                                22


<210>  64
<211>  22
<212>  RNA
<213>  Homo sapiens

<400>  64

agggcccccc cucaauccug uu                                                22

<210>  65
<211>  22
<212>  RNA
<213>  Homo sapiens

<400>  65
ugguuuaccg ucccacauac au                                                22

<210>  66
<211>  22
<212>  RNA
<213>  Homo sapiens

<400>  66
cagugcaaua guauugucaa ag                                                22

<210>  67
<211>  22
<212>  RNA
<213>  Homo sapiens

<400>  67
uaagugcuuc cauguuuugg ug                                                22

<210>  68
<211>  22
<212>  RNA
<213>  Homo sapiens

<400>  68
uguaaacauc cuugacugga ag                                                22

<210>  69
<211>  22
<212>  RNA
<213>  Homo sapiens

<400>  69
aaaagcuggg uugagagggc ga                                                22

<210>  70
<211>  22
<212>  RNA
<213>  Homo sapiens

<400>  70
uaagccaggg auuguggguu cg                                                22

<210>  71
<211>  22
<212>  RNA
<213>  Homo sapiens

<400>  71
aaacaugaau ugcugcugua uc                                                22

<210>  72
<211>  22
<212>  RNA
<213>  Homo sapiens

<400>  72
gcacauuaca cggucgaccu cu                                                22

<210> 73
<211> 22
<212> RNA
<213> Homo sapiens

<400> 73
ccacugcccc aggugcugcu gg 22


<210> 74
<211> 22
<212> RNA
<213> Homo sapiens

<400> 74
   cgcauccccu agggcauugg ug 22


<210> 75
<211> 22
<212> RNA
<213> Homo sapiens

<400> 75
ccucugggcc cuuccuccag cc 22


<210> 76
<211> 22
<212> RNA
<213> Homo sapiens

<400> 76
cuggcccucu cugcccuucc gu 22


<210> 77
<211> 22
<212> RNA
<213> Homo sapiens

<400> 77
aacacaccca gcuaaccuuu uu 22


<210> 78
<211> 22
<212> RNA
<213> Homo sapiens

<400> 78
uggcaguguc uuagcugguu gu 22


<210> 79
<211> 22
<212> RNA
<213> Homo sapiens

<400> 79
aggcaguguc auuagcugau ug 22


<210> 80
<211> 22
<212> RNA
<213> Homo sapiens

<400> 80
aggcagugua guuagcugau ug 22


<210> 81
<211> 22
<212> RNA

<213>  Homo sapiens

<400>  81
uauugcacuu gucccggccu gu                                                22


<210>  82
<211>  22
<212>  RNA
<213>  Homo sapiens

<400>  82
aaagugcugu ucgugcaggu ag                                                22


<210>  83
<211>  22
<212>  RNA
<213>  Homo sapiens

<400>  83
uucaacgggu auuuauugag ca                                                22


<210>  84
<211>  22
<212>  RNA
<213>  Homo sapiens

<400>  84
uuuggcacua gcacauuuuu gc                                                22


<210>  85
<211>  22
<212>  RNA
<213>  Homo sapiens

<400>  85
ugagguagua aguuguauug uu                                                22


<210>  86
<211>  22
<212>  RNA
<213>  Mouse

<400>  86
caaagugcua acagugcagg ua                                                22


<210>  87
<211>  22
<212>  RNA
<213>  Mouse

<400>  87
cccuguagaa ccgaauuugu gu                                                22


<210>  88
<211>  22
<212>  RNA
<213>  Mouse

<400>  88
uauggcuuuu cauuccuaug ug                                                22


<210>  89
<211>  22
<212>  RNA
<213>  Mouse

<400>  89

ucagugcauc acagaacuuu gu 22

<210> 90
<211> 22
<212> RNA
<213> Mouse

<400> 90
cuagacugag gcuccuugag ga 22

<210> 91
<211> 22
<212> RNA
<213> Mouse

<400> 91
uuaaugcuaa uugugauagg gg 22

<210> 92
<211> 22
<212> RNA
<213> Mouse

<400> 92
cccaguguuu agacuaccug uu 22

<210> 93
<211> 22
<212> RNA
<213> Mouse

<400> 93
uaauacugcc ugguaaugau ga 22

<210> 94
<211> 22
<212> RNA
<213> Mouse

<400> 94
ugaaauguuu aggaccacua ga 22

<210> 95
<211> 22
<212> RNA
<213> Mouse

<400> 95
uucccuuugu cauccuuugc cu 22

<210> 96
<211> 22
<212> RNA
<213> Mouse

<400> 96
uacugcauca ggaacugacu gg 22

<210> 97
<211> 22
<212> RNA
<213> Mouse

<400> 97
uaagucacua gugguuccgu uu 22

<210> 98
<211> 22
<212> RNA
<213> Mouse

<400> 98
cacuagauug ugagcugcug ga 22

<210> 99
<211> 22
<212> RNA
<213> Mouse

<400> 99
cucaaacuau gggggcacuu uu 22

<210> 100
<211> 22
<212> RNA
<213> Mouse

<400> 100
aaagugcuuc cacuuugugu gc 22

<210> 101
<211> 22
<212> RNA
<213> Mouse

<400> 101
caucaaagug gaggcccucu cu 22

<210> 102
<211> 22
<212> RNA
<213> Mouse

<400> 102
aagugccgcc agguuuugag ug 22

<210> 103
<211> 22
<212> RNA
<213> Mouse

<400> 103
   acucaaacug ggggcucuuu ug 22

<210> 104
<211> 22
<212> RNA
<213> Mouse

<400> 104
agugccgcag aguuuguagu gu 22

<210> 105
<211> 22
<212> RNA
<213> Mouse

<400> 105
aaagugcuuc ccuuuugugu gu 22

<210> 106
<211> 22
<212> RNA

<213>  Mouse

<400>  106
aaagugcuac uacuuuugag uc                                                22


<210>  107
<211>  22
<212>  RNA
<213>  Mouse

<400>  107
auguaugugu gcaugugcau gu                                                22


<210>  108
<211>  22
<212>  RNA
<213>  Mouse

<400>  108
ggcagaggag ggcuguucuu cc                                                22


<210>  109
<211>  22
<212>  RNA
<213>  Mouse

<400>  109
uaugcaaggg caagcucucu uc                                                22


<210>  110
<211>  22
<212>  RNA
<213>  Mouse

<400>  110
aggcaagaug cuggcauagc ug                                                22


<210>  111
<211>  22
<212>  RNA
<213>  Mouse

<400>  111
aaacaugaag cgcugcaaca cc                                                22


<210>  112
<211>  22
<212>  RNA
<213>  Mouse

<400>  112
ccuaguaggu gcucaguaag ug                                                22


<210>  113
<211>  22
<212>  RNA
<213>  Mouse

<400>  113
ccucugggcc cuuccuccag uc                                                22


<210>  114
<211>  22
<212>  RNA
<213>  Mouse

<400>  114

gcaaagcaca gggccugcag ag                                                22

<210>  115
<211>  22
<212>  RNA
<213>  Mouse

<400>  115
gccccugggc cuauccuaga ac                                                22

<210>  116
<211>  22
<212>  RNA
<213>  Mouse

<400>  116
uucagcuccu auaugaugcc uu                                                22

<210>  117
<211>  22
<212>  RNA
<213>  Mouse

<400>  117
uccagcauca gugauuuugu ug                                                22

<210>  118
<211>  22
<212>  RNA
<213>  Mouse

<400>  118
ucccuguccu ccaggagcuc ac                                                22

<210>  119
<211>  22
<212>  RNA
<213>  Mouse

<400>  119
uccgucucag uuacuuuaua gc                                                22

<210>  120
<211>  22
<212>  RNA
<213>  Mouse

<400>  120
ucgaucgguc ggucggucag uc                                                22

<210>  121
<211>  22
<212>  RNA
<213>  Mouse

<400>  121
ucucacacag aaaucgcacc cg                                                22

<210>  122
<211>  22
<212>  RNA
<213>  Mouse

<400>  122
ugaucuagcc aaagccugac ug                                                22

<210>  123
<211>  22
<212>  RNA
<213>  Mouse

<400>  123
ugcugacccc uaguccagug cu                                                22


<210>  124
<211>  22
<212>  RNA
<213>  Mouse

<400>  124
ugucugcccg agugccugcc uc                                                22


<210>  125
<211>  22
<212>  RNA
<213>  Mouse

<400>  125
uaggcagugu aauuagcuga uu                                                22


<210>  126
<211>  22
<212>  RNA
<213>  Mouse

<400>  126
uucacaaagc ccauacacuu uc                                                22


<210>  127
<211>  22
<212>  RNA
<213>  Mouse

<400>  127
ucccugagga gcccuuugag cc                                                22


<210>  128
<211>  22
<212>  RNA
<213>  Mouse

<400>  128
uggaagacuu gugauuuugu ug                                                22


<210>  129
<211>  22
<212>  RNA
<213>  Mouse

<400>  129
uauugcacuu gucccggccu ga                                                22


<210>  130
<211>  22
<212>  RNA
<213>  Mouse

<400>  130
caaagugcug uucgugcagg ua                                                22


<210>  131
<211>  22
<212>  RNA

<213>  Rat

<400>  131
ccuugagggg caugagggua gu                                              22


<210>  132
<211>  22
<212>  RNA
<213>  Rat

<400>  132
guggugugcu aguuacuuuu gg                                              22


<210>  133
<211>  22
<212>  RNA
<213>  Rat

<400>  133
ucaagagcaa uaacgaaaaa ug                                              22


<210>  134
<211>  22
<212>  RNA
<213>  Rat

<400>  134
ucacccuucc auaucuaguc uc                                              22


<210>  135
<211>  22
<212>  RNA
<213>  Rat

<400>  135
ucucccuccg ugugcccagu au                                              22


<210>  136
<211>  22
<212>  RNA
<213>  Rat

<400>  136
ugucccucug ggucgcccag cu                                              22


<210>  137
<211>  22
<212>  RNA
<213>  Rat

<400>  137
cagcccugcu gucuuaaccu cu                                              22


<210>  138
<211>  22
<212>  RNA
<213>  Rat

<400>  138
agaguaguag guugcauagu ac                                              22


<210>  139
<211>  22
<212>  RNA
<213>  Homo sapiens

<400>  139

uuaucagaau cuccaggggu ac                                                22

<210>  140
<211>  22
<212>  RNA
<213>  Homo sapiens

<400>  140
aauccuugga accuaggugu ga                                                22

<210>  141
<211>  22
<212>  RNA
<213>  Homo sapiens

<400>  141
auugcacggu auccaucugu aa                                                22

<210>  142
<211>  22
<212>  RNA
<213>  Homo sapiens

<400>  142
cggcggggac ggcgauuggu cc                                                22

<210>  143
<211>  22
<212>  RNA
<213>  Homo sapiens

<400>  143
uaaugccccu aaaaauccuu au                                                22

<210>  144
<211>  22
<212>  RNA
<213>  Homo sapiens

<400>  144
uaacugguug aacaacugaa cc                                                22

<210>  145
<211>  22
<212>  RNA
<213>  Caenorhabditis elegans

<400>  145
ugagguagua gguuguauag uu                                                22

<210>  146
<211>  22
<212>  RNA
<213>  Caenorhabditis elegans

<400>  146
ucccugagac cucaagugug ag                                                22

<210>  147
<211>  22
<212>  RNA
<213>  Caenorhabditis elegans

<400>  147
uggaauguaa agaaguaugu ag                                                22

<210>  148
<211>  22
<212>  RNA
<213>  Caenorhabditis elegans

<400>  148
uaucacagcc agcuuugaug ug                                                22


<210>  149
<211>  22
<212>  RNA
<213>  Caenorhabditis elegans

<400>  149
aggcagugug guuagcuggu ug                                                22


<210>  150
<211>  22
<212>  RNA
<213>  Caenorhabditis elegans

<400>  150
ucaccgggug gaaacuagca gu                                                22


<210>  151
<211>  22
<212>  RNA
<213>  Caenorhabditis elegans

<400>  151
ucaccgggug aaaauucgca ug                                                22


<210>  152
<211>  22
<212>  RNA
<213>  Caenorhabditis elegans

<400>  152
ucaccgggug aacacuugca gu                                                22


<210>  153
<211>  22
<212>  RNA
<213>  Caenorhabditis elegans

<400>  153
ucaccgggag aaaaacugga gu                                                22


<210>  154
<211>  22
<212>  RNA
<213>  Caenorhabditis elegans

<400>  154
ucaccgggug uaaaucagcu ug                                                22


<210>  155
<211>  22
<212>  RNA
<213>  Caenorhabditis elegans

<400>  155
ucaccgggug uacaucagcu aa                                                22


<210>  156
<211>  22
<212>  RNA

<213>  Caenorhabditis elegans

<400>  156
ucaccgggug aaaaaucacc ua                                              22


<210>  157
<211>  22
<212>  RNA
<213>  Caenorhabditis elegans

<400>  157
caccggguua acaucuacag ag                                              22


<210>  158
<211>  22
<212>  RNA
<213>  Caenorhabditis elegans

<400>  158
uaucacaguu uacuugcugu cg                                              22


<210>  159
<211>  22
<212>  RNA
<213>  Caenorhabditis elegans

<400>  159
ugacuagaga cacauucagc uu                                              22


<210>  160
<211>  22
<212>  RNA
<213>  Caenorhabditis elegans

<400>  160
ugacuagaga cacauucagc uu                                              22


<210>  161
<211>  22
<212>  RNA
<213>  Caenorhabditis elegans

<400>  161
ugucauggag ucgcucucuu ca                                              22


<210>  162
<211>  22
<212>  RNA
<213>  Caenorhabditis elegans

<400>  162
ugucauggag gcgcucucuu ca                                              22


<210>  163
<211>  22
<212>  RNA
<213>  Caenorhabditis elegans

<400>  163
ugagguaggc ucaguagaug cg                                              22


<210>  164
<211>  22
<212>  RNA
<213>  Caenorhabditis elegans

<400>  164

aagcaccacg agaagcugca ga 22

<210> 165
<211> 22
<212> RNA
<213> Caenorhabditis elegans

<400> 165
ugauaugucu gguauucuug gg 22

<210> 166
<211> 22
<212> RNA
<213> Caenorhabditis elegans

<400> 166
uacccguagc uccuauccau gu 22

<210> 167
<211> 22
<212> RNA
<213> Caenorhabditis elegans

<400> 167
cacccguaca uauguuuccg ug 22

<210> 168
<211> 22
<212> RNA
<213> Caenorhabditis elegans

<400> 168
cacccguaca uuuguuuccg ug 22

<210> 169
<211> 22
<212> RNA
<213> Caenorhabditis elegans

<400> 169
uacccguaau cuucauaauc cg 22

<210> 170
<211> 22
<212> RNA
<213> Caenorhabditis elegans

<400> 170
uacccguaua aguuucugcu ga 22

<210> 171
<211> 22
<212> RNA
<213> Caenorhabditis elegans

<400> 171
uacccguaau guuuccgcug ag 22

<210> 172
<211> 22
<212> RNA
<213> Caenorhabditis elegans

<400> 172
uacccuguag aucgagcugu gu 22

<210>  173
<211>  22
<212>  RNA
<213>  Caenorhabditis elegans

<400>  173
ugagaucguu caguacggca au                                                22


<210>  174
<211>  22
<212>  RNA
<213>  Caenorhabditis elegans

<400>  174
ucgaaucguu uaucaggaug au                                                22


<210>  175
<211>  22
<212>  RNA
<213>  Caenorhabditis elegans

<400>  175
uauuaugcac auuuucuagu uc                                                22


<210>  176
<211>  22
<212>  RNA
<213>  Caenorhabditis elegans

<400>  176
ugacuagaac cguuacucau cu                                                22


<210>  177
<211>  22
<212>  RNA
<213>  Caenorhabditis elegans

<400>  177
ugauauguaa ucuagcuuac ag                                                22


<210>  178
<211>  22
<212>  RNA
<213>  Caenorhabditis elegans

<400>  178
augacacuga agcgaguugg aa                                                22


<210>  179
<211>  22
<212>  RNA
<213>  Caenorhabditis elegans

<400>  179
uaugacacug aagcguuacc ga                                                22


<210>  180
<211>  22
<212>  RNA
<213>  Caenorhabditis elegans

<400>  180
uaugacacug aagcguaacc ga                                                22


<210>  181
<211>  22
<212>  RNA

<213>  Caenorhabditis elegans

<400>  181
caugacacug auuagggaug ug                                              22


<210>  182
<211>  22
<212>  RNA
<213>  Caenorhabditis elegans

<400>  182
ucacaaccuc cuagaaagag ua                                              22


<210>  183
<211>  22
<212>  RNA
<213>  Caenorhabditis elegans

<400>  183
ucgaagacuc aaaaguguag ac                                              22


<210>  184
<211>  22
<212>  RNA
<213>  Caenorhabditis elegans

<400>  184
ucgaaaauua aaaaguguag aa                                              22


<210>  185
<211>  22
<212>  RNA
<213>  Caenorhabditis elegans

<400>  185
uaauacgucg uugguguuuc ca                                              22


<210>  186
<211>  22
<212>  RNA
<213>  Caenorhabditis elegans

<400>  186
ugaaagacau ggguagugaa cg                                              22


<210>  187
<211>  22
<212>  RNA
<213>  Caenorhabditis elegans

<400>  187
aggcaagaug uuggcauagc ug                                              22


<210>  188
<211>  22
<212>  RNA
<213>  Caenorhabditis elegans

<400>  188
uggcaagaug uaggcaguuc ag                                              22


<210>  189
<211>  22
<212>  RNA
<213>  Caenorhabditis elegans

<400>  189

uggcaagaaa uggcagucua ca                                                22

<210>  190
<211>  22
<212>  RNA
<213>  Caenorhabditis elegans

<400>  190
uuaaagcuac caaccggcuu ca                                                22

<210>  191
<211>  22
<212>  RNA
<213>  Caenorhabditis elegans

<400>  191
uucguuguug augaagccuu ga                                                22

<210>  192
<211>  22
<212>  RNA
<213>  Caenorhabditis elegans

<400>  192
uucaucaggc cauagcuguc ca                                                22

<210>  193
<211>  22
<212>  RNA
<213>  Caenorhabditis elegans

<400>  193
uggaggccug guuguuugug cu                                                22

<210>  194
<211>  22
<212>  RNA
<213>  Caenorhabditis elegans

<400>  194
auaaagcuag guuaccaaag cu                                                22

<210>  195
<211>  22
<212>  RNA
<213>  Caenorhabditis elegans

<400>  195
agcuuucgac augauucuga ac                                                22

<210>  196
<211>  22
<212>  RNA
<213>  Caenorhabditis elegans

<400>  196
ugagaucauu aguugaaagc cg                                                22

<210>  197
<211>  22
<212>  RNA
<213>  Caenorhabditis elegans

<400>  197
ugagaucauc gugaaagcua gu                                                22

<210>  198
<211>  22
<212>  RNA
<213>  Caenorhabditis elegans

<400>  198
ugagaucauc gugaaagcca gu                                                22


<210>  199
<211>  22
<212>  RNA
<213>  Caenorhabditis elegans

<400>  199
uagcaccaua uaaauucagu aa                                                22


<210>  200
<211>  22
<212>  RNA
<213>  Caenorhabditis elegans

<400>  200
ugagguagua uguaauauug ua                                                22


<210>  201
<211>  22
<212>  RNA
<213>  Caenorhabditis elegans

<400>  201
uacaaaguau uugaaaaguc gu                                                22


<210>  202
<211>  22
<212>  RNA
<213>  Caenorhabditis elegans

<400>  202
uaagugaaug cuuugccaca gu                                                22


<210>  203
<211>  22
<212>  RNA
<213>  Caenorhabditis elegans

<400>  203
gugagcaaag uuucaggugu gc                                                22


<210>  204
<211>  22
<212>  RNA
<213>  Caenorhabditis elegans

<400>  204
ugauauguug uuugaaugcc cc                                                22


<210>  205
<211>  22
<212>  RNA
<213>  Caenorhabditis elegans

<400>  205
uaaggcacgc ggugaaugcc ac                                                22


<210>  206
<211>  22
<212>  RNA

<213>  Caenorhabditis elegans

<400>  206
aauggcacug caugaauuca cg                                                22


<210>  207
<211>  22
<212>  RNA
<213>  Caenorhabditis elegans

<400>  207
aaugacacug guuaucuuuu cc                                                22


<210>  208
<211>  22
<212>  RNA
<213>  Caenorhabditis elegans

<400>  208
guauuaguug ugcgaccagg ag                                                22


<210>  209
<211>  22
<212>  RNA
<213>  Caenorhabditis elegans

<400>  209
uaagcucgug aucaacaggc ag                                                22


<210>  210
<211>  22
<212>  RNA
<213>  Caenorhabditis elegans

<400>  210
uaaaugcauc uuaacugcgg ug                                                22


<210>  211
<211>  22
<212>  RNA
<213>  Caenorhabditis elegans

<400>  211
uugagcaaug cgcaugugcg gg                                                22


<210>  212
<211>  22
<212>  RNA
<213>  Caenorhabditis elegans

<400>  212
uuauugcucg agaauacccu uu                                                22


<210>  213
<211>  22
<212>  RNA
<213>  Caenorhabditis elegans

<400>  213
uauugcacuc uccccggccu ga                                                22


<210>  214
<211>  22
<212>  RNA
<213>  Caenorhabditis elegans

<400>  214

uaauacuguc agguaaugac gc                                                22

<210>  215
<211>  22
<212>  RNA
<213>  Caenorhabditis elegans

<400>  215
ucccugagaa uucucgaaca gc                                                22

<210>  216
<211>  22
<212>  RNA
<213>  Caenorhabditis elegans

<400>  216
uuuguacucc gaugccauuc ag                                                22

<210>  217
<211>  22
<212>  RNA
<213>  Caenorhabditis elegans

<400>  217
uuuguacuac acauagguac ug                                                22

<210>  218
<211>  22
<212>  RNA
<213>  Caenorhabditis elegans

<400>  218
uuuguacuac acaaaaguac ug                                                22

<210>  219
<211>  22
<212>  RNA
<213>  Caenorhabditis elegans

<400>  219
uacuggcccc caaaucuucg cu                                                22

<210>  220
<211>  22
<212>  RNA
<213>  Caenorhabditis elegans

<400>  220
ugagguaggu gcgagaaaug ac                                                22

<210>  221
<211>  22
<212>  RNA
<213>  Caenorhabditis elegans

<400>  221
uugcguaggc cuuugcuucg ag                                                22

<210>  222
<211>  22
<212>  RNA
<213>  Caenorhabditis elegans

<400>  222
cgguacgauc gcggcgggau au                                                22

<210> 223
<211> 22
<212> RNA
<213> Caenorhabditis elegans

<400> 223
ucuuugguug uacaaagugg ua 22


<210> 224
<211> 22
<212> RNA
<213> Caenorhabditis elegans

<400> 224
auuggucccc uccaaguagc uc 22


<210> 225
<211> 22
<212> RNA
<213> Caenorhabditis elegans

<400> 225
uuacauguuu cggguaggag cu 22


<210> 226
<211> 22
<212> RNA
<213> Caenorhabditis elegans

<400> 226
ugacuagagc cuauucucuu cu 22


<210> 227
<211> 22
<212> RNA
<213> Caenorhabditis elegans

<400> 227
uacacgugca cggauaacgc uc 22


<210> 228
<211> 22
<212> RNA
<213> Caenorhabditis elegans

<400> 228
ucacaggacu uuugagcguu gc 22


<210> 229
<211> 22
<212> RNA
<213> Caenorhabditis elegans

<400> 229
ucacagucaa cuguuggcau gg 22


<210> 230
<211> 22
<212> RNA
<213> Caenorhabditis elegans

<400> 230
uuaaguagug gugccgcucu ua 22


<210> 231
<211> 22
<212> RNA

<213>  Caenorhabditis elegans

<400>  231
uaaguaguag ugccgcaggu aa                                                22


<210>  232
<211>  22
<212>  RNA
<213>  Caenorhabditis elegans

<400>  232
cacaccucac uaacacugac ca                                                22


<210>  233
<211>  22
<212>  RNA
<213>  Caenorhabditis elegans

<400>  233
ugcaaaucuu ucgcgacugu ag                                                22


<210>  234
<211>  22
<212>  RNA
<213>  Caenorhabditis elegans

<400>  234
uggaaugcau agaagacugu ac                                                22


<210>  235
<211>  22
<212>  RNA
<213>  Caenorhabditis elegans

<400>  235
gaguaucagg aguacccagu ga                                                22


<210>  236
<211>  22
<212>  RNA
<213>  Caenorhabditis elegans

<400>  236
gguuuugaga ggaauccuuu ua                                                22


<210>  237
<211>  22
<212>  RNA
<213>  Caenorhabditis elegans

<400>  237
aguaaaucuc auccuaaucu gg                                                22


<210>  238
<211>  22
<212>  RNA
<213>  Caenorhabditis elegans

<400>  238
gugaugucga acucuuguag ga                                                22


<210>  239
<211>  22
<212>  RNA
<213>  Caenorhabditis elegans

<400>  239

uagcuuuuua guuucacgg ug                                                22

<210>  240
<211>  22
<212>  RNA
<213>  Caenorhabditis elegans

<400>  240
   guuucucgau guuuucugau ac                                                22

<210>  241
<211>  22
<212>  RNA
<213>  Caenorhabditis elegans

<400>  241
ggcggguggu uguuguuaug gg                                               22

<210>  242
<211>  22
<212>  RNA
<213>  Caenorhabditis elegans

<400>  242
ugagggagga agggugguau uu                                               22

<210>  243
<211>  22
<212>  RNA
<213>  Caenorhabditis elegans

<400>  243
aggcaagacu uuggcaaagc uu                                               22

<210>  244
<211>  22
<212>  RNA
<213>  Caenorhabditis elegans

<400>  244
cccgugaagu gucugcugca au                                               22

<210>  245
<211>  22
<212>  RNA
<213>  Caenorhabditis elegans

<400>  245
ggcaagaauu agaagcaguu ug                                               22

<210>  246
<211>  22
<212>  RNA
<213>  Caenorhabditis elegans

<400>  246
ggcaagacuc uggcaaaacu ug                                               22

<210>  247
<211>  22
<212>  RNA
<213>  Caenorhabditis elegans

<400>  247
ggcaugaugu agcaguggag au                                               22

<210> 248
<211> 22
<212> RNA
<213> Caenorhabditis elegans

<400> 248
ucgccgggug ggaaagcauu cg 22


<210> 249
<211> 22
<212> RNA
<213> Caenorhabditis elegans

<400> 249
uguaggcaug gguguuugga ag 22


<210> 250
<211> 22
<212> RNA
<213> Caenorhabditis elegans

<400> 250
ugcccguacu gugucggcug cu 22


<210> 251
<211> 22
<212> RNA
<213> Drosophila melanogaster

<400> 251
guuaauggca cuggaagaau uc 22


<210> 252
<211> 22
<212> RNA
<213> Drosophila melanogaster

<400> 252
uggacggaga acugauaagg gc 22


<210> 253
<211> 22
<212> RNA
<213> Drosophila melanogaster

<400> 253
uuuugugacc gacacuaacg gg 22


<210> 254
<211> 22
<212> RNA
<213> Drosophila melanogaster

<400> 254
ucagguaccu gaaguagcgc gc 22


<210> 255
<211> 22
<212> RNA
<213> Drosophila melanogaster

<400> 255
cauugcacuu gucccggccu au 22


<210> 256
<211> 22
<212> RNA

<213>  Drosophila melanogaster

<400>  256
ugauugucca aacgcaauuc uu                                              22


<210>  257
<211>  22
<212>  RNA
<213>  Drosophila melanogaster

<400>  257
uaggaacuuc auaccgugcu cu                                              22


<210>  258
<211>  22
<212>  RNA
<213>  Drosophila melanogaster

<400>  258
uaaaugcacu aucugguacg ac                                              22


<210>  259
<211>  22
<212>  RNA
<213>  Drosophila melanogaster

<400>  259
ucggugggac uuucguccgu uu                                              22


<210>  260
<211>  22
<212>  RNA
<213>  Drosophila melanogaster

<400>  260
   uugguccccu ucaaccagcu gu                                               22


<210>  261
<211>  22
<212>  RNA
<213>  Drosophila melanogaster

<400>  261
ugacuagauc cacacucauu aa                                              22


<210>  262
<211>  22
<212>  RNA
<213>  Drosophila melanogaster

<400>  262
aggugcauug uagucgcauu gu                                              22


<210>  263
<211>  22
<212>  RNA
<213>  Drosophila melanogaster

<400>  263
uguauuuacg uugcauauga aa                                              22


<210>  264
<211>  22
<212>  RNA
<213>  Drosophila melanogaster

<400>  264

ugucauggaa uugcucucuu ug                                                22

<210>  265
<211>  22
<212>  RNA
<213>  Drosophila melanogaster

<400>  265
aaucuagccu cuacuaggcu uu                                                22

<210>  266
<211>  22
<212>  RNA
<213>  Drosophila melanogaster

<400>  266
uaaauaucag cugguaauuc ug                                                22

<210>  267
<211>  22
<212>  RNA
<213>  Drosophila melanogaster

<400>  267
ugaagucagc aacuugauuc ca                                                22

<210>  268
<211>  22
<212>  RNA
<213>  Drosophila melanogaster

<400>  268
uggcagugug guuagcuggu ug                                                22

<210>  269
<211>  22
<212>  RNA
<213>  Drosophila melanogaster

<400>  269
uaaggcacgc ggugaaugcc aa                                                22

<210>  270
<211>  22
<212>  RNA
<213>  Drosophila melanogaster

<400>  270
uaaagcuaga uuaccaaagc au                                                22

<210>  271
<211>  22
<212>  RNA
<213>  Drosophila melanogaster

<400>  271
uaggaacuua auaccgugcu cu                                                22

<210>  272
<211>  22
<212>  RNA
<213>  Drosophila melanogaster

<400>  272
uugugcgugu gacagcggcu au                                                22

<210> 273
<211> 22
<212> RNA
<213> Drosophila melanogaster

<400> 273
uagcaccauu cgaaaucagu gc 22


<210> 274
<211> 22
<212> RNA
<213> Drosophila melanogaster

<400> 274
aacccguaaa uccgaacuug ug 22


<210> 275
<211> 22
<212> RNA
<213> Drosophila melanogaster

<400> 275
aauugcacua gucccggccu gc 22


<210> 276
<211> 22
<212> RNA
<213> Drosophila melanogaster

<400> 276
ugacuagacc gaacacucgu gc 22


<210> 277
<211> 22
<212> RNA
<213> Drosophila melanogaster

<400> 277
uguguugaaa aucguuugca cg 22


<210> 278
<211> 22
<212> RNA
<213> Drosophila melanogaster

<400> 278
uugagcaaaa uuucaggugu gu 22


<210> 279
<211> 22
<212> RNA
<213> Drosophila melanogaster

<400> 279
cuuggcacug ggagaauuca ca 22


<210> 280
<211> 22
<212> RNA
<213> Drosophila melanogaster

<400> 280
uuucaugucg auuucauuuc au 22


<210> 281
<211> 22
<212> RNA

<213>  Drosophila melanogaster

<400>  281
uaaauauuua aguggagccu gc                                                22


<210>  282
<211>  22
<212>  RNA
<213>  Drosophila melanogaster

<400>  282
ugagaucauu uugaaagcug au                                                22


<210>  283
<211>  22
<212>  RNA
<213>  Drosophila melanogaster

<400>  283
uuuagguuuc acaggaaacu gg                                                22


<210>  284
<211>  22
<212>  RNA
<213>  Drosophila melanogaster

<400>  284
uggcaagaug ucggaauagc ug                                                22


<210>  285
<211>  22
<212>  RNA
<213>  Drosophila melanogaster

<400>  285
uaaucucaau uuguaaaugu ga                                                22


<210>  286
<211>  22
<212>  RNA
<213>  Drosophila melanogaster

<400>  286
auuguacuuc aucaggugcu cu                                                22


<210>  287
<211>  22
<212>  RNA
<213>  Drosophila melanogaster

<400>  287
ucuuugguau ucuagcugua ga                                                22


<210>  288
<211>  22
<212>  RNA
<213>  Drosophila melanogaster

<400>  288
ucagguacuu agugacucuc aa                                                22


<210>  289
<211>  22
<212>  RNA
<213>  Drosophila melanogaster

<400>  289

ucuuugguga uuuuagcugu au                                                22

<210>  290
<211>  22
<212>  RNA
<213>  Drosophila melanogaster

<400>  290
ucccugagac ccuaacuugu ga                                                22

<210>  291
<211>  22
<212>  RNA
<213>  Drosophila melanogaster

<400>  291
ucacaaccuc cuugagugag cg                                                22

<210>  292
<211>  22
<212>  RNA
<213>  Drosophila melanogaster

<400>  292
aaucacagga uuauacugug ag                                                22

<210>  293
<211>  22
<212>  RNA
<213>  Drosophila melanogaster

<400>  293
uggcaagaug ucggcauagc ug                                                22

<210>  294
<211>  22
<212>  RNA
<213>  Drosophila melanogaster

<400>  294
gcacugggua aaguuugucc ua                                                22

<210>  295
<211>  22
<212>  RNA
<213>  Drosophila melanogaster

<400>  295
uauugcacac uucccggccu uu                                                22

<210>  296
<211>  22
<212>  RNA
<213>  Drosophila melanogaster

<400>  296
uauugcacau ucaccggccu ga                                                22

<210>  297
<211>  22
<212>  RNA
<213>  Drosophila melanogaster

<400>  297
uauugcacuu gagacggccu ga                                                22

<210>  298
<211>  22
<212>  RNA
<213>  Drosophila melanogaster

<400>  298
uauugcacuu uucacagccc ga                                                22

<210>  299
<211>  21
<212>  RNA
<213>  Drosophila melanogaster

<400>  299
uauucgagcc aauaaguucg g                                                 21

<210>  300
<211>  22
<212>  RNA
<213>  Drosophila melanogaster

<400>  300
uuuugauugu ugcucagaaa gc                                                22

<210>  301
<211>  22
<212>  RNA
<213>  Drosophila melanogaster

<400>  301
ugucuuuuuc cgcuuacugg cg                                                22

<210>  302
<211>  22
<212>  RNA
<213>  Drosophila melanogaster

<400>  302
ugaacacagc uggugguauc ca                                                22

<210>  303
<211>  22
<212>  RNA
<213>  Drosophila melanogaster

<400>  303
ucacugggcu uuguuuaucu ca                                                22

<210>  304
<211>  22
<212>  RNA
<213>  Drosophila melanogaster

<400>  304
uaucacagcc agcuuugaug gg                                                22

<210>  305
<211>  22
<212>  RNA
<213>  Drosophila melanogaster

<400>  305
acguauacug aauguauccu ga                                                22

<210>  306
<211>  22
<212>  RNA

<213>  Drosophila melanogaster

<400>  306
cgguauaccu ucaguauacg ua                                                22


<210>  307
<211>  22
<212>  RNA
<213>  Artificial

<220>
<223>  Anti-microRNA molecule

<400>  307
cacaaguucg gaucuacggg uu                                                22


<210>  308
<211>  22
<212>  RNA
<213>  Artificial

<220>
<223>  Anti-microRNA molecule

<400>  308
cauagcccug uacaaugcug cu                                                22


<210>  309
<211>  22
<212>  RNA
<213>  Artificial

<220>
<223>  anti-microRNA molecule

<400>  309
ccacaggagu cugagcauuu ga                                                22


<210>  310
<211>  22
<212>  RNA
<213>  Artificial

<220>
<223>  anti-microRNA molecule

<400>  310
uaccugcacu guaagcacuu uu                                                22


<210>  311
<211>  22
<212>  RNA
<213>  Artificial

<220>
<223>  anti-microRNA molecule

<400>  311
uaucugcacu gucagcacuu ua                                                22


<210>  312
<211>  22
<212>  RNA
<213>  Artificial

<220>
<223>  anti-microRNA molecule

<400>  312

gauagcccug uacaaugcug cu                                              22

<210>  313
<211>  22
<212>  RNA
<213>  Artificial

<220>
<223>  anti-microRNA molecule

<400>  313
acaaauucgg uucuacaggg ua                                              22

<210>  314
<211>  22
<212>  RNA
<213>  Artificial

<220>
<223>  anti-microRNA molecule

<400>  314
gaaagagacc gguucacugu ga                                              22

<210>  315
<211>  22
<212>  RNA
<213>  Artificial

<220>
<223>  anti-microRNA molecule

<400>  315
augcccuuuc aucauugcac ug                                              22

<210>  316
<211>  22
<212>  RNA
<213>  Artificial

<220>
<223>  anti-microRNA molecule

<400>  316
uccgugguuc uacccugugg ua                                              22

<210>  317
<211>  22
<212>  RNA
<213>  Artificial

<220>
<223>  anti-microRNA molecule

<400>  317
guagugcuuu cuacuuuaug gg                                              22

<210>  318
<211>  22
<212>  RNA
<213>  Artificial

<220>
<223>  anti-microRNA molecule

<400>  318
uacuagacug ugagcuccuc ga                                              22

<210>  319
<211>  22
<212>  RNA
<213>  Artificial

<220>
<223>  anti-microRNA molecule

<400>  319
ccccuaucac gauuagcauu aa                                                22


<210>  320
<211>  22
<212>  RNA
<213>  Artificial

<220>
<223>  anti-microRNA molecule

<400>  320
cucaccgaca gcguugaaug uu                                                22


<210>  321
<211>  22
<212>  RNA
<213>  Artificial

<220>
<223>  anti-microRNA molecule

<400>  321
cccaccgaca gcaaugaaug uu                                                22


<210>  322
<211>  22
<212>  RNA
<213>  Artificial

<220>
<223>  anti-microRNA molecule

<400>  322
acucaccgac agguugaaug uu                                                22


<210>  323
<211>  22
<212>  RNA
<213>  Artificial

<220>
<223>  anti-microRNA molecule

<400>  323
ugugaguucu accauugcca aa                                                22


<210>  324
<211>  22
<212>  RNA
<213>  Artificial

<220>
<223>  anti-microRNA molecule

<400>  324
agugaauucu accagugcca ua                                                22


<210>  325
<211>  22
<212>  RNA

<213>  Artificial

<220>
<223>  anti-microRNA molecule

<400>  325
acccuuauca guucuccguc ca                                                22


<210>  326
<211>  22
<212>  RNA
<213>  Artificial

<220>
<223>  anti-microRNA molecule

<400>  326
ucaggaacug ccuuucucuc ca                                                22


<210>  327
<211>  22
<212>  RNA
<213>  Artificial

<220>
<223>  anti-microRNA molecule

<400>  327
agcccaaaag gagaauucuu ug                                                22


<210>  328
<211>  22
<212>  RNA
<213>  Artificial

<220>
<223>  anti-microRNA molecule

<400>  328
ccggcugcaa cacaagacac ga                                                22


<210>  329
<211>  22
<212>  RNA
<213>  Artificial

<220>
<223>  anti-microRNA molecule

<400>  329
cugcaaaccc ugcauguggg ag                                                22


<210>  330
<211>  22
<212>  RNA
<213>  Artificial

<220>
<223>  anti-microRNA molecule

<400>  330
acccuccacc augcaaggga ug                                                22


<210>  331
<211>  22
<212>  RNA
<213>  Artificial

<220>

<223>  anti-microRNA molecule

<400>  331
cugauaucag cucaguaggc ac                                                22


<210>  332
<211>  22
<212>  RNA
<213>  Artificial

<220>
<223>  anti-microRNA molecule

<400>  332
accuaauaua ucaaacauau ca                                                22


<210>  333
<211>  22
<212>  RNA
<213>  Artificial

<220>
<223>  anti-microRNA molecule

<400>  333
agcugcuuuu gggauuccgu ug                                                22


<210>  334
<211>  22
<212>  RNA
<213>  Artificial

<220>
<223>  anti-microRNA molecule

<400>  334
uggcugucaa uucauagguc ag                                                22


<210>  335
<211>  22
<212>  RNA
<213>  Artificial

<220>
<223>  anti-microRNA molecule

<400>  335
acugggacuu uguaggccag uu                                                22


<210>  336
<211>  22
<212>  RNA
<213>  Artificial

<220>
<223>  anti-microRNA molecule

<400>  336
ucaucuugcc cgcaaagacc ca                                                22


<210>  337
<211>  22
<212>  RNA
<213>  Artificial

<220>
<223>  anti-microRNA molecule

<400>  337

uccacaugga guugcuguua ca                                                22

<210>  338
<211>  22
<212>  RNA
<213>  Artificial

<220>
<223>  anti-microRNA molecule

<400>  338
ugccaauauu ucugugcugc ua                                                22

<210>  339
<211>  22
<212>  RNA
<213>  Artificial

<220>
<223>  anti-microRNA molecule

<400>  339
cccaacaaca ugaaacuacc ua                                                22

<210>  340
<211>  22
<212>  RNA
<213>  Artificial

<220>
<223>  anti-microRNA molecule

<400>  340
gcugggugga gaagguggug aa                                                22

<210>  341
<211>  22
<212>  RNA
<213>  Artificial

<220>
<223>  anti-microRNA molecule

<400>  341
gaaccuaucu ccccucugga cc                                                22

<210>  342
<211>  22
<212>  RNA
<213>  Artificial

<220>
<223>  anti-microRNA molecule

<400>  342
uaaccaaugu gcagacuacu gu                                                22

<210>  343
<211>  22
<212>  RNA
<213>  Artificial

<220>
<223>  anti-microRNA molecule

<400>  343
aacagguagu cugaacacug gg                                                22

<210>  344
<211>  22
<212>  RNA
<213>  Artificial

<220>
<223>  anti-microRNA molecule

<400>  344
aacagauagu cuaaacacug gg                                                22


<210>  345
<211>  22
<212>  RNA
<213>  Artificial

<220>
<223>  anti-microRNA molecule

<400>  345
acaucguuac cagacagugu ua                                                22


<210>  346
<211>  22
<212>  RNA
<213>  Artificial

<220>
<223>  anti-microRNA molecule

<400>  346
ucauuaccag gcaguauuag ag                                                22


<210>  347
<211>  22
<212>  RNA
<213>  Artificial

<220>
<223>  anti-microRNA molecule

<400>  347
uccaucauua cccggcagua uu                                                22


<210>  348
<211>  22
<212>  RNA
<213>  Artificial

<220>
<223>  anti-microRNA molecule

<400>  348
cuaguggucc uaaacauuuc ac                                                22


<210>  349
<211>  22
<212>  RNA
<213>  Artificial

<220>
<223>  anti-microRNA molecule

<400>  349
aggcauagga ugacaaaggg aa                                                22


<210>  350
<211>  22
<212>  RNA

<213>  Artificial

<220>
<223>  anti-microRNA molecule

<400>  350
cagacuccgg uggaaugaag ga                                                22


<210>  351
<211>  22
<212>  RNA
<213>  Artificial

<220>
<223>  anti-microRNA molecule

<400>  351
ccacacacuu ccuuacauuc ca                                                22


<210>  352
<211>  22
<212>  RNA
<213>  Artificial

<220>
<223>  anti-microRNA molecule

<400>  352
acaagcuuuu ugcucgucuu au                                                22


<210>  353
<211>  22
<212>  RNA
<213>  Artificial

<220>
<223>  anti-microRNA molecule

<400>  353
ucagccgcug ucacacgcac ag                                                22


<210>  354
<211>  22
<212>  RNA
<213>  Artificial

<220>
<223>  anti-microRNA molecule

<400>  354
aggcgaagga ugacaaaggg aa                                                22


<210>  355
<211>  22
<212>  RNA
<213>  Artificial

<220>
<223>  anti-microRNA molecule

<400>  355
uggccgugac uggagacugu ua                                                22


<210>  356
<211>  22
<212>  RNA
<213>  Artificial

<220>

<223>  anti-microRNA molecule

<400>  356
gguacaauca acggucgaug gu                                                22


<210>  357
<211>  22
<212>  RNA
<213>  Artificial

<220>
<223>  anti-microRNA molecule

<400>  357
acugccuguc ugugccugcu gu                                                22


<210>  358
<211>  22
<212>  RNA
<213>  Artificial

<220>
<223>  anti-microRNA molecule

<400>  358
ugucugucaa uucauagguc au                                                22


<210>  359
<211>  22
<212>  RNA
<213>  Artificial

<220>
<223>  anti-microRNA molecule

<400>  359
ucacaguugc cagcugagau ua                                                22


<210>  360
<211>  22
<212>  RNA
<213>  Artificial

<220>
<223>  anti-microRNA molecule

<400>  360
ccaaucaguu ccugaugcag ua                                                22


<210>  361
<211>  22
<212>  RNA
<213>  Artificial

<220>
<223>  anti-microRNA molecule

<400>  361
cacaugguua gaucaagcac aa                                                22


<210>  362
<211>  22
<212>  RNA
<213>  Artificial

<220>
<223>  anti-microRNA molecule

<400>  362

aagaauugcg uuuggacaau ca                                              22

<210>  363
<211>  22
<212>  RNA
<213>  Artificial

<220>
<223>  anti-microRNA molecule

<400>  363
caaaguguca gauacggugu gg                                              22

<210>  364
<211>  22
<212>  RNA
<213>  Artificial

<220>
<223>  anti-microRNA molecule

<400>  364
aaacccagca gacaauguag cu                                              22

<210>  365
<211>  22
<212>  RNA
<213>  Artificial

<220>
<223>  anti-microRNA molecule

<400>  365
gacccaguag ccagauguag cu                                              22

<210>  366
<211>  22
<212>  RNA
<213>  Artificial

<220>
<223>  anti-microRNA molecule

<400>  366
ugggguauuu gacaaacuga ca                                              22

<210>  367
<211>  22
<212>  RNA
<213>  Artificial

<220>
<223>  anti-microRNA molecule

<400>  367
aaacggaacc acuagugacu ug                                              22

<210>  368
<211>  22
<212>  RNA
<213>  Artificial

<220>
<223>  anti-microRNA molecule

<400>  368
cucaauagac ugugagcucc uu                                              22

<210> 369
<211> 22
<212> RNA
<213> Artificial

<220>
<223> anti-microRNA molecule

<400> 369
gaaagugccc ccacaguuug ag 22


<210> 370
<211> 22
<212> RNA
<213> Artificial

<220>
<223> anti-microRNA molecule

<400> 370
   aacaggauug agggggggcc cu 22


<210> 371
<211> 22
<212> RNA
<213> Artificial

<220>
<223> anti-microRNA molecule

<400> 371
auguaugugg gacgguaaac ca 22


<210> 372
<211> 22
<212> RNA
<213> Artificial

<220>
<223> anti-microRNA molecule

<400> 372
cuuugacaau acuauugcac ug 22


<210> 373
<211> 22
<212> RNA
<213> Artificial

<220>
<223> anti-microRNA molecule

<400> 373
caccaaaaca uggaagcacu ua 22


<210> 374
<211> 22
<212> RNA
<213> Artificial

<220>
<223> anti-microRNA molecule

<400> 374
cuuccaguca aggauguuua ca 22


<210> 375
<211> 22
<212> RNA

<213>  Artificial

<220>
<223>  anti-microRNA molecule

<400>  375
ucgcccucuc aacccagcuu uu                                              22


<210>  376
<211>  22
<212>  RNA
<213>  Artificial

<220>
<223>  anti-microRNA molecule

<400>  376
cgaacccaca aucccuggcu ua                                              22


<210>  377
<211>  22
<212>  RNA
<213>  Artificial

<220>
<223>  anti-microRNA molecule

<400>  377
gauacagcag caauucaugu uu                                              22


<210>  378
<211>  22
<212>  RNA
<213>  Artificial

<220>
<223>  anti-microRNA molecule

<400>  378
agaggucgac cguguaaugu gc                                              22


<210>  379
<211>  22
<212>  RNA
<213>  Artificial

<220>
<223>  anti-microRNA molecule

<400>  379
ccagcagcac cuggggcagu gg                                              22


<210>  380
<211>  22
<212>  RNA
<213>  Artificial

<220>
<223>  anti-microRNA molecule

<400>  380
caccaaugcc cuaggggaug cg                                              22


<210>  381
<211>  22
<212>  RNA
<213>  Artificial

<220>

<223>  anti-microRNA molecule

<400>  381
ggcuggagga agggcccaga gg                                                22


<210>  382
<211>  22
<212>  RNA
<213>  Artificial

<220>
<223>  anti-microRNA molecule

<400>  382
acggaagggc agagagggcc ag                                                22


<210>  383
<211>  22
<212>  RNA
<213>  Artificial

<220>
<223>  anti-microRNA molecule

<400>  383
aaaaagguua gcugggugug uu                                                22


<210>  384
<211>  22
<212>  RNA
<213>  Artificial

<220>
<223>  anti-microRNA molecule

<400>  384
acaaccagcu aagacacugc ca                                                22


<210>  385
<211>  22
<212>  RNA
<213>  Artificial

<220>
<223>  anti-microRNA molecule

<400>  385
caaucagcua augacacugc cu                                                22


<210>  386
<211>  22
<212>  RNA
<213>  Artificial

<220>
<223>  anti-microRNA molecule

<400>  386
caaucagcua acuacacugc cu                                                22


<210>  387
<211>  22
<212>  RNA
<213>  Artificial

<220>
<223>  anti-microRNA molecule

<400>  387

acaggccggg acaagugcaa ua                                                22

<210>  388
<211>  22
<212>  RNA
<213>  Artificial

<220>
<223>  anti-microRNA molecule

<400>  388
cuaccugcac gaacagcacu uu                                                22

<210>  389
<211>  22
<212>  RNA
<213>  Artificial

<220>
<223>  anti-microRNA molecule

<400>  389
ugcucaauaa auacccguug aa                                                22

<210>  390
<211>  22
<212>  RNA
<213>  Artificial

<220>
<223>  anti-microRNA molecule

<400>  390
gcaaaaaugu gcuagugcca aa                                                22

<210>  391
<211>  22
<212>  RNA
<213>  Artificial

<220>
<223>  anti-microRNA molecule

<400>  391
aacaauacaa cuuacuaccu ca                                                22

<210>  392
<211>  22
<212>  RNA
<213>  Artificial

<220>
<223>  anti-microRNA molecule

<400>  392
uaccugcacu guuagcacuu ug                                                22

<210>  393
<211>  22
<212>  RNA
<213>  Artificial

<220>
<223>  anti-microRNA molecule

<400>  393
acacaaauuc gguucuacag gg                                                22

<210>  394
<211>  22
<212>  RNA
<213>  Artificial

<220>
<223>  anti-microRNA molecule

<400>  394
cacauaggaa ugaaaagcca ua                                                22


<210>  395
<211>  22
<212>  RNA
<213>  Artificial

<220>
<223>  anti-microRNA molecule

<400>  395
acaaaguucu gugaugcacu ga                                                22


<210>  396
<211>  22
<212>  RNA
<213>  Artificial

<220>
<223>  anti-microRNA molecule

<400>  396
uccucaagga gccucagucu ag                                                22


<210>  397
<211>  22
<212>  RNA
<213>  Artificial

<220>
<223>  anti-microRNA molecule

<400>  397
ccccuaucac aauuagcauu aa                                                22


<210>  398
<211>  22
<212>  RNA
<213>  Artificial

<220>
<223>  anti-microRNA molecule

<400>  398
aacagguagu cuaaacacug gg                                                22


<210>  399
<211>  22
<212>  RNA
<213>  Artificial

<220>
<223>  anti-microRNA molecule

<400>  399
ucaucauuac caggcaguau ua                                                22


<210>  400
<211>  22
<212>  RNA

<213>  Artificial

<220>
<223>  anti-microRNA molecule

<400>  400
   ucuagugguc cuaaacauuu ca                                                22

<210>  401
<211>  22
<212>  RNA
<213>  Artificial

<220>
<223>  anti-microRNA molecule

<400>  401
aggcaaagga ugacaaaggg aa                                                22

<210>  402
<211>  22
<212>  RNA
<213>  Artificial

<220>
<223>  anti-microRNA molecule

<400>  402
ccagucaguu ccugaugcag ua                                                22

<210>  403
<211>  22
<212>  RNA
<213>  Artificial

<220>
<223>  anti-microRNA molecule

<400>  403
aaacggaacc acuagugacu ua                                                22

<210>  404
<211>  22
<212>  RNA
<213>  Artificial

<220>
<223>  anti-microRNA molecule

<400>  404
uccagcagcu cacaaucuag ug                                                22

<210>  405
<211>  22
<212>  RNA
<213>  Artificial

<220>
<223>  anti-microRNA molecule

<400>  405
aaaagugccc ccauaguuug ag                                                22

<210>  406
<211>  22
<212>  RNA
<213>  Artificial

<220>

<223>  anti-microRNA molecule

<400>  406
gcacacaaag uggaagcacu uu                                                22


<210>  407
<211>  22
<212>  RNA
<213>  Artificial

<220>
<223>  anti-microRNA molecule

<400>  407
agagagggcc uccacuuuga ug                                                22


<210>  408
<211>  22
<212>  RNA
<213>  Artificial

<220>
<223>  anti-microRNA molecule

<400>  408
cacucaaaac cuggcggcac uu                                                22


<210>  409
<211>  22
<212>  RNA
<213>  Artificial

<220>
<223>  anti-microRNA molecule

<400>  409
caaaagagcc cccaguuuga gu                                                22


<210>  410
<211>  22
<212>  RNA
<213>  Artificial

<220>
<223>  anti-microRNA molecule

<400>  410
acacuacaaa cucugcggca cu                                                22


<210>  411
<211>  22
<212>  RNA
<213>  Artificial

<220>
<223>  anti-microRNA molecule

<400>  411
acacacaaaa gggaagcacu uu                                                22


<210>  412
<211>  22
<212>  RNA
<213>  Artificial

<220>
<223>  anti-microRNA molecule

<400>  412

gacucaaaag uaguagcacu uu                                                22

<210>  413
<211>  22
<212>  RNA
<213>  Artificial

<220>
<223>  anti-microRNA molecule

<400>  413
acaugcacau gcacacauac au                                                22

<210>  414
<211>  22
<212>  RNA
<213>  Artificial

<220>
<223>  anti-microRNA molecule

<400>  414
ggaagaacag cccuccucug cc                                                22

<210>  415
<211>  22
<212>  RNA
<213>  Artificial

<220>
<223>  anti-microRNA molecule

<400>  415
gaagagagcu ugcccuugca ua                                                22

<210>  416
<211>  22
<212>  RNA
<213>  Artificial

<220>
<223>  anti-microRNA molecule

<400>  416
cagcuaugcc agcaucuugc cu                                                22

<210>  417
<211>  22
<212>  RNA
<213>  Artificial

<220>
<223>  anti-microRNA molecule

<400>  417
gguguugcag cgcuucaugu uu                                                22

<210>  418
<211>  22
<212>  RNA
<213>  Artificial

<220>
<223>  anti-microRNA molecule

<400>  418
cacuuacuga gcaccuacua gg                                                22

<210> 419
<211> 22
<212> RNA
<213> Artificial

<220>
<223> anti-microRNA molecule

<400> 419
gacuggagga agggcccaga gg 22


<210> 420
<211> 22
<212> RNA
<213> Artificial

<220>
<223> anti-microRNA molecule

<400> 420
cucugcaggc ccugugcuuu gc 22


<210> 421
<211> 22
<212> RNA
<213> Artificial

<220>
<223> anti-microRNA molecule

<400> 421
guucuaggau aggcccaggg gc 22


<210> 422
<211> 22
<212> RNA
<213> Artificial

<220>
<223> anti-microRNA molecule

<400> 422
aaggcaucau auaggagcug aa 22


<210> 423
<211> 22
<212> RNA
<213> Artificial

<220>
<223> anti-microRNA molecule

<400> 423
caacaaaauc acugaugcug ga 22


<210> 424
<211> 22
<212> RNA
<213> Artificial

<220>
<223> anti-microRNA molecule

<400> 424
gugagcuccu ggaggacagg ga 22


<210> 425
<211> 22
<212> RNA

<213>  Artificial

<220>
<223>  anti-microRNA molecule

<400>  425
gcuauaaagu aacugagacg ga                                                22


<210>  426
<211>  22
<212>  RNA
<213>  Artificial

<220>
<223>  anti-microRNA molecule

<400>  426
gacugaccga ccgaccgauc ga                                                22


<210>  427
<211>  22
<212>  RNA
<213>  Artificial

<220>
<223>  anti-microRNA molecule

<400>  427
cgggugcgau uucuguguga ga                                                22


<210>  428
<211>  22
<212>  RNA
<213>  Artificial

<220>
<223>  anti-microRNA molecule

<400>  428
cagucaggcu uuggcuagau ca                                                22


<210>  429
<211>  22
<212>  RNA
<213>  Artificial

<220>
<223>  anti-microRNA molecule

<400>  429
agcacuggac uaggggucag ca                                                22


<210>  430
<211>  22
<212>  RNA
<213>  Artificial

<220>
<223>  anti-microRNA molecule

<400>  430
gaggcaggca cucgggcaga ca                                                22


<210>  431
<211>  22
<212>  RNA
<213>  Artificial

<220>

<223>  anti-microRNA molecule

<400>  431
aaucagcuaa uuacacugcc ua                                                22


<210>  432
<211>  22
<212>  RNA
<213>  Artificial

<220>
<223>  anti-microRNA molecule

<400>  432
gaaaguguau gggcuuugug aa                                                22


<210>  433
<211>  22
<212>  RNA
<213>  Artificial

<220>
<223>  anti-microRNA molecule

<400>  433
ggcucaaagg gcuccucagg ga                                                22


<210>  434
<211>  22
<212>  RNA
<213>  Artificial

<220>
<223>  anti-microRNA molecule

<400>  434
caacaaaauc acaagucuuc ca                                                22


<210>  435
<211>  22
<212>  RNA
<213>  Artificial

<220>
<223>  anti-microRNA molecule

<400>  435
ucaggccggg acaagugcaa ua                                                22


<210>  436
<211>  22
<212>  RNA
<213>  Artificial

<220>
<223>  anti-microRNA  molecule

<400>  436
uaccugcacg aacagcacuu ug                                                22


<210>  437
<211>  22
<212>  RNA
<213>  Artificial

<220>
<223>  anti-microRNA molecule

<400>  437

acuacccuca ugccccucaa gg                                                22

<210>  438
<211>  22
<212>  RNA
<213>  Artificial

<220>
<223>  anti-microRNA molecule

<400>  438
ccaaaaguaa cuagcacacc ac                                                22

<210>  439
<211>  22
<212>  RNA
<213>  Artificial

<220>
<223>  anti-microRNA molecule

<400>  439
cauuuuucgu uauugcucuu ga                                                22

<210>  440
<211>  22
<212>  RNA
<213>  Artificial

<220>
<223>  anti-microRNA molecule

<400>  440
gagacuagau auggaagggu ga                                                22

<210>  441
<211>  22
<212>  RNA
<213>  Artificial

<220>
<223>  anti-microRNA molecule

<400>  441
auacugggca cacggaggga ga                                                22

<210>  442
<211>  22
<212>  RNA
<213>  Artificial

<220>
<223>  anti-microRNA molecule

<400>  442
agcugggcga cccagaggga ca                                                22

<210>  443
<211>  22
<212>  RNA
<213>  Artificial

<220>
<223>  anti-microRNA molecule

<400>  443
agagguuaag acagcagggc ug                                                22

<210> 444
<211> 22
<212> RNA
<213> Artificial

<220>
<223> anti-microRNA molecule

<400> 444
guacuaugca accuacuacu cu 22

<210> 445
<211> 22
<212> RNA
<213> Artificial

<220>
<223> anti-microRNA molecule

<400> 445
guaccccugg agauucugau aa 22

<210> 446
<211> 22
<212> RNA
<213> Artificial

<220>
<223> anti-microRNA molecule

<400> 446
ucacaccuag guuccaagga uu 22

<210> 447
<211> 22
<212> RNA
<213> Artificial

<220>
<223> anti-microRNA molecule

<400> 447
uuacagaugg auaccgugca au 22

<210> 448
<211> 22
<212> RNA
<213> Artificial

<220>
<223> anti-microRNA molecule

<400> 448
ggaccaaucg ccguccccgc cg 22

<210> 449
<211> 22
<212> RNA
<213> Artificial

<220>
<223> anti-microRNA molecule

<400> 449
auaaggauuu uuaggggcau ua 22

<210> 450
<211> 22
<212> RNA

<213>  Artificial

<220>
<223>  anti-microRNA molecule

<400>  450
gguucaguug uucaaccagu ua                                              22


<210>  451
<211>  22
<212>  RNA
<213>  Artificial

<220>
<223>  anti-microRNA molecule

<400>  451
aacuauacaa ccuacuaccu ca                                              22


<210>  452
<211>  22
<212>  RNA
<213>  Artificial

<220>
<223>  anti-microRNA molecule

<400>  452
cucacacuug aggucucagg ga                                              22


<210>  453
<211>  22
<212>  RNA
<213>  Artificial

<220>
<223>  anti-microRNA molecule

<400>  453
cuacauacuu cuuuacauuc ca                                              22


<210>  454
<211>  22
<212>  RNA
<213>  Artificial

<220>
<223>  anti-microRNA molecule

<400>  454
cacaucaaag cuggcuguga ua                                              22


<210>  455
<211>  22
<212>  RNA
<213>  Artificial

<220>
<223>  anti-microRNA molecule

<400>  455
caaccagcua accacacugc cu                                              22


<210>  456
<211>  22
<212>  RNA
<213>  Artificial

<220>

<223>  anti-microRNA molecule

<400>  456
acugcuaguu uccacccggu ga                                                22


<210>  457
<211>  22
<212>  RNA
<213>  Artificial

<220>
<223>  anti-microRNA molecule

<400>  457
caugcgaauu uucacccggu ga                                                22


<210>  458
<211>  22
<212>  RNA
<213>  Artificial

<220>
<223>  anti-microRNA molecule

<400>  458
acugcaagug uucacccggu ga                                                22


<210>  459
<211>  22
<212>  RNA
<213>  Artificial

<220>
<223>  anti-microRNA molecule

<400>  459
acuccaguuu uucucccggu ga                                                22


<210>  460
<211>  22
<212>  RNA
<213>  Artificial

<220>
<223>  anti-microRNA molecule

<400>  460
caagcugauu uacacccggu ga                                                22


<210>  461
<211>  22
<212>  RNA
<213>  Artificial

<220>
<223>  anti-microRNA molecule

<400>  461
uuagcugaug uacacccggu ga                                                22


<210>  462
<211>  22
<212>  RNA
<213>  Artificial

<220>
<223>  anti-microRNA molecule

<400>  462

uaggugauuu uucacccggu ga                                                22

<210>  463
<211>  22
<212>  RNA
<213>  Artificial

<220>
<223>  anti-microRNA molecule

<400>  463
cucuguagau guuaacccgg ug                                                22

<210>  464
<211>  22
<212>  RNA
<213>  Artificial

<220>
<223>  anti-microRNA molecule

<400>  464
cgacagcaag uaaacuguga ua                                                22

<210>  465
<211>  22
<212>  RNA
<213>  Artificial

<220>
<223>  anti-microRNA molecule

<400>  465
aagcugaaug ugucucuagu ca                                                22

<210>  466
<211>  22
<212>  RNA
<213>  Artificial

<220>
<223>  anti-microRNA molecule

<400>  466
aagcugaaug ugucucuagu ca                                                22

<210>  467
<211>  22
<212>  RNA
<213>  Artificial

<220>
<223>  anti-microRNA molecule

<400>  467
ugaagagagc gacuccauga ca                                                22

<210>  468
<211>  22
<212>  RNA
<213>  Artificial

<220>
<223>  anti-microRNA molecule

<400>  468
ugaagagagc gccuccauga ca                                                22

<210>  469
<211>  22
<212>  RNA
<213>  Artificial

<220>
<223>  anti-microRNA molecule

<400>  469
cgcaucuacu gagccuaccu ca                                                22


<210>  470
<211>  22
<212>  RNA
<213>  Artificial

<220>
<223>  anti-microRNA molecule

<400>  470
ucugcagcuu cucguggugc uu                                                22


<210>  471
<211>  22
<212>  RNA
<213>  Artificial

<220>
<223>  anti-microRNA molecule

<400>  471
cccaagaaua ccagacauau ca                                                22


<210>  472
<211>  22
<212>  RNA
<213>  Artificial

<220>
<223>  anti-microRNA molecule

<400>  472
acauggauag gagcuacggg ua                                                22


<210>  473
<211>  22
<212>  RNA
<213>  Artificial

<220>
<223>  anti-microRNA molecule

<400>  473
cacggaaaca uauguacggg ug                                                22


<210>  474
<211>  22
<212>  RNA
<213>  Artificial

<220>
<223>  anti-microRNA molecule

<400>  474
cacggaaaca aauguacggg ug                                                22


<210>  475
<211>  22
<212>  RNA

<213>  Artificial

<220>
<223>  anti-microRNA molecule

<400>  475
cggauuauga agauuacggg ua                                              22


<210>  476
<211>  22
<212>  RNA
<213>  Artificial

<220>
<223>  anti-microRNA molecule

<400>  476
ucagcagaaa cuuauacggg ua                                              22


<210>  477
<211>  22
<212>  RNA
<213>  Artificial

<220>
<223>  anti-microRNA molecule

<400>  477
cucagcggaa acauuacggg ua                                              22


<210>  478
<211>  22
<212>  RNA
<213>  Artificial

<220>
<223>  anti-microRNA molecule

<400>  478
acacagcucg aucuacaggg ua                                              22


<210>  479
<211>  22
<212>  RNA
<213>  Artificial

<220>
<223>  anti-microRNA molecule

<400>  479
auugccguac ugaacgaucu ca                                              22


<210>  480
<211>  22
<212>  RNA
<213>  Artificial

<220>
<223>  anti-microRNA molecule

<400>  480
aucauccuga uaaacgauuc ga                                              22


<210>  481
<211>  22
<212>  RNA
<213>  Artificial

<220>

<223>  anti-microRNA molecule

<400>  481
gaacuagaaa augugcauaa ua                                                22


<210>  482
<211>  22
<212>  RNA
<213>  Artificial

<220>
<223>  anti-microRNA molecule

<400>  482
agaugaguaa cgguucuagu ca                                                22


<210>  483
<211>  22
<212>  RNA
<213>  Artificial

<220>
<223>  anti-microRNA molecule

<400>  483
cuguaagcua gauuacauau ca                                                22


<210>  484
<211>  22
<212>  RNA
<213>  Artificial

<220>
<223>  anti-microRNA molecule

<400>  484
uuccaacucg cuucaguguc au                                                22


<210>  485
<211>  22
<212>  RNA
<213>  Artificial

<220>
<223>  anti-microRNA molecule

<400>  485
ucgguaacgc uucaguguca ua                                                22


<210>  486
<211>  22
<212>  RNA
<213>  Artificial

<220>
<223>  anti-microRNA molecule

<400>  486
ucgguuacgc uucaguguca ua                                                22


<210>  487
<211>  22
<212>  RNA
<213>  Artificial

<220>
<223>  anti-microRNA molecule

<400>  487

cacaucccua aucaguguca ug                                                22

<210>  488
<211>  22
<212>  RNA
<213>  Artificial

<220>
<223>  anti-microRNA molecule

<400>  488
uacucuuucu aggagguugu ga                                                22

<210>  489
<211>  22
<212>  RNA
<213>  Artificial

<220>
<223>  anti-microRNA molecule

<400>  489
gucuacacuu uugagucuuc ga                                                22

<210>  490
<211>  22
<212>  RNA
<213>  Artificial

<220>
<223>  anti-microRNA molecule

<400>  490
uucuacacuu uuuaauuuuc ga                                                22

<210>  491
<211>  22
<212>  RNA
<213>  Artificial

<220>
<223>  anti-microRNA molecule

<400>  491
uggaaacacc aacgacguau ua                                                22

<210>  492
<211>  22
<212>  RNA
<213>  Artificial

<220>
<223>  anti-microRNA molecule

<400>  492
cguucacuac ccaugucuuu ca                                                22

<210>  493
<211>  22
<212>  RNA
<213>  Artificial

<220>
<223>  anti-microRNA molecule

<400>  493
cagcuaugcc aacaucuugc cu                                                22

<210> 494
<211> 22
<212> RNA
<213> Artificial

<220>
<223> anti-microRNA molecule

<400> 494
cugaacugcc uacaucuugc ca 22

<210> 495
<211> 22
<212> RNA
<213> Artificial

<220>
<223> anti-microRNA molecule

<400> 495
uguagacugc cauuucuugc ca 22

<210> 496
<211> 22
<212> RNA
<213> Artificial

<220>
<223> anti-microRNA molecule

<400> 496
ugaagccggu ugguagcuuu aa 22

<210> 497
<211> 22
<212> RNA
<213> Artificial

<220>
<223> anti-microRNA molecule

<400> 497
ucaaggcuuc aucaacaacg aa 22

<210> 498
<211> 22
<212> RNA
<213> Artificial

<220>
<223> anti-microRNA molecule

<400> 498
uggacagcua uggccugaug aa 22

<210> 499
<211> 22
<212> RNA
<213> Artificial

<220>
<223> anti-microRNA molecule

<400> 499
agcacaaaca accaggccuc ca 22

<210> 500
<211> 22
<212> RNA

<213>  Artificial

<220>
<223>  anti-microRNA molecule

<400>  500
agcuuuggua accuagcuuu au                                                22


<210>  501
<211>  22
<212>  RNA
<213>  Artificial

<220>
<223>  anti-microRNA molecule

<400>  501
guucagaauc augucgaaag cu                                                22


<210>  502
<211>  22
<212>  RNA
<213>  Artificial

<220>
<223>  anti-microRNA molecule

<400>  502
cggcuuucaa cuaaugaucu ca                                                22


<210>  503
<211>  22
<212>  RNA
<213>  Artificial

<220>
<223>  anti-microRNA molecule

<400>  503
acuagcuuuc acgaugaucu ca                                                22


<210>  504
<211>  22
<212>  RNA
<213>  Artificial

<220>
<223>  anti-microRNA molecule

<400>  504
acuggcuuuc acgaugaucu ca                                                22


<210>  505
<211>  22
<212>  RNA
<213>  Artificial

<220>
<223>  anti-microRNA molecule

<400>  505
uuacugaauu uauauggugc ua                                                22


<210>  506
<211>  22
<212>  RNA
<213>  Artificial

<220>

<223>  anti-microRNA molecule

<400>  506
uacaauauua cauacuaccu ca                                                22


<210>  507
<211>  22
<212>  RNA
<213>  Artificial

<220>
<223>  anti-microRNA molecule

<400>  507
acgacuuuuc aaauacuuug ua                                                22


<210>  508
<211>  22
<212>  RNA
<213>  Artificial

<220>
<223>  anti-microRNA molecule

<400>  508
acuguggcaa agcauucacu ua                                                22


<210>  509
<211>  22
<212>  RNA
<213>  Artificial

<220>
<223>  anti-microRNA molecule

<400>  509
gcacaccuga aacuuugcuc ac                                                22


<210>  510
<211>  22
<212>  RNA
<213>  Artificial

<220>
<223>  anti-microRNA molecule

<400>  510
ggggcauuca aacaacauau ca                                                22


<210>  511
<211>  22
<212>  RNA
<213>  Artificial

<220>
<223>  anti-microRNA molecule

<400>  511
guggcauuca ccgcgugccu ua                                                22


<210>  512
<211>  22
<212>  RNA
<213>  Artificial

<220>
<223>  anti-microRNA molecule

<400>  512

cgugaauuca ugcagugcca uu                                                22

<210>  513
<211>  22
<212>  RNA
<213>  Artificial

<220>
<223>  anti-microRNA molecule

<400>  513
ggaaaagaua accaguguca uu                                                22

<210>  514
<211>  22
<212>  RNA
<213>  Artificial

<220>
<223>  anti-microRNA molecule

<400>  514
cuccuggucg cacaacuaau ac                                                22

<210>  515
<211>  22
<212>  RNA
<213>  Artificial

<220>
<223>  anti-microRNA molecule

<400>  515
cugccuguug aucacgagcu ua                                                22

<210>  516
<211>  22
<212>  RNA
<213>  Artificial

<220>
<223>  anti-microRNA molecule

<400>  516
caccgcaguu aagaugcauu ua                                                22

<210>  517
<211>  22
<212>  RNA
<213>  Artificial

<220>
<223>  anti-microRNA molecule

<400>  517
cccgcacaug cgcauugcuc aa                                                22

<210>  518
<211>  22
<212>  RNA
<213>  Artificial

<220>
<223>  anti-microRNA molecule

<400>  518
aaaggguauu cucgagcaau aa                                                22

<210> 519
<211> 22
<212> RNA
<213> Artificial

<220>
<223> anti-microRNA molecule

<400> 519
ucaggccggg gagagugcaa ua 22


<210> 520
<211> 22
<212> RNA
<213> Artificial

<220>
<223> anti-microRNA molecule

<400> 520
gcgucauuac cugacaguau ua 22


<210> 521
<211> 22
<212> RNA
<213> Artificial

<220>
<223> anti-microRNA molecule

<400> 521
gcuguucgag aauucucagg ga 22


<210> 522
<211> 22
<212> RNA
<213> Artificial

<220>
<223> anti-microRNA molecule

<400> 522
cugaauggca ucggaguaca aa 22


<210> 523
<211> 22
<212> RNA
<213> Artificial

<220>
<223> anti-microRNA molecule

<400> 523
caguaccuau guguaguaca aa 22


<210> 524
<211> 22
<212> RNA
<213> Artificial

<220>
<223> anti-microRNA molecule

<400> 524
caguacuuuu guguaguaca aa 22


<210> 525
<211> 22
<212> RNA

<213>  Artificial

<220>
<223>  anti-microRNA molecule

<400>  525
agcgaagauu ugggggccag ua                                              22


<210>  526
<211>  22
<212>  RNA
<213>  Artificial

<220>
<223>  anti-microRNA molecule

<400>  526
gucauuucuc gcaccuaccu ca                                              22


<210>  527
<211>  22
<212>  RNA
<213>  Artificial

<220>
<223>  anti-microRNA molecule

<400>  527
cucgaagcaa aggccuacgc aa                                              22


<210>  528
<211>  22
<212>  RNA
<213>  Artificial

<220>
<223>  anti-microRNA molecule

<400>  528
auaucccgcc gcgaucguac cg                                              22


<210>  529
<211>  22
<212>  RNA
<213>  Artificial

<220>
<223>  anti-microRNA molecule

<400>  529
uaccacuuug uacaaccaaa ga                                              22


<210>  530
<211>  22
<212>  RNA
<213>  Artificial

<220>
<223>  anti-microRNA molecule

<400>  530
gagcuacuug gaggggacca au                                              22


<210>  531
<211>  22
<212>  RNA
<213>  Artificial

<220>

<223>  anti-microRNA molecule

<400>  531
agcuccuacc cgaaacaugu aa                                                22


<210>  532
<211>  22
<212>  RNA
<213>  Artificial

<220>
<223>  anti-microRNA molecule

<400>  532
agaagagaau aggcucuagu ca                                                22


<210>  533
<211>  22
<212>  RNA
<213>  Artificial

<220>
<223>  anti-microRNA molecule

<400>  533
gagcguuauc cgugcacgug ua                                                22


<210>  534
<211>  22
<212>  RNA
<213>  Artificial

<220>
<223>  anti-microRNA molecule

<400>  534
gcaacgcuca aaaguccugu ga                                                22


<210>  535
<211>  22
<212>  RNA
<213>  Artificial

<220>
<223>  anti-microRNA molecule

<400>  535
ccaugccaac aguugacugu ga                                                22


<210>  536
<211>  22
<212>  RNA
<213>  Artificial

<220>
<223>  anti-microRNA molecule

<400>  536
uaagagcggc accacuacuu aa                                                22


<210>  537
<211>  22
<212>  RNA
<213>  Artificial

<220>
<223>  anti-microRNA molecule

<400>  537

uuaccugcgg cacuacuacu ua                                                22

<210>  538
<211>  22
<212>  RNA
<213>  Artificial

<220>
<223>  anti-microRNA molecule

<400>  538
uggucagugu uagugaggug ug                                                22

<210>  539
<211>  22
<212>  RNA
<213>  Artificial

<220>
<223>  anti-microRNA molecule

<400>  539
cuacagucgc gaaagauuug ca                                                22

<210>  540
<211>  22
<212>  RNA
<213>  Artificial

<220>
<223>  anti-microRNA molecule

<400>  540
guacagucuu cuaugcauuc ca                                                22

<210>  541
<211>  22
<212>  RNA
<213>  Artificial

<220>
<223>  anti-microRNA molecule

<400>  541
ucacugggua cuccugauac uc                                                22

<210>  542
<211>  22
<212>  RNA
<213>  Artificial

<220>
<223>  anti-microRNA molecule

<400>  542
uaaaaggauu ccucucaaaa cc                                                22

<210>  543
<211>  22
<212>  RNA
<213>  Artificial

<220>
<223>  anti-microRNA molecule

<400>  543
ccagauuagg augagauuua cu                                                22

<210> 544
<211> 22
<212> RNA
<213> Artificial

<220>
<223> anti-microRNA molecule

<400> 544
uccuacaaga guucgacauc ac 22


<210> 545
<211> 22
<212> RNA
<213> Artificial

<220>
<223> anti-microRNA molecule

<400> 545
caccgugaaa acuaaaaagc ua 22


<210> 546
<211> 22
<212> RNA
<213> Artificial

<220>
<223> anti-microRNA molecule

<400> 546
guaucagaaa acaucgagaa ac 22


<210> 547
<211> 22
<212> RNA
<213> Artificial

<220>
<223> anti-microRNA molecule

<400> 547
cccauaacaa caaccacccg cc 22


<210> 548
<211> 22
<212> RNA
<213> Artificial

<220>
<223> anti-microRNA molecule

<400> 548
aaauaccacc cuuccucccu ca 22


<210> 549
<211> 22
<212> RNA
<213> Artificial

<220>
<223> anti-microRNA molecule

<400> 549
aagcuuugcc aaagucuugc cu 22


<210> 550
<211> 22
<212> RNA

<213>  Artificial

<220>
<223>  anti-microRNA molecule

<400>  550
auugcagcag acacuucacg gg                                                22


<210>  551
<211>  22
<212>  RNA
<213>  Artificial

<220>
<223>  anti-microRNA molecule

<400>  551
caaacugcuu cuaauucuug cc                                                22


<210>  552
<211>  22
<212>  RNA
<213>  Artificial

<220>
<223>  anti-microRNA molecule

<400>  552
caaguuuugc cagagucuug cc                                                22


<210>  553
<211>  22
<212>  RNA
<213>  Artificial

<220>
<223>  anti-microRNA molecule

<400>  553
aucuccacug cuacaucaug cc                                                22


<210>  554
<211>  22
<212>  RNA
<213>  Artificial

<220>
<223>  anti-microRNA molecule

<400>  554
cgaaugcuuu cccacccggc ga                                                22


<210>  555
<211>  22
<212>  RNA
<213>  Artificial

<220>
<223>  anti-microRNA molecule

<400>  555
cuuccaaaca cccaugccua ca                                                22


<210>  556
<211>  22
<212>  RNA
<213>  Artificial

<220>

<223>  anti-microRNA molecule

<400>  556
agcagccgac acaguacggg ca                                                22


<210>  557
<211>  22
<212>  RNA
<213>  Artificial

<220>
<223>  anti-microRNA molecule

<400>  557
gaauucuucc agugccauua ac                                                22


<210>  558
<211>  22
<212>  RNA
<213>  Artificial

<220>
<223>  anti-microRNA molecule

<400>  558
gcccuuauca guucuccguc ca                                                22


<210>  559
<211>  22
<212>  RNA
<213>  Artificial

<220>
<223>  anti-microRNA molecule

<400>  559
cccguuagug ucggucacaa aa                                                22


<210>  560
<211>  22
<212>  RNA
<213>  Artificial

<220>
<223>  anti-microRNA molecule

<400>  560
gcgcgcuacu ucagguaccu ga                                                22


<210>  561
<211>  22
<212>  RNA
<213>  Artificial

<220>
<223>  anti-microRNA molecule

<400>  561
auaggccggg acaagugcaa ug                                                22


<210>  562
<211>  22
<212>  RNA
<213>  Artificial

<220>
<223>  anti-microRNA molecule

<400>  562

aagaauugcg uuuggacaau ca 22

<210> 563
<211> 22
<212> RNA
<213> Artificial

<220>
<223> anti-microRNA molecule

<400> 563
agagcacggu augaaguucc ua 22

<210> 564
<211> 22
<212> RNA
<213> Artificial

<220>
<223> anti-microRNA molecule

<400> 564
gucguaccag auagugcauu ua 22

<210> 565
<211> 22
<212> RNA
<213> Artificial

<220>
<223> anti-microRNA molecule

<400> 565
aaacggacga aagucccacc ga 22

<210> 566
<211> 22
<212> RNA
<213> Artificial

<220>
<223> anti-microRNA molecule

<400> 566
acagcugguu gaaggggacc aa 22

<210> 567
<211> 22
<212> RNA
<213> Artificial

<220>
<223> anti-microRNA molecule

<400> 567
uuaaugagug uggaucuagu ca 22

<210> 568
<211> 22
<212> RNA
<213> Artificial

<220>
<223> anti-microRNA molecule

<400> 568
acaaugcgac uacaaugcac cu 22

<210> 569
<211> 22
<212> RNA
<213> Artificial

<220>
<223> anti-microRNA molecule

<400> 569
uuucauaugc aacguaaaua ca 22

<210> 570
<211> 22
<212> RNA
<213> Artificial

<220>
<223> anti-microRNA molecule

<400> 570
caaagagagc aauuccauga ca 22

<210> 571
<211> 22
<212> RNA
<213> Artificial

<220>
<223> anti-microRNA molecule

<400> 571
aaagccuagu agaggcuaga uu 22

<210> 572
<211> 22
<212> RNA
<213> Artificial

<220>
<223> anti-microRNA molecule

<400> 572
cagaauuacc agcugauauu ua 22

<210> 573
<211> 22
<212> RNA
<213> Artificial

<220>
<223> anti-microRNA molecule

<400> 573
uggaaucaag uugcugacuu ca 22

<210> 574
<211> 22
<212> RNA
<213> Artificial

<220>
<223> anti-microRNA molecule

<400> 574
caaccagcua accacacugc ca 22

<210> 575
<211> 22
<212> RNA

<213>  Artificial

<220>
<223>  anti-microRNA molecule

<400>  575
uuggcauuca ccgcgugccu ua                                              22


<210>  576
<211>  22
<212>  RNA
<213>  Artificial

<220>
<223>  anti-microRNA molecule

<400>  576
augcuuuggu aaucuagcuu ua                                              22


<210>  577
<211>  22
<212>  RNA
<213>  Artificial

<220>
<223>  anti-microRNA molecule

<400>  577
agagcacggu auuaaguucc ua                                              22


<210>  578
<211>  22
<212>  RNA
<213>  Artificial

<220>
<223>  anti-microRNA molecule

<400>  578
auagccgcug ucacacgcac aa                                              22


<210>  579
<211>  22
<212>  RNA
<213>  Artificial

<220>
<223>  anti-microRNA molecule

<400>  579
gcacugauuu cgaauggugc ua                                              22


<210>  580
<211>  22
<212>  RNA
<213>  Artificial

<220>
<223>  anti-microRNA molecule

<400>  580
cacaaguucg gauuuacggg uu                                              22


<210>  581
<211>  22
<212>  RNA
<213>  Artificial

<220>

<223>  anti-microRNA molecule

<400>  581
gcaggccggg acuagugcaa uu                                                22


<210>  582
<211>  22
<212>  RNA
<213>  Artificial

<220>
<223>  anti-microRNA molecule

<400>  582
gcacgagugu ucggucuagu ca                                                22


<210>  583
<211>  22
<212>  RNA
<213>  Artificial

<220>
<223>  anti-microRNA molecule

<400>  583
cgugcaaacg auuuucaaca ca                                                22


<210>  584
<211>  22
<212>  RNA
<213>  Artificial

<220>
<223>  anti-microRNA molecule

<400>  584
acacaccuga aauuuugcuc aa                                                22


<210>  585
<211>  22
<212>  RNA
<213>  Artificial

<220>
<223>  anti-microRNA molecule

<400>  585
ugugaauucu cccagugcca ag                                                22


<210>  586
<211>  22
<212>  RNA
<213>  Artificial

<220>
<223>  anti-microRNA molecule

<400>  586
augaaaugaa aucgacauga aa                                                22


<210>  587
<211>  22
<212>  RNA
<213>  Artificial

<220>
<223>  anti-microRNA molecule

<400>  587

gcaggcucca cuuaaauauu ua                                                22

<210>  588
<211>  22
<212>  RNA
<213>  Artificial

<220>
<223>  anti-microRNA molecule

<400>  588
aucagcuuuc aaaaugaucu ca                                                22

<210>  589
<211>  22
<212>  RNA
<213>  Artificial

<220>
<223>  anti-microRNA molecule

<400>  589
ccaguuuccu gugaaaccua aa                                                22

<210>  590
<211>  22
<212>  RNA
<213>  Artificial

<220>
<223>  anti-microRNA molecule

<400>  590
cagcuauucc gacaucuugc ca                                                22

<210>  591
<211>  22
<212>  RNA
<213>  Artificial

<220>
<223>  anti-microRNA molecule

<400>  591
ucacauuuac aaauugagau ua                                                22

<210>  592
<211>  22
<212>  RNA
<213>  Artificial

<220>
<223>  anti-microRNA molecule

<400>  592
agagcaccug augaaguaca au                                                22

<210>  593
<211>  22
<212>  RNA
<213>  Artificial

<220>
<223>  anti-microRNA molecule

<400>  593
ucuacagcua gaauaccaaa ga                                                22

<210> 594
<211> 22
<212> RNA
<213> Artificial

<220>
<223> anti-microRNA molecule

<400> 594
uugagaguca cuaaguaccu ga 22


<210> 595
<211> 22
<212> RNA
<213> Artificial

<220>
<223> anti-microRNA molecule

<400> 595
auacagcuaa aaucaccaaa ga 22


<210> 596
<211> 22
<212> RNA
<213> Artificial

<220>
<223> anti-microRNA molecule

<400> 596
ucacaaguua gggucucagg ga 22


<210> 597
<211> 22
<212> RNA
<213> Artificial

<220>
<223> anti-microRNA molecule

<400> 597
cgcucacuca aggagguugu ga 22


<210> 598
<211> 22
<212> RNA
<213> Artificial

<220>
<223> anti-microRNA molecule

<400> 598
cucacaguau aauccuguga uu 22


<210> 599
<211> 22
<212> RNA
<213> Artificial

<220>
<223> anti-microRNA molecule

<400> 599
cagcuaugcc gacaucuugc ca 22


<210> 600
<211> 22
<212> RNA

<213>  Artificial

<220>
<223>  anti-microRNA molecule

<400>  600
uaggacaaac uuuacccagu gc                                              22


<210>  601
<211>  22
<212>  RNA
<213>  Artificial

<220>
<223>  anti-microRNA molecule

<400>  601
aaaggccggg aagugugcaa ua                                              22


<210>  602
<211>  22
<212>  RNA
<213>  Artificial

<220>
<223>  anti-microRNA molecule

<400>  602
ucaggccggu gaaugugcaa ua                                              22


<210>  603
<211>  22
<212>  RNA
<213>  Artificial

<220>
<223>  anti-microRNA molecule

<400>  603
ucaggccguc ucaagugcaa ua                                              22


<210>  604
<211>  22
<212>  RNA
<213>  Artificial

<220>
<223>  anti-microRNA molecule

<400>  604
ucgggcugug aaaagugcaa ua                                              22


<210>  605
<211>  21
<212>  RNA
<213>  Artificial

<220>
<223>  anti-microRNA molecule

<400>  605
ccgaacuuau uggcucgaau a                                               21


<210>  606
<211>  22
<212>  RNA
<213>  Artificial

<220>

<223>  anti-microRNA molecule

<400>  606
gcuuucugag caacaaucaa aa                                                22


<210>  607
<211>  22
<212>  RNA
<213>  Artificial

<220>
<223>  anti-microRNA molecule

<400>  607
cgccaguaag cggaaaaaga ca                                                22


<210>  608
<211>  22
<212>  RNA
<213>  Artificial

<220>
<223>  anti-microRNA molecule

<400>  608
uggauaccac cagcuguguu ca                                                22


<210>  609
<211>  22
<212>  RNA
<213>  Artificial

<220>
<223>  anti-microRNA molecule

<400>  609
ugagauaaac aaagcccagu ga                                                22


<210>  610
<211>  22
<212>  RNA
<213>  Artificial

<220>
<223>  anti-microRNA molecule

<400>  610
cccaucaaag cuggcuguga ua                                                22


<210>  611
<211>  22
<212>  RNA
<213>  Artificial

<220>
<223>  anti-microRNA molecule

<400>  611
ucaggauaca uucaguauac gu                                                22


<210>  612
<211>  22
<212>  RNA
<213>  Artificial

<220>
<223>  anti-microRNA molecule

<400>  612

uacguauacu gaagguauac cg                                                22

<210>  613
<211>  23
<212>  RNA
<213>  Artificial

<220>
<223>  2'-O-methyl microRNA molecule

<220>
<221>  misc_structure
<222>  (23)..(23)
<223>  3' aminolinker attached to the a nucleotide at position 23

<400>  613
gucaacauca gucugauaag cua                                               23

<210>  614
<211>  21
<212>  RNA
<213>  Artificial

<220>
<223>  2'-O-methyl antisense molecule

<220>
<221>  misc_structure
<222>  (21)..(21)
<223>  3' aminolinker attached to the u nucleotide at position 21

<400>  614
aaggcaagcu gacccugaag u                                                 21

<210>  615
<211>  21
<212>  RNA
<213>  Artificial

<220>
<223>  2' O- methyl reverse seqeunce

<220>
<221>  misc_structure
<222>  (21)..(21)
<223>  3' aminolinker attached to the a nucleotide at position 21

<400>  615
   ugaaguccca gucgaacgga a                                                21

<210>  616
<211>  26
<212>  DNA
<213>  Artificial

<220>
<223>  2'-deoxy microRNA molecule

<220>
<221>  misc_structure
<222>  (26)..(26)
<223>  3' aminolinker attached to the g nucleotide at position 26

<400>  616
gtcaacatca gtctgataag ctagcg                                            26

<210> 617
<211> 24
<212> DNA
<213> Artificial

<220>
<223> 2'-deoxy antisense molecule

<220>
<221> misc_structure
<222> (24)..(24)
<223> 3' aminolinker attached to the g nucleotide at position 24


<400> 617
aaggcaagct gaccctgaag tgcg                                              24


<210> 618
<211> 88
<212> DNA
<213> Artificial

<220>
<223> primer

<400> 618
gaacaattgc ttttacagat gcacatatcg aggtgaacat cacgtacgtc aacatcagtc      60

tgataagcta tcggttggca gaagctat                                          88


<210> 619
<211> 90
<212> DNA
<213> Artificial

<220>
<223> primer

<400> 619
ggcataaaga attgaagaga gttttcactg catacgacga ttctgtgatt tgtattcagc      60

ccatatcgtt tcatagcttc tgccaaccga                                        90


<210> 620
<211> 35
<212> DNA
<213> Artificial

<220>
<223> primer

<400> 620
taatacgact cactatagaa caattgcttt tacag                                  35


<210> 621
<211> 35
<212> DNA
<213> Artificial

<220>
<223> primer

<400> 621
atttaggtga cactataggc ataaagaatt gaaga                                  35


<210> 622
<211> 33
<212> DNA
<213> Artificial

<220>
<223>  synthetic oligonucleotide molecule

<400>  622
ggcctcaaca tcagtctgat aagctaggta cct                                    33


<210>  623
<211>  33
<212>  DNA
<213>  Artificial

<220>
<223>  synthetic oligonucleotide molecule

<400>  623
ggccaggtac ctagcttatc agactgatgt tga                                    33

## Description

This application is a continuing application of U.S. Application Serial Number 10/778,908 filed on February 13, 2004. The specification of U.S. Application Serial Number 10/778,908 is hereby incorporated by reference in its entirety.

The invention claimed herein was made with the help of grant number 1 R01 GM068476-01 from NIH/NIGMS. The U.S. government has certain rights in the invention.

### BACKGROUND OF THE INVENTION

RNA silencing is a fundamental mechanism of gene regulation that uses double-stranded RNA (dsRNA) derived 21- to 28-nucleotide (nt) small RNAs to guide mRNA degradation, control mRNA translation or chromatin modification. Recently, several hundred novel genes were identified in plants and animals that encode transcripts that contain short dsRNA hairpins.

Defined 22-nt RNAs, referred to as microRNAs (miRNAs), are reported to be excised by dsRNA specific endonucleases from the hairpin precursors. The miRNAs are incorporated into ribonucleoprotein particles (miRNPs).

Plant miRNAs target mRNAs containing sequence segments with high complementarity for degradation or suppress translation of partially complementary mRNAs. Animal miRNAs appear to act predominantly as translational repressors. However, animal miRNAs have also been reported to guide RNA degradation. This indicates that animal miRNPs act like small interfering RNA (siRNA)-induced silencing complexes (RISCs).

Understanding the biological function of miRNAs requires knowledge of their mRNA targets. Bioinformatic approaches have been used to predict mRNA targets, among which transcription factors and proapoptotic genes were prominent candidates. Processes such as *Notch* signaling, cell proliferation, morphogenesis and axon guidance appear to be controlled by miRNA genes.

Therefore, there is a need for materials and methods that can help elucidate the function of known and future microRNAs. Due to the ability of microRNAs to induce RNA degradation or repress translation of mRNA which encode important proteins, there is also a need for novel compositions for inhibiting microRNA-indcued cleavage or repression of mRNAs.

### SUMMARY THE INVENTION

In one embodiment, the invention provides an isolated single stranded anti-microRNA molecule comprising a minimum of ten moieties and a maximum of fifty moieties on a molecular backbone, the molecular backbone comprising backbone units, each moiety comprising a base bonded to a backbone unit, each base forming a Watson-Crick base pair with a complementary base wherein at least ten contiguous bases have the same sequence as a sequence of bases in any one of the anti-microRNA molecules shown in Tables 1-4, except that up to thirty percent of the bases pairs may be wobble base pairs, and up to 10% of the contiguous bases may be additions, deletions, mismatches, or combinations thereof; no more than fifty percent of the contiguous moieties contain deoxyribonuleotide backbone units; the moiety in the molecule at the position corresponding to position 11 of the microRNA is non-complementary; and the molecule is capable of inhibiting microRNP activity.

In another embodiment, the invention provides a method for inhibiting microRNP activity in a cell, the microRNP comprising a microRNA molecule, the microRNA molecule comprising a sequences of bases complementary of the sequence of bases in a single stranded anti-microRNA molecule, the method comprising introducing into the cell the single-stranded anti-microRNA molecule comprising a sequence of a minimum of ten moieties and a maximum of fifty moieties on a molecular backbone, the molecular backbone comprising backbone units, each moiety comprising a base bonded to a backbone unit, each base forming a Watson-Crick base pair with a complementary base, wherein at least ten contiguous bases of the anti-microRNA molecule are complementary to the microRNA, except that up to thirty percent of the bases may be substituted by wobble base pairs, and up to ten percent of the at least ten moieties may be additions, deletions, mismatches, or combinations thereof; no more than fifty percent of the contiguous moieties contain deoxyribonuleotide backbone units; and the moiety in the molecule at the position corresponding to position 11 of the microRNA is non-complementary.

In another embodiment, the invention provides an isolated microRNA molecule comprising a minimum of ten moieties and a maximum of fifty moieties on a molecular backbone, the molecular backbone comprising backbone units, each moiety comprising a base bonded to a backbone unit, wherein at least ten contiguous bases have the same sequence as a sequence of bases in any one of the microRNA molecules shown in Table 2, except that up to thirty percent of the bases pairs may be wobble base pairs, and up to 10% of the contiguous bases may be additions, deletions, mismatches, or combinations thereof; and no more than fifty percent of the contiguous moieties contain deoxyribonucleotide backbone units.

In another embodiment, the invention provides an isolated microRNA molecule comprising a minimum of ten moieties and a maximum of fifty moieties on a molecular backbone, the molecular backbone comprising backbone units, each moiety comprising a base bonded to a backbone unit, wherein at least ten contiguous bases have any one of the microRNA sequences shown in Tables 1, 3 and 4, except that up to thirty percent of the bases pairs may be wobble base pairs, and up to 10% of the contiguous bases may be additions, deletions, mismatches, or combinations thereof; no more than fifty percent of the contiguous moieties contain deoxyribonuleotide backbone units; and is modified for increased nuclease resistance.

In yet another embodiment, the invention provides an isolated single stranded anti-microRNA molecule comprising a minimum of ten moieties and a maximum of fifty moieties on a molecular backbone, the molecular backbone comprising backbone units, each moiety comprising a base bonded to a backbone unit, each base forming a Watson-Crick base pair with a complementary base wherein at least ten contiguous bases have the same sequence as a sequence of bases in any one of the anti-microRNA molecules shown in Tables 1-4, except that up to thirty percent of the bases pairs may be wobble base pairs, and up to 10% of the contiguous bases may be additions, deletions, mismatches, or combinations thereof; no more than fifty percent of the contiguous moieties contain deoxyribonuleotide backbone units; and the molecule is capable of inhibiting microRNP activity.

In yet a further embodiment, the invention provides a method for inhibiting microRNP activity in a cell, the microRNP comprising a microRNA molecule, the microRNA molecule comprising a sequences of bases complementary of the sequence of bases in a single stranded anti-microRNA molecule, the method comprising introducing into the cell the single-stranded anti-microRNA molecule comprising a sequence of a minimum of ten moieties and a maximum of fifty moieties on a molecular backbone, the molecular backbone comprising backbone units, each moiety comprising a base bonded to a backbone unit, each base forming a Watson-Crick base pair with a complementary base, wherein at least ten contiguous bases of the anti-microRNA molecule are complementary to the microRNA, except that up to thirty percent of the bases may be substituted by wobble base pairs, and up to ten percent of the at least ten moieties may be additions, deletions, mismatches, or combinations thereof; and no more than fifty percent of the contiguous moieties contain deoxyribonuleotide backbone units.

### DESCRIPTION OF THE FIGURES

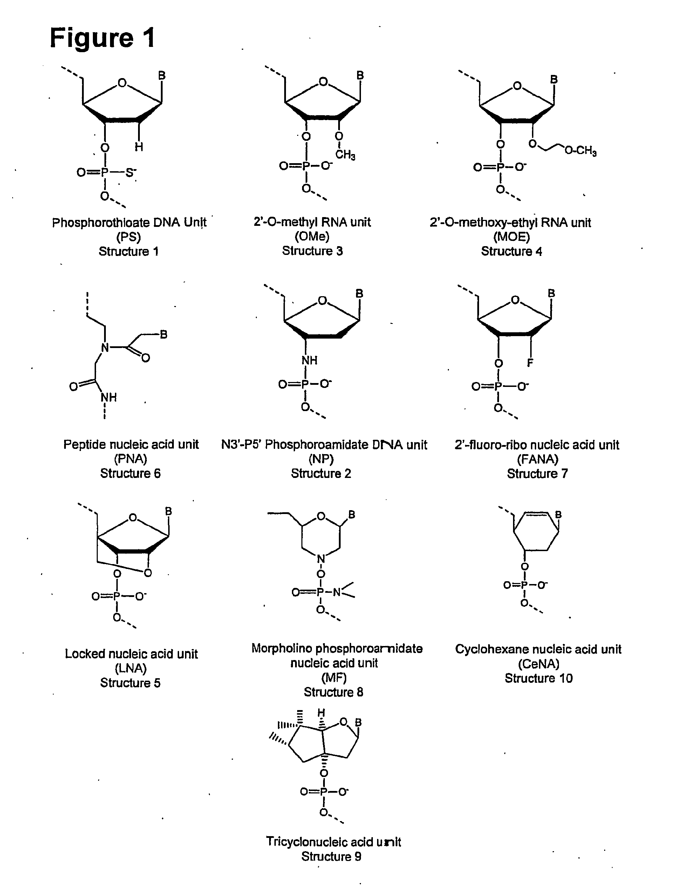

Figure 1 shows the modified nucleotide units discussed in the specification. B denotes any one of the following nucleic acid bases: adenosine, cytidine, guanosine, thymine, or uridine.

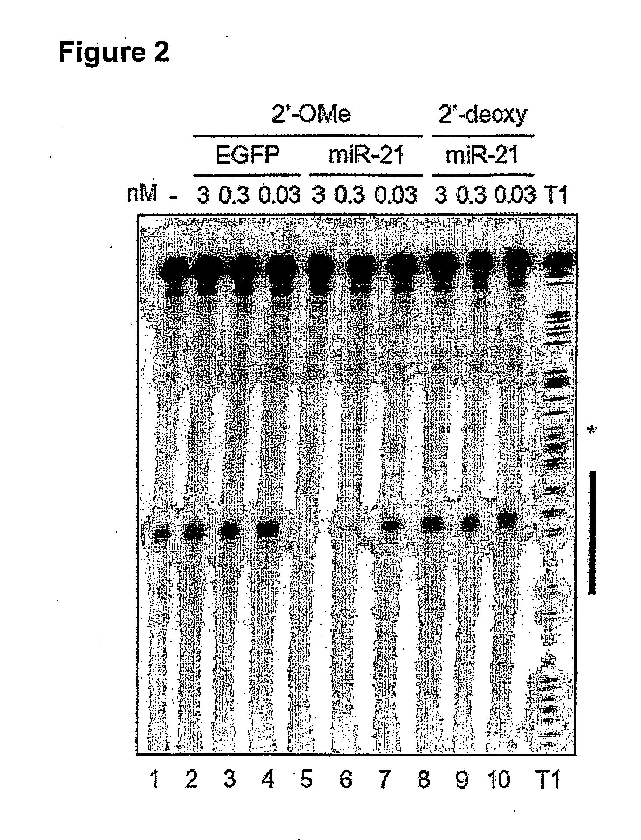

Figure 2. Antisense 2'-O-methyl oligoribonucleotide specifically inhibit miR-21 guided cleavage activity in HeLa cell S100 cytoplasmic extracts. The black bar to the left of the RNase T1 ladder represents the region of the target RNA complementary to miR-21. Oligonucleotides complementary to miR-21 were pre-incubated in S100 extracts prior to the addition of ³²P-cap-labelled cleavage substrate. Cleavage bands and T1 hydrolysis bands appear as doublets after a 1-nt slipping of the T7 RNA polymerase near the middle of the transcript indicated by the asterisk.

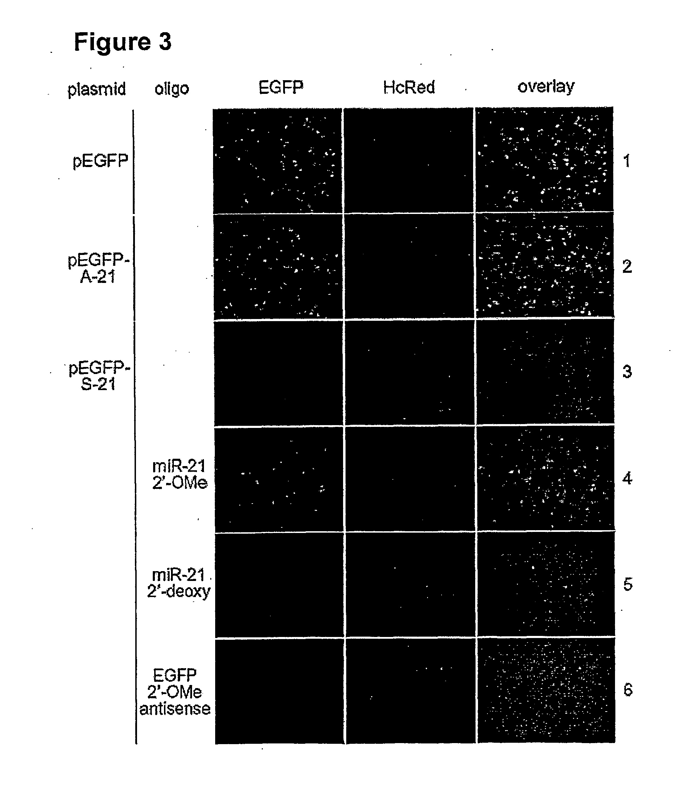

Figure 3. Antisense 2'-O-methyl oligoribonucleotides interfere with endogenous miR-21 RNP cleavage in HeLa cells. HeLa cells were transfected with pHcRed and pEGFP or its derivatives, with or without inhibitory or control oligonucleotides. EGFP and HcRed protein fluorescence were excited and recorded individually by fluorescence microscopy 24 h after transfection. Co-expression of co-transfected reporter plasmids was documented by superimposing of the fluorescence images in the right panel.

### DETAILED DESCRIPTION OF THE INVENTION

The invention relates to an isolated single stranded anti-microRNA molecule. The molecule comprises a minimum number of ten moieties, preferably a minimum of thirteen, more preferably a minimum of fifteen, even more preferably a minimum of 18, and most preferably a minimum of 21 moieties.

The anti-microRNA molecule comprises a maximum number of fifty moieties, preferably a maximum of forty, more preferably a maximum of thirty, even more preferably a maximum of twenty-five, and most preferably a maximum of twenty-three moieties. A suitable range of minimum and maximum number of moieties may be obtained by combining any of the above minima with any of the above maxima.

Each moiety comprises a base bonded to a backbone unit. In this specification, a base refers to any one of the nucleic acid bases present in DNA or RNA. The base can be a purine or pyrimidine. Examples of purine bases include adenine (A) and guanine (G). Examples of pyrimidine bases include thymine (T), cytosine (C) and uracil (U). Each base of the moiety forms a Watson-Crick base pair with a complementary base.

Watson-Crick base pairs as used herein refers to the hydrogen bonding interaction between, for example, the following bases: adenine and thymine (A = T); adenine and uracil (A = U); and cytosine and guanine (C = G). The adenine can be replaced with 2,6-diaminopurine without compromising base-pairing.

The backbone unit may be any molecular unit that is able stably to bind to a base and to form an oligomeric chain. Suitable backbone units are well known to those in the art.

For example, suitable backbone units include sugar-phosphate groups, such as the sugar-phosphate groups present in ribonucleotides, deoxyribonucleotides, phosphorothioate deoxyribose groups, N'3-N'5 phosphoroamidate deoxyribose groups, 2'O-alkyl-ribose phosphate groups, 2'-O-alkyl-alkoxy ribose phosphate groups, ribose phosphate group containing a methylene bridge, 2'-Fluororibose phosphate groups, morpholino phosphoroamidate groups, cyclohexene groups, tricycle phosphate groups, and amino acid molecules.

In one embodiment, the anti-microRNA molecule comprises at least one moiety which is a ribonucleotide moiety or a deoxyribonucleotide moiety.

In another embodiment, the anti-microRNA molecule comprises at least one moiety which confers increased nuclease resistance. The nuclease can be an exonuclease, an endonuclease, or both. The exonuclease can be a 3'→5 ' exonuclease or a 5'→3' exonuclease. Examples of 3'→5' human exonuclease include PNPT1, Wemer syndrome helicase, RRP40, RRP41, RRP42, RRP45, and RRP46. Examples of 5'→3' exonuclease include XRN2, and FEN1. Examples of endonucleases include Dicer, Drosha, RNase4, Ribonuclease P, Ribonuclease H1, DHP1, ERCC-1 and OGG1. Examples of nucleases which function as both an exonuclease and an endonuclease include APE1 and EXO1.

An anti-microRNA molecule comprising at least one moiety which confers increased nuclease resistance means a sequence of moieties wherein at least one moiety is not recognized by a nuclease. Therefore, the nuclease resistance of the molecule is increased compared to a sequence containing only unmodified ribonucleotide, unmodified deoxyribonucleotide or both. Such modified moieties are well known in the art, and were reviewed, for example, by Kurreck, Eur. J. Biochem. 270, 1628-1644 (2003).

A modified moiety can occur at any position in the anti-microRNA molecule. For example, to protect the anti-microRNA molecule against 3'→5' exonucleases, the molecule can have at least one modified moiety at the 3' end of the molecule and preferably at least two modified moieties at the 3' end. If it is desirable to protect the molecule against 5'→3' exonuclease, the anti-microRNA molecule can have at least one modified moiety and preferably at least two modified moieties at the 5' end of the molecule. The anti-microRNA molecule can also have at least one and preferably at least two modified moieties between the 5' and 3' end of the molecule to increase resistance of the molecule to endonucleases. In one embodiment, all of the moieties are nuclease resistant.

In another embodiment, the anti-microRNA molecule comprises at least one modified deoxyribonucleotide moiety. Suitable modified deoxyribonucleotide moieties are known in the art.

A suitable example of a modified deoxyribonucleotide moiety is a phosphorothioate deoxyribonucleotide moiety. See structure 1 in figure 1. An anti-microRNA molecule comprising more than one phosphorothioate deoxyribonucleotide moiety is referred to as phosphorothioate (PS) DNA. See, for example, Eckstein, Antisense Nucleic Acids Drug Dev. 10, 117-121 (2000).

Another suitable example of a modified deoxyribonucleotide moiety is an N'3-N'5 phosphoroamidate deoxyribonucleotide moiety. See structure 2 in figure 1. An oligonucleotide molecule comprising more than one phosphoroamidate deoxyribonucleotide moiety is referred to as phosphoroamidate (NP) DNA. See, for example, Gryaznov et al., J. Am. Chem. Soc.116, 3143-3144 (1994).

In another embodiment, the molecule comprises at least one modified ribonucleotide moiety. Suitable modified ribonucleotide moieties are known in the art.

A suitable example of a modified ribonucleotide moiety is a ribonucleotide moiety that is substituted at the 2' position. The substituents at the 2' position may, for example, be a C₁ to C₄ alkyl group. The C₁ to C₄ alkyl group may be saturated or unsaturated, and unbranched or branched. Some examples of C₁ to C₄ alkyl groups include ethyl, isopropyl, and allyl. The preferred C₁ to C₄ alkyl group is methyl. See structure 3 in figure 1. An oligoribonucleotide molecule comprising more than one ribonucleotide moeity that is substituted at the 2' position with a C₁ to C₄ alkyl group is referred to as a 2'-O -(C₁-C₄ alkyl) RNA, e.g.,2'-O-methyl RNA (OMe RNA).

Another suitable example of a substituent at the 2' position of a modified ribonucleotide moiety is a C₁ to C₄ alkoxy - C₁ to C₄ alkyl group. The C₁ to C₄ alkoxy (alkyloxy) and C₁ to C₄ alkyl group may comprise any of the alkyl groups described above. The preferred C₁ to C₄ alkoxy - C₁ to C₄ alkyl group is methoxyethyl. See structure 4 in figure 1. An oligonucleotide molecule comprising more than one ribonucleotide moiety that is substituted at the 2' position with a C₁ to C₄ alkoxy-C₁ to C₄ alkyl group is referred to as a 2'-O-(C₁ to C₄ alkoxy - C₄ to C₄ alkyl) RNA, e:g., 2'-O-methoxyethyl RNA (MOE RNA).

Another suitable example of a modified ribonucleotide moiety is a ribonucleotide that has a methylene bridge between the 2'-oxygen atom and the 4'-carbon atom. See structure 5 in figure 1. An oligoribonucleotide molecule comprising more than one ribonucleotide moiety that has a methylene bridge between the 2'-oxygen atom and the 4'-carbon atom is referred to as locked nucleic acid (LNA). See, for example, Kurreck et al., Nucleic Acids Res. 30, 1911-1918 (2002); Elayadi et al., Curr. Opinion Invest. Drugs 2, 558-561 (2001); Ørum et al., Curr. Opinion Mol. Ther. 3, 239-243 (2001); Koshkin et al., Tetrahedron 54, 3607-3630 (1998); Obika et al., Tetrahedron Lett.39, 5401-5404 (1998). Locked nucleic acids are commercially available from Proligo (Paris, France and Boulder, Colorado, USA).

Another suitable example of a modified ribonucleotide moiety is a ribonucleotide that is substituted at the 2' position with fluoro group. A modified ribonucleotide moiety having a fluoro group at the 2' position is a 2'-fluororibonucleotide moiety. Such moieties are known in the art. Molecules comprising more than one 2'-fluororibonucleotide moiety are referred to herein as 2'-fluororibo nucleic acids (FANA). See structure 7 in figure 1. Damha et al., J. Am. Chem. Soc. 120, 12976-12977 (1998).

In another embodiment, the anti-microRNA molecule comprises at least one base bonded to an amino acid residue. Moieties that have at least one base bonded to an amino acid residue will be referred to herein as peptide nucleic acid (PNA) moieties. Such moieties are nuclease resistance, and are known in the art. Molecules having more than one PNA moiety are referred to as peptide nucleic acids. See structure 6 in figure 1. Nielson, Methods Enzymol. 313, 156-164 (1999); Elayadi, *et al*, *id*.; Braasch et al., Biochemistry 41, 4503-4509 (2002), Nielsen et al., Science 254, 1497-1500 (1991).

The amino acids can be any amino acid, including natural or non-natural amino acids. Naturally occurring amino acids include, for example, the twenty most common amino acids normally found in proteins, i.e., alanine (Ala), arginine (Arg), asparagine (Asn), aspartic acid (Asp), cysteine (Cys), glutamine (Glu), glutamic acid (Glu), glycine (Gly), histidine (His), isoleucine (Ileu), leucine (Leu), lysine (Lys), methionine (Met), phenylalanine (Phe), proline (Pro), serine (Ser), threonine (Thr), tryptophan, (Trp), tyrosine (Tyr), and valine (Val).

The non-natural amino acids may, for example, comprise alkyl, aryl, or alkylaryl groups. Some examples of alkyl amino acids include α-aminobutyric acid, β-aminobutyric acid, γ-aminobutyric acid, δ-aminovaleric acid, and ε-aminocaproic acid. Some examples of aryl amino acids include ortho-, meta, and para-aminobenzoic acid. Some examples of alkylaryl amino acids include ortho-, meta-, and para-aminophenylacetic acid, and γ-phenyl-β-aminobutyric acid.

Non-naturally occurring amino acids also include derivatives of naturally occurring amino acids. The derivative of a naturally occurring amino acid may, for example, include the addition or one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include hydroxyl, C₁-C₄ alkoxy, amino, methylamino, dimethylamino, nitro, halo (i.e., fluoro, chloro, bromo, or iodo), or branched or unbranched C₁-C₄ alkyl, such as methyl, ethyl, n-propyl, isopropyl, butyl, isobutyl, or t-butyl.

Furthermore, other examples of non-naturally occurring amino acids which are derivatives of naturally occurring amino acids include norvaline (Nva), norleucine (Nle), and hydroxyproline (Hyp).

The amino acids can be identical or different from one another. Bases are attached to the amino acid unit by molecular linkages. Examples of linkages are methylene carbonyl, ethylene carbonyl and ethyl linkages. (Nielsen et al., Peptide Nucleic Acids-Protocols and Applications, Horizon Scientific Press, pages 1-19; Nielsen et al., Science 254: 1497-1500.)

One example of a PNA moiety is N-(2-aminoethyl)-glycine. Further examples of PNA moieties include cyclohexyl PNA, retro-inverso, phosphone, propionyl and aminoproline PNA.

PNA can be chemically synthesized by methods known in the art, e.g. by modified Fmoc or tBoc peptide synthesis protocols. The PNA has many desirable properties, including high melting temperatures (Tm), high base-pairing specificity with nucleic acid and an uncharged molecular backbone. Additionally, the PNA does not confer RNase H sensitivity on the target RNA, and generally has good metabolic stability.

Peptide nucleic acids are also commercially available from Applied Biosystems (Foster City, California, USA):

In another embodiment, the anti-microRNA molecule comprises at least one morpholino phosphoroamidate nucleotide moiety. A morpholino phosphoroamidate nucleotide moiety is a modified moiety which is nuclease resistant. Such moieties are known in the art. Molecules comprising more than one morpholino phosphoroamidate nucleotide moiety are referred to as morpholino (MF) nucleic acids. See structure 8 in figure 1. Heasman, Dev. Biol. 243, 209-214 (2002). Morpholono oligonucleotides are commercially available from Gene Tools LLC (Corvallis, Oregon, USA).

In another embodiment, the anti-microRNA molecule comprises at least one cyclohexene nucleotide moiety. A cyclohexene nucleotide moiety is a modified moiety which is nuclease resistant. Such moieties are known in the art. Molecules comprising more than one cyclohexene nucleotide moiety are referred to as cyclohexene nucleic acids (CeNA). See structure 10 in figure 1. Wang et al., J. Am. Chem. Soc. 122, 8595-8602 (2000), Verbeure et al., Nucleic Acids Res. 29, 4941-4947 (2001).

In another embodiment, the anti-microRNA molecule comprises at least one tricyclo nucleotide moiety. A tricyclo nucleotide moiety is a modified moiety which is nuclease resistant. Such moieties are known in the art. Steffens et al., J. Am. Chem. Soc. 119, 11548-11549 (1997), Renneberg et al., J. Am. Chem. Soc.124, 5993-6002 (2002). Molecules comprising more than one tricyclo nucleotide moiety are referred to as tricycle nucleic acids (tcDNA). See structure 9 in figure 1.

In another embodiment, to increase nuclease resistance of the anti-microRNA molecules of the present invention to exonucleases, inverted nucleotide caps can be attached to the 5' end, the 3' end, or both ends of the molecule. An inverted nucleotide cap refers to a 3'→5' sequence of nucleic acids attached to the anti-microRNA molecule at the 5' and/or the 3' end. There is no limit to the maximum number of nucleotides in the inverted cap just as long as it does not interfere with binding of the anti-microRNA molecule to its target microRNA. Any nucleotide can be used in the inverted nucleotide cap. Typically, the inverted nucleotide cap is one nucleotide in length. The nucleotide for the inverted cap is generally thymine, but can be any nucleotide such as adenine, guanine, uracil, or cytosine.

Alternatively, an ethylene glycol compound and/or amino linkers can be attached to the either or both ends of the anti-microRNA molecule. Amino linkers can also be used to increase nuclease resistance of the anti-microRNA molecules to endonucleases. The table below lists some examples of amino linkers. The below listed amino linker are commercially available from TriLink Biotechnologies, San Diego, CA.

| |
|---|
| 2'-Deoxycytidine-5-C6 Amino Linker (3' Terminus) |
| 2'-Deoxycytidine-5-C6 Amino Linker (5' or Internal) |
| 3' C3 Amino Linker |
| 3' C6 Amino Linker |
| 3' C7 Amino Linker |
| 5' C12 Amino Linker |
| 5' C3 Amino Linker |
| 5' C6 Amino Linker |
| C7 Internal Amino Linker |
| Thymidine-5-C2 Amino Linker (5' or Internal) |
| Thymidine-5-C6 Amino Linker (3' Terminus) |
| Thymidine-5-C6 Amino Linker (Internal) |

Chimeric anti-microRNA molecules containing a mixture of any of the moieties mentioned above are also known, and may be made by methods known, in the art. See, for example, references cited above, and Wang et al, Proc. Natl. Acad. Sci. USA 96, 13989-13994 (1999), Liang et al., Eur. J. Biochem. 269, 5753-5758 (2002), Lok et al., Biochemistry 41, 3457-3467 (2002), and Damha et al., J. Am. Chem. Soc. 120, 12976-12977 (2002).

The molecules of the invention comprise at least ten contiguous, preferably at least thirteen contiguous, more preferably at least fifteen contiguous, and even more preferably at least twenty contiguous bases that have the same sequence as a sequence of bases in any one of the anti-microRNA molecules shown in Tables 1-4. The anti-microRNA molecules optimally comprise the entire sequence of any one of the anti-microRNA molecule sequences shown in Tables 1-4.

For the contiguous bases mentioned above, up to thirty percent of the base pairs may be substituted by wobble base pairs. As used herein, wobble base pairs refers to either: i) substitution of a cytosine with a uracil, or 2) the substitution of a adenine with a guanine, in the sequence of the anti-microRNA molecule. These wobble base pairs are generally referred to as UG or GU wobbles. Below is a table showing the number of contiguous bases and the maximum number of wobble base pairs in the anti-microRNA molecule:

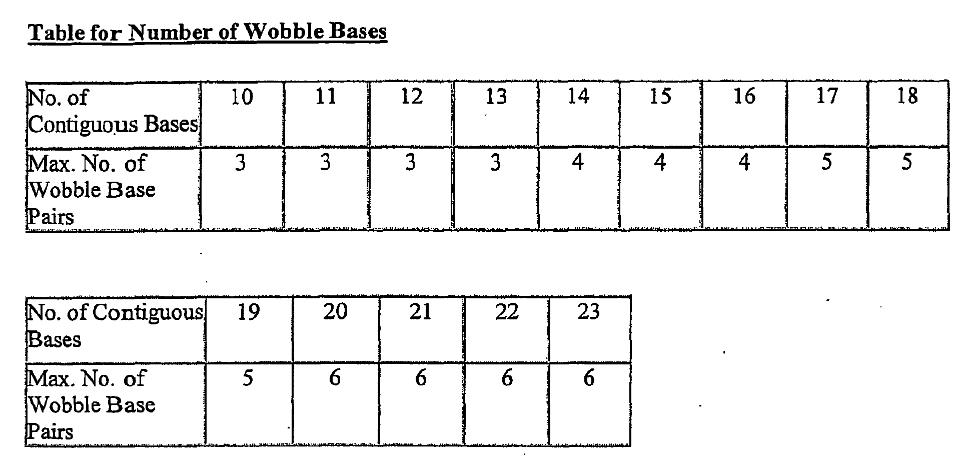

Further, up to ten percent, and preferably up to five percent of the contiguous bases can be additions, deletions, mismatches or combinations thereof. Additions refer to the insertion in the contiguous sequence of any moiety described above comprising any one of the bases described above. Deletions refer to the removal of any moiety present in the contiguous sequence. Mismatches refer to the substitution of one of the moieties comprising a base in the contiguous sequence with any of the above described moieties comprising a different base.

The additions, deletions or mismatches can occur anywhere in the contiguous sequence, for example, at either end of the contiguous sequence or within the contiguous sequence of the anti-microRNA molecule. If the contiguous sequence is relatively short, such as from about ten to about 15 moieties in length, preferably the additions, deletions or mismatches occur at the end of the contiguous sequence. If the contiguous sequence is relatively long, such as a minimum of sixteen contiguous sequences, then the additions, deletions, or mismatches can occur anywhere in the contiguous sequence. Below is a table showing the number of contiguous bases and the maximum number of additions, deletions, mismatches or combinations thereof:

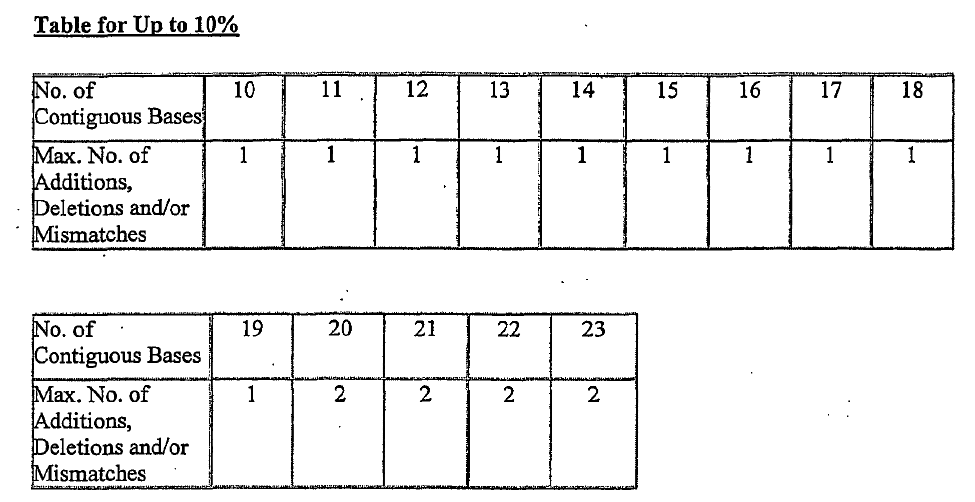

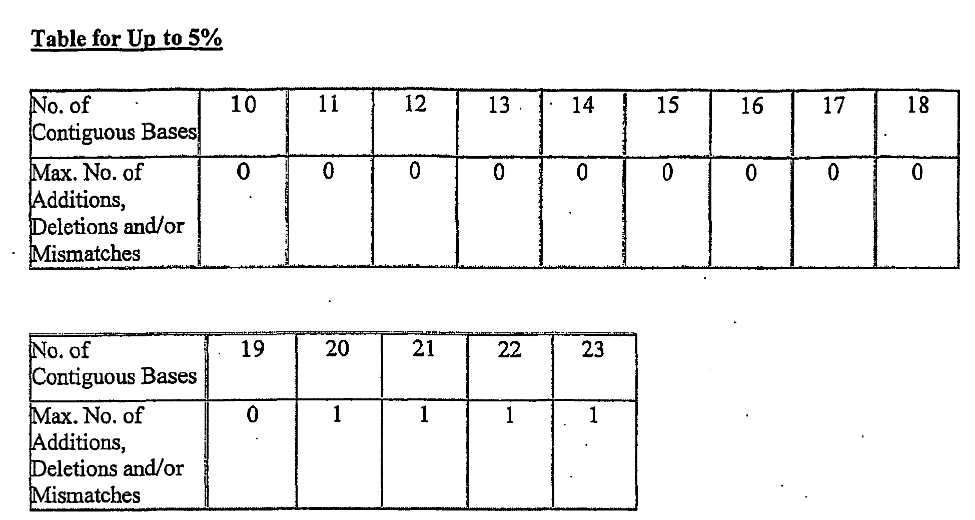

Furthermore, no more than fifty percent, and preferably no more than thirty percent, of the contiguous moieties contain deoxyribonucleotide backbone units. Below is a table showing the number of contiguous bases and the maximum number of deoxyribonucleotide backbone units:

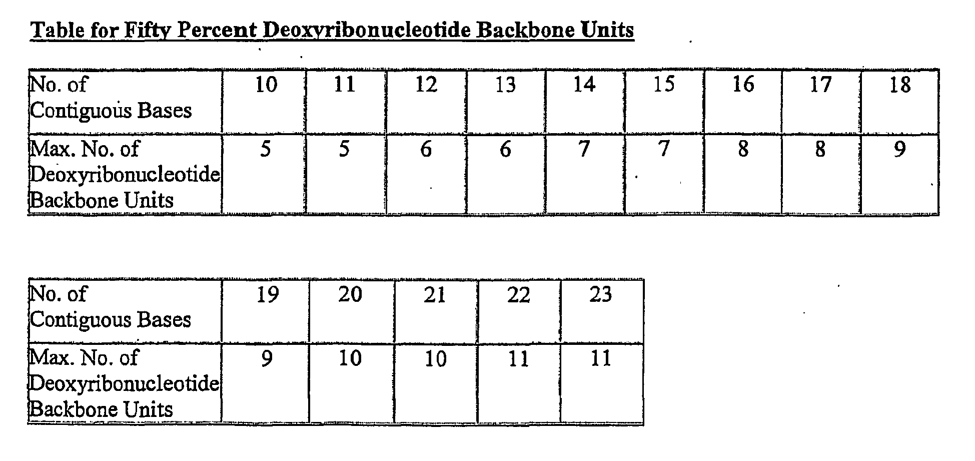

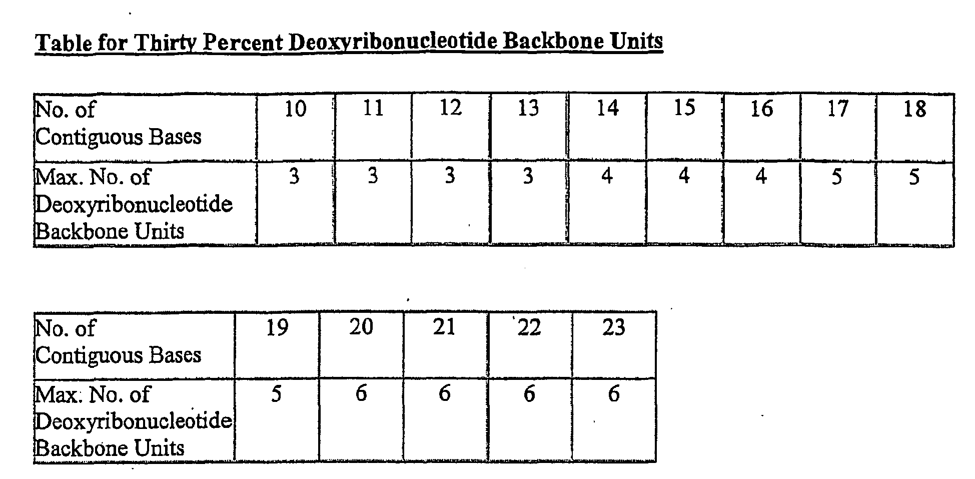

The moiety in the anti-RNA molecule at the position corresponding to position 11 of the microRNA is optionally non-complementary to a microRNA. The moiety in the anti-microRNA molecule corresponding to position 11 of the microRNA can be rendered non-complementary by an addition, deletion or mismatch as described above.

In another embodiment, if the anti-microRNA molecule comprises only unmodified moieties, then the anti-microRNA molecules comprises at least one base, in the at least ten contiguous bases, which is non-complementary to the microRNA and/or comprises an inverted nucleotide cap, ethylene glycol compound or an amino linker.

In yet another embodiment, if the at least ten contiguous bases in an anti-microRNA molecule is perfectly (i.e., 100%) complementary to ten contiguous bases in a microRNA, then the anti-microRNA molecule contains at least one modified moiety in the at least ten contiguous bases and/or comprises an inverted nucleotide cap, ethylene glycol compound or an amino linker.

As stated above, the maximum length of the anti-microRNA molecule is 50 moieties. Any number of moieties having any base sequence can be added to the contiguous base sequence. The additional moieties can be added to the 5' end, the 3' end, or to both ends of the contiguous sequence.

MicroRNA molecules are derived from genomic loci and are produced from specific microRNA genes. Mature microRNA molecules are processed from precursor transcripts that form local hairpin structures. The hairpin structures are typically cleaved by an enzyme known as Dicer, which generates one microRNA duplex. See Bartel, Cell 116, 281-297 (2004) for a review on microRNA molecules. The article by Bartel is hereby incorporated by reference.

Each strand of a microRNA is packaged in a microRNA ribonucleoprotein complex (microRNP). A microRNP in, for example, humans, also includes the proteins eIF2C2, the helicase Gemin3, and Gemin 4.

The sequence of bases in the anti-microRNA molecules of the present invention can be derived from a microRNA from any species e.g. such as a fly (e.g., *Drosophila melanogaster*)*,* a worm (e.g., *C*. *elegans).* Preferably the sequence of bases is found in mammals, especially humans (*H. sapiens),* mice (e.g., *M. musculus*)*,* and rats *(R. norvegicus).*

The anti-microRNA molecule is preferably isolated, which means that it is essentially free of other nucleic acids. Essentially free from other nucleic acids means that it is at least 90%, preferably at least 95% and, more preferably, at least 98% free of other nucleic acids.

Preferably, the molecule is essentially pure, which means that the molecules is free not only of other nucleic acids, but also of other materials used in the synthesis of the molecule, such as, for example, enzymes used in the synthesis of the molecule. The molecule is at least 90% free, preferably at least 95% free and, more preferably, at least 98% free of such materials.

The anti-microRNA molecules of the present invention are capable of inhibiting microRNP activity, preferable in a cell. Inhibiting microRNP activity refers to the inhibition of cleavage of the microRNA's target sequence or the repression of translation of the microRNA's target sequence. The method comprises introducing into the cell a single-stranded microRNA molecule.

Any anti-microRNA molecule can be used in the methods of the present invention, as long as the anti-microRNA is complementary, subject to the restrictions described above, to the microRNA present in the microRNP. Such anti-microRNAs include, for example, the anti-microRNA molecules mentioned above (see Table 1-4), and the anti-microRNAs molecules described in international PCT application number WO 03/029459 A2, the sequences of which are incorporated herein by reference.

The invention also includes any one of the microRNA molecules having the sequences as shown in Table 2. The novel microRNA molecules in Table 2 may optionally be modified as described above for anti-microRNA molecules. The other microRNA molecules in Tables 1, 3 and 4 are modified for increased nuclease resistance as described above for anti-microRNA molecules.

### Utility

The anti-microRNA molecules and the microRNA molecules of the present invention have numerous *in vivo, in vitro,* and *ex vivo* applications.

For example, the anti-microRNA molecules and microRNA of the present invention may be used as a modulator of the expression of genes which are at least partially complementary to the anti-microRNA molecules and microRNA. For example, if a particular microRNA is beneficial for the survival of a cell, an appropriate isolated microRNA of the present invention may be introduced into the cell to promote survival. Alternatively, if a particular microRNA is harmful (e.g., induces apoptosis, induces cancer, etc.), an appropriate anti-microRNA molecule can be introduced into the cell in order to inhibit the activity of the microRNA and reduce the harm.

In addition, anti-microRNA molecules and/or microRNAs of the present invention can be introduced into a cell to study the function of the microRNA. Any of the anti-microRNA molecules and/or microRNAs listed above can be introduced into a cell for studying their function. For example, a microRNA in a cell can be inhibited with a suitable anti-microRNA molecule. The function of the microRNA can be inferred by observing changes associated with inhibition of the microRNA in the cell in order to inhibit the activity of the microRNA and reduce the harm.

The cell can be any cell which expresses microRNA molecules, including the microRNA molecules listed herein. Alternatively, the cell can be any cell transfected with an expression vector containing the nucleotide sequence of a microRNA.

Examples of cells include, but are not limited to, endothelial cells, epithelial cells, leukocytes (e.g., T cells, B cells, neutrophils, macrophages, eosinophils, basophils, dendritic cells, natural killer cells and monocytes), stem cells, hemopoietic cells, embryonic cells, cancer cells.

The anti-microRNA molecules or microRNAs can be introduced into a cell by any method known to those skilled in the art. Useful delivery systems, include for example, liposomes and charged lipids. Liposomes typically encapsulate oligonucleotide molecules within their aqueous center. Charged lipids generally form lipid- oligonucleotide molecule complexes as a result of opposing charges.

These liposomes-oligonucleotide molecule complexes or lipid- oligonucleotide molecule complexes are usually internalized by endocytosis. The liposomes or charged lipids generally comprise helper lipids which disrupt the endosomal membrane and release the oligonucleotide molecules.

Other methods for introducing an anti-microRNA molecule or a microRNA into a cell include use of delivery vehicles, such as dendrimers, biodegradable polymers, polymers of amino acids, polymers of sugars, and oligonucleotide-binding nanoparticles. In addition, pluoronic gel as a depot reservoir can be used to deliver the anti-microRNA oligonucleotide molecules over a prolonged period. The above methods are described in, for example, Hughes et al., Drug Discovery Today 6, 303-315 (2001); Liang et al. Eur. J. Biochem. 269 5753-5758 (2002); and Becker et al., In Antisense Technology in the Central Nervous System (Leslie, R.A., Hunter, A.J. & Robertson, H.A., eds), pp.147-157, Oxford University Press.

Targeting of an anti-microRNA molecule or a microRNA to a particular cell can be performed by any method known to those skilled in the art. For example, the anti-microRNA molecule or microRNA can be conjugated to an antibody or ligand specifically recognized by receptors on the cell.

The sequences of microRNA and anti-microRNA molecules are shown in Tables 1-4 below. Human sequences are indicated with the prefix "hsa." Mouse sequences are indicated with the prefix "mmu." Rat sequences are indicated with the prefix "rno." *C. elegan* sequences are indicated with the prefix "cel." Drosophila sequences are indicated with the prefix "dme."

**Table 1: Human, Mouse and Rat microRNA and anti-microRNA sequences.**

| microRNA name | microRNA sequence (5' to 3') | Anti-microRNA molecule sequence (5' to 3') |
|---|---|---|
| hsa-miR-100. | AACCCGUAGAUCCGAACUUGUG | CACAAGUUCGGAUCUACGGGUU |
| hsa-miR-103 | AGCAGCAUUGUACAGGGCUAUG | CAUAGCCCUGUACAAUGCUGCU |
| hsa-miR-105-5p | UCAAAUGCUCAGACUCCUGUGG | CCACAGGAGUCUGAGCAUUUGA |
| hsa-miR-106a | AAAAGUGCUUACAGUGCAGGUA | UACCUGCACUGUAAGCACUUUU |
| hsa-miR-106b | UAAAGUGCUGACAGUGCAGAUA | UAUCUGCACUGUCAGCACUUUA |
| hsa-miR-107 | AGCAGCAUUGUACAGGGCUAUC | GAUAGCCCUGUACAAUGCUGCU |
| hsa-miR-10b | UACCCUGUAGAACCGAAUUUGU | ACAAAUUCGGUUCUACAGGGUA |
| hsa-miR-128b | UCACAGUGAACCGGUCUCUUUC | GAAAGAGACCGGUUCACUGUGA |
| hsa-miR-130b | CAGUGCAAUGAUGAAAGGGCAU | AUGCCCUUUCAUCAUUGCACUG |
| hsa-miR-140-3p | UACCACAGGGUAGAACCACGGA | UCCGUGGUUCUACCCUGUGGUA |
| hsa-miR-142-5p | CCCAUAAAGUAGAAAGCACUAC | GUAGUGCUUUCUACUUUAUGGG |
| hsa-miR-151-5p | UCGAGGAGCUCACAGUCUAGUA | UACUAGACUGUGAGCUCCUCGA |
| hsa-miR-155 | UUAAUGCUAAUCGUGAUAGGGG | CCCCUAUCACGAUUAGCAUUAA |
| hsa-miR-181a | AACAUUCAACGCUGUCGGUGAG | CUCACCGACAGCGUUGAAUGUU |
| hsa-miR-181b | AACAUUCAUUGCUGUCGGUGGG | CCCACCGACAGCAAUGAAUGUU |
| hsa-miR-181c | AACAUUCAACCUGUCGGUGAGU | ACUCACCGACAGGWGAAUGUU |
| hsa-miR-182 | UUUGGCAAUGGUAGAACUCACA | UGUGAGUUCUACCAUUGCCAAA |
| hsa-miR-183 | UAUGGCACUGGUAGAAUUCACU | AGUGAAUUCUACCAGUGCCAUA |
| hsa-miR-184 | UGGACGGAGAACUGAUAAGGGU | ACCCUUAUCAGUUCUCCGUCCA |
| hsa-miR-185 | UGGAGAGAAAGGCAGUUCCUGA | UCAGGAACUGCCUUUCUCUCCA |
| hsa-miR-186 | CAAAGAAUUCUCCUUUUGGGCU | AGCCCAAAAGGAGAAUUCUUUG |
| hsa-miR-187 | UCGUGUCUUGUGUUGCAGCCGG | CCGGCUGCAACACAAGACACGA |
| hsa-miR-188-3p | CUCCCACAUGCAGGGUUUGCAG | CUGCAAACCCUGCAUGUGGGAG |
| hsa-miR-188-5p | CAUCCCUUGCAUGGUGGAGGGU | ACCCUCCACCAUGCAAGGGAUG |
| hsa-miR-189 | GUGCCUACUGAGCUGAUAUCAG | CUGAUAUCAGCUCAGUAGGCAC |
| hsa-miR-190 | UGAUAUGUUUGAUAUAUUAGGU | ACCUAAUAUAUCAAACAUAUCA |
| hsa-miR-191 | CAACGGAAUCCCAAAAGCAGCU | AGCUGCUUUUGGGAUUCCGUUG |
| hsa-miR-192 | CUGACCUAUGAAUUGACAGCCA | UGGCUGUCAAUUCAUAGGUCAG |
| hsa-miR-193-3p | AACUGGCCUACAAAGUCCCAGU | ACUGGGACUUUGUAGGCCAGUU |
| hsa-miR-193-5p | UGGGUCUUUGCGGGCAAGAUGA | UCAUCUUGCCCGCAAAGACCCA |
| hsa-miR-194 | UGUAACAGCAACUCCAUGUGGA | UCCACAUGGAGUUGCUGUUACA |
| hsa-miR-195 | UAGCAGCACAGAAAUAUUGGCA | UGCCAAUAUUUCUGUGCUGCUA |
| hsa-miR-196 | UAGGUAGUUUCAUGUUGUUGGG | CCCAACAACAUGAAACUACCUA |
| hsa-miR-197 | UUCACCACCUUCUCCACCCAGC | GCUGGGUGGAGAAGGUGGUGAA |
| hsa-miR-198 | GGUCCAGAGGGGAGAUAGGUUC | GAACCUAUCUCCCCUCUGGACC |
| hsa-miR-199a-3p | ACAGUAGUCUGCACAUUGGUUA | UAACCAAUGUGCAGACUACUGU |
| hsa-miR-199a-5p | CCCAGUGUUCAGACUACCUGUU | AACAGGUAGUCUGAACACUGGG |
| hsa-miR-199b | CCCAGUGUUUAGACUAUCUGUU | AACAGAUAGUCUAAACACUGGG |
| hsa-miR-200a | UAACACUGUCUGGUAACGAUGU | ACAUCGUUACCAGACAGUGUUA |
| hsa-miR-200b | CUCUAAUACUGCCUGGUAAUGA | UCAUUACCAGGCAGUAUUAGAG |
| hsa-miR-200c | AAUACUGCCGGGUAAUGAUGGA | UCCAUCAUUACCCGGCAGUAUU |
| hsa-miR-203 | GUGAAAUGUUUAGGACCACUAG | CUAGUGGUCCUAAACAUUUCAC |
| hsa-miR-204 | UUCCCUUUGUCAUCCUAUGCCU | AGGCAUAGGAUGACAAAGGGAA |
| hsa-miR-205 | UCCUUCAUUCCACCGGAGUCUG | CAGACUCCGGUGGAAUGAAGGA |
| hsa-miR-206 | UGGAAUGUAAGGAAGUGUGUGG | CCACACACUUCCUUACAUUCCA |
| hsa-miR-208 | AUAAGACGAGCAAAAAGCUUGU | ACAAGCUUUUUGCUCGUCUUAU |
| hsa-miR-210 | CUGUGCGUGUGACAGCGGCUGA | UCAGCCGCUGUCACACGCACAG |
| hsa-miR-211 | UUCCCUUUGUCAUCCUUCGCCU | AGGCGAAGGAUGACAAAGGGAA |
| hsa-miR-212 | UAACAGUCUCCAGUCACGGCCA | UGGCCGUGACUGGAGACUGUUA |
| hsa-miR-213 | ACCAUCGACCGUUGAUUGUACC | GGUACAAUCAACGGUCGAUGGU |
| hsa-miR-214 | ACAGCAGGCACAGACAGGCAGU | ACUGCCUGUCUGUGCCUGCUGU |
| hsa-miR-215 | AUGACCUAUGAAUUGACAGACA | UGUCUGUCAAUUCAUAGGUCAU |
| hsa-miR-216 | UAAUCUCAGCUGGCAACUGUGA | UCACAGUUGCCAGCUGAGAUUA |
| hsa-miR-217 | UACUGCAUCAGGAACUGAUUGG | CCAAUCAGUUCCUGAUGCAGUA |
| hsa-miR-218 | UUGUGCUUGAUCUAACCAUGUG | CACAUGGUUAGAUCAAGCACAA |
| hsa-miR-219 | UGAUUGUCCAAACGCAAUUCUU | AAGAAUUGCGUUUGGACAAUCA |
| hsa-miR-220 | CCACACCGUAUCUGACACUUUG | CAAAGUGUCAGAUACGGUGUGG |
| hsa-miR-221 | AGCUACAUUGUCUGCUGGGUUU | AAACCCAGCAGACAAUGUAGCU |
| hsa-miR-222 | AGCUACAUCUGGCUACUGGGUC | GACCCAGUAGCCAGAUGUAGCU |
| hsa-miR-223 | UGUCAGUUUGUCAAAUACCCCA | UGGGGUAUUUGACAAACUGACA |
| hsa-miR-224 | CAAGUCACUAGUGGUUCCGUUU | AAACGGAACCACUAGUGACUUG |
| hsa-miR-28-5p | AAGGAGCUCACAGUCUAUUGAG | CUCAAUAGACUGUGAGCUCCUU |
| hsa-miR-290 | CUCAAACUGUGGGGGCACUUUC | GAAAGUGCCCCCACAGUUUGAG |
| hsa-miR-296 | AGGGCCCCCCCUCAAUCCUGUU | AACAGGAUUGAGGGGGGGCCCU |
| hsa-miR-299 | UGGUUUACCGUCCCACAUACAU | AUGUAUGUGGGACGGUAAACCA |
| hsa-miR-301 | CAGUGCAAUAGUAUUGUCAAAG | CUUUGACAAUACUAUUGCACUG |
| hsa-miR-302 | UAAGUGCWCCAUGUUUUGGUG | CACCAAAACAUGGAAGCACUUA |
| hsa-miR-30e | UGUAAACAUCCUUGACUGGAAG | CUUCCAGUCAAGGAUGUUUACA |
| hsa-miR-320 | AAAAGCUGGGUUGAGAGGGCGA | UCGCCCUCUCAACCCAGCUUUU |
| hsa-miR-321 | UAAGCCAGGGAUUGUGGGUUCG | CGAACCCACAAUCCCUGGCUUA |
| hsa-miR-322 | AAACAUGAAUUGCUGCUGUAUC | GAUACAGCAGCAAUUCAUGUUU |
| hsa-miR-323 | GCACAUUACACGGUCGACCUCU | AGAGGUCGACCGUGUAAUGUGC |
| hsa-miR-324-3p | CCACUGCCCCAGGUGCUGCUGG | CCAGCAGCACCUGGGGCAGUGG |
| hsa-miR-324-5p | CGCAUCCCCUAGGGCAUUGGUG | CACCAAUGCCCUAGGGGAUGCG |
| hsa-miR-326 | CCUCUGGGCCCUUCCUCCAGCC | GGCUGGAGGAAGGGCCCAGAGG |
| hsa-miR-328 | CUGGCCCUCUCUGCCCUUCCGU | ACGGAAGGGCAGAGAGGGCCAG |
| hsa-miR-329 | AACACACCCAGCUAACCUUUUU | AAAAAGGUUAGCUGGGUGUGUU |
| hsa-miR-34a | UGGCAGUGUCUUAGCUGGUUGU | ACAACCAGCUAAGACACUGCCA |
| hsa-miR-34b | AGGCAGUGUCAUUAGCUGAUUG | CAAUCAGCUAAUGACACUGCCU |
| hsa-miR-34c | AGGCAGUGUAGUUAGCUGAUUG | CAAUCAGCUAACUACACUGCCU |
| hsa-miR-92 | UAWGCACUUGUCCCGGCCUGU | ACAGGCCGGGACAAGUGCAAUA |
| hsa-miR-93 | AAAGUGCUGUUCGUGCAGGUAG | CUACCUGCACGAACAGCACUUU |
| hsa-miR-95 | UUCAACGGGUAUUUAUUGAGCA | UGCUCAAUAAAUACCCGUUGAA |
| hsa-miR-96 | UUUGGCACUAGCACAUUUUUGC | GCAAAAAUGUGCUAGUGCCAAA |
| hsa-miR-98 | UGAGGUAGUAAGUUGUAUUGUU | AACAAUACAACUUACUACCUCA |
| mmu-miR-106a | CAAAGUGCUAACAGUGCAGGUA | UACCUGCACUGUUAGCACUUUG |
| mmu-miR-10b | CCCUGUAGAACCGAAUUUGUGU | ACACAAAUUCGGUUCUACAGGG |
| mmu-miR-135b | UAUGGCUUUUCAUUCCUAUGUG | CACAUAGGAAUGAAAAGCCAUA |
| mmu-miR-148b | UCAGUGCAUCACAGAACUUUGU | ACAAAGUUCUGUGAUGCACUGA |
| mmu-miR-151-3p | CUAGACUGAGGCUCCUUGAGGA | UCCUCAAGGAGCCUCAGUCUAG |
| mmu-miR-155 | UUAAUGCUAAUUGUGAUAGGGG | CCCCUAUCACAAUUAGCAUUAA |
| mmu-miR-199b | CCCAGUGUUUAGACUACCUGUU | AACAGGUAGUCUAAACACUGGG |
| mmu-miR-200b | UAAUACUGCCUGGUAAUGAUGA | UCAUCAUUACCAGGCAGUAUUA |
| mmu-miR-203 | UGAAAUGUUUAGGACCACUAGA | UCUAGUGGUCCUAAACAUUUCA |
| mmu-miR-211 | UUCCCUUUGUCAUCCUUUGCCU | AGGCAAAGGAUGACAAAGGGAA |
| mmu-miR-217 | UACUGCAUCAGGAACUGACUGG | CCAGUCAGUUCCUGAUGCAGUA |
| mmu-miR-224 | UAAGUCACUAGUGGUUCCGUUU | AAACGGAACCACUAGUGACUUA |
| mmu-miR-28-3p | CACUAGAUUGUGAGCUGCUGGA | UCCAGCAGCUCACAAUCUAGUG |
| mmu-miR-290 | CUCAAACUAUGGGGGCACUUUU | AAAAGUGCCCCCAUAGUUUGAG |
| mmu-miR-291-3p | AAAGUGCUUCCACUUUGUGUGC | GCACACAAAGUGGAAGCACUUU |
| mmu-miR-291-5p | CAUCAAAGUGGAGGCCCUCUCU | AGAGAGGGCCUCCACUUUGAUG |
| mmu-miR-292-3p | AAGUGCCGCCAGGUUUUGAGUG | CACUCAAAACCUGGCGGCACUU |
| mmu-miR-292-5p | ACUCAAACUGGGGGCUCUUUUG | CAAAAGAGCCCCCAGUUUGAGU |
| mmu-miR-293 | AGUGCCGCAGAGUUUGUAGUGU | ACACUACAAACUCUGCGGCACU |
| mmu-miR-294 | . AAAGUGCUUCCCUUUUGUGUGU | ACACACAAAAGGGAAGCACUUU |
| mmu-miR-295 | AAAGUGCUACUACUUUUGAGUC | GACUCAAAAGUAGUAGCACUUU |
| mmu-miR-297 | AUGUAUGUGUGCAUGUGCAUGU | ACAUGCACAUGCACACAUACAU |
| mmu-miR-298 | GGCAGAGGAGGGCUGUUCUUCC | GGAAGAACAGCCCUCCUCUGCC |
| mmu-miR-300 | UAUGCAAGGGCAAGCUCUCUUC | GAAGAGAGCUUGCCCUUGCAUA |
| mmu-miR-31 | AGGCAAGAUGCUGGCAUAGCUG | CAGCUAUGCCAGCAUCUUGCCU |
| mmu-miR-322 | AAACAUGAAGCGCUGCAACACC | GGUGUUGCAGCGCUUCAUGUUU |
| mmu-miR-325 | CCUAGUAGGUGCUCAGUAAGUG | CACUUACUGAGCACCUACUAGG |
| mmu-miR-326 | CCUCUGGGCCCUUCCUCCAGUC | GACUGGAGGAAGGGCCCAGAGG |
| mmu-miR-330 | GCAAAGCACAGGGCCUGCAGAG | CUCUGCAGGCCCUGUGCUUUGC |
| mmu-miR-331 | GCCCCUGGGCCUAUCCUAGAAC | GUUCUAGGAUAGGCCCAGGGGC |
| mmu-miR-337 | UUCAGCUCCUAUAUGAUGCCUU | AAGGCAUCAUAUAGGAGCUGAA |
| mmu-miR- 338 | UCCAGCAUCAGUGAUUUUGUUG | CAACAAAAUCUCUGAUGCUGGA |
| mmu-miR-339 | UCCCUGUCCUCCAGGAGCUCAC | GUGAGCUCCUGGAGGACAGGGA |
| mmu-miR-340 | UCCGUCUCAGUUACUUUAUAGC | GCUAUAAAGUAACUGAGACGGA |
| mmu-miR-341 | UCGAUCGGUCGGUCGGUCAGUC | GACUGACCGACCGACCGAUCGA |
| mmu-miR-342 | UCUCACACAGAAAUCGCACCCG | CGGGUGCGAUUUCUGUGUGAGA |
| mmu-miR-344 | UGAUCUAGCCAAAGCCUGACUG | CAGUCAGGCUUUGGCUAGAUCA |
| mmu-miR-345 | UGCUGACCCCUAGUCCAGUGCU | AGCACUGGACUAGGGGUCAGCA |
| mmu-miR-346 | UGUCUGCCCGAGUGCCUGCCUC | GAGGCAGGCACUCGGGCAGACA |
| mmu-miR-34b | UAGGCAGUGUAAUUAGCUGAUU | AAUCAGCUAAUUACACUGCCUA |
| mmu-miR-350 | UUCACAAAGCCCAUACACUUUC | GAAAGUGUAUGGGCUUUGUGAA |
| mu-miR-351 | UCCCUGAGGAGCCCUUUGAGCC | GGCUCAAAGGGCUCCUCAGGGA |
| mmu-miR-7b | UGGAAGACUUGUGAUUUUGUUG | CAACAAAAUCACAAGUCUUCCA |
| mmu-miR-92 | UAUUGCACUUGUCCCGGCCUGA | UCAGGCCGGGACAAGUGCAAUA |
| mmu-miR-93 | CAAAGUGCUGUUCGUGCAGGUA | UACCUGCACGAACAGCACUUUG |
| rno-miR-327 | CCUUGAGGGGCAUGAGGGUAGU | ACUACCCUCAUGCCCCUCAAGG |
| rno-miR-333 | GUGGUGUGCUAGUUACUUUUGG | CCAAAAGUAACUAGCACACCAC |
| rno-miR-335 | UCAAGAGCAAUAACGAAAAAUG | CAUUUUUCGUUAUUGCUCUUGA |
| rno-miR-336 | UCACCCWCCAUAUCUAGUCUC | GAGACUAGAUAUGGAAGGGUGA |
| rno-miR-343 | UCUCCCUCCGUGUGCCCAGUAU | AUACUGGGCACACGGAGGGAGA |
| rno-miR-347 | UGUCCCUCUGGGUCGCCCAGCU | AGCUGGGCGACCCAGAGGGACA |
| rno-miR-349 | CAGCCCUGCUGUCUUAACCUCU | AGAGGUUAAGACAGCAGGGCUG |
| rno-miR-352 | AGAGUAGUAGGUUGCAUAGUAC | GUACUAUGCAACCUACUACUCU |

**Table 2: Novel Human microRNA and anti-microRNA sequences.**

| microRNA name | microRNA sequence (5' to 3') | Anti-microRNA molecule sequence (5' to 3') |
|---|---|---|
| hsa-miR-361 | UUAUCAGAAUCUCCAGGGGUAC | GUACCCCUGGAGAUUCUGAUAA |
| hsa-miR-362 | AAUCCUUGGAACCUAGGUGUGA | UCACACCUAGGUUCCAAGGAUU |
| hsa-miR-363 | AUUGCACGGUAUCCAUCUGUAA | UUACAGAUGGAUACCGUGCAAU |
| hsa-miR-364. | CGGCGGGGACGGCGAUUGGUCC | GGACCAAUCGCCGUCCCCGCCG |
| hsa-miR-365 | UAAUGCCCCUAAAAAUCCUUAU | AUAAGGAUUUUUAGGGGCAUUA |
| hsa-miR-366 | UAACUGGUUGAACAACUGAACC | GGUUCAGUUGUUCAACCAGUUA |

**Table 3: C. elegans microRNA and anti-microRNA sequences.**

| microRNA name | microRNA sequence (5' to 3') | Anti-microRNA molecule sequence (5' to 3') |
|---|---|---|
| Cel-let-7 | UGAGGUAGUAGGUUGUAUAGUU | AACUAUACAACCUACUACCUCA |
| Cel-lin-4 | UCCCUGAGACCUCAAGUGUGAG | CUCACACUUGAGGUCUCAGGGA |
| Cel-miR-1 | UGGAAUGUAAAGAAGUAUGUAG | CUACAUACUUCUUUACAUUCCA |
| Cel-miR-2 | UAUCACAGCCAGCUUUGAUGUG | CACAUCAAAGCUGGCUGUGAUA |
| Cel-miR-34 | AGGCAGUGUGGUUAGCUGGUUG | CAACCAGCUAACCACACUGCCU |
| Cel-miR-35 | UCACCGGGUGGAAACUAGCAGU | ACUGCUAGUUUCCACCCGGUGA |
| Cel-miR-36 | UCACCGGGUGAAAAUUCGCAUG | CAUGCGAAUUUUCACCCGGUGA |
| Cel-miR-37 | UCACCGGGUGAACACUUGCAGU | ACUGCAAGUGUUCACCCGGUGA |
| Cel-miR-38 | UCACCGGGAGAAAAACUGGAGU | ACUCCAGUUUUUCUCCCGGUGA |
| Cel-miR-39 | UCACCGGGUGUAAAUCAGCUUG | CAAGCUGAUUUACACCCGGUGA |
| Cel-miR-40 | UCACCGGGUGUACAUCAGCUAA | UUAGCUGAUGUACACCCGGUGA |
| Cel-miR-41 | UCACCGGGUGAAAAAUCACCUA | UAGGUGAUUUUUCACCCGGUGA |
| Cel-miR-42 | CACCGGGUUAACAUCUACAGAG | CUCUGUAGAUGUUAACCCGGUG |
| Cel-miR-43 | UAUCACAGUUUACUUGCUGUCG | CGACAGCAAGUAAACUGUGAUA |
| Cel-miR-44 | UGACUAGAGACACAUUCAGCUU | AAGCUGAAUGUGUCUCUAGUCA |
| Cel-miR-45 | UGACUAGAGACACAUUCAGCUU | AAGCUGAAUGUGUCUCUAGUCA |
| Cel-miR-46 | UGUCAUGGAGUCGCUCUCUUCA | UGAAGAGAGCGACUCCAUGACA |
| Cel-miR-47 | UGUCAUGGAGGCGCUCUCUUCA | UGAAGAGAGCGCCUCCAUGACA |
| Cel-miR-48 | UGAGGUAGGCUCAGUAGAUGCG | CGCAUCUACUGAGCCUACCUCA |
| Cel-miR-49 | AAGCACCACGAGAAGCUGCAGA | UCUGCAGCUUCUCGUGGUGCUU |
| Cel-miR-50 | UGAUAUGUCUGGUAUUCUUGGG | CCCAAGAAUACCAGACAUAUCA |
| Cel-miR-51 | UACCCGUAGCUCCUAUCCAUGU | ACAUGGAUAGGAGCUACGGGUA |
| Cel-miR-52 | CACCCGUACAUAUGUUUCCGUG | CACGGAAACAUAUGUACGGGUG |
| Cel-miR-53 | CACCCGUACAUUUGUUUCCGUG | CACGGAAACAAAUGUACGGGUG |
| Cel-miR-54 | UACCCGUAAUCUUCAUAAUCCG | CGGAUUAUGAAGAUUACGGGUA |
| Cel-miR-55 | UACCCGUAUAAGUUUCUGCUGA | UCAGCAGAAACUUAUACGGGUA |
| Cel-miR-56 | UACCCGUAAUGUUUCCGCUGAG | CUCAGCGGAAACAUUACGGGUA |
| Cel-miR-57 | UACCCUGUAGAUCGAGCUGUGU | ACACAGCUCGAUCUACAGGGUA |
| Cel-miR-58 | UGAGAUCGUUCAGUACGGCAAU | AUUGCCGUACUGAACGAUCUCA |
| Cel-miR-59 | UCGAAUCGUUUAUCAGGAUGAU | AUCAUCCUGAUAAACGAUUCGA |
| Cel-miR-60 | UAUUAUGCACAUUUUCUAGUUC | GAACUAGAAAAUGUGCAUAAUA |
| Cel-miR-61 | UGACUAGAACCGUUACUCAUCU | AGAUGAGUAACGGUUCUAGUCA |
| Cel-miR-62 | UGAUAUGUAAUCUAGCUUACAG | CUGUAAGCUAGAUUACAUAUCA |
| Cel-miR-63 | AUGACACUGAAGCGAGUUGGAA | UUCCAACUCGCUUCAGUGUCAU |
| Cel-miR-64 | UAUGACACUGAAGCGUUACCGA | UCGGUAACGCUUCAGUGUCAUA |
| Cel-miR-65 | UAUGACACUGAAGCGUAACCGA | UCGGUUACGCUUCAGUGUCAUA |
| Cel-miR-66 | CAUGACACUGAUUAGGGAUGUG | CACAUCCCUAAUCAGUGUCAUG |
| Cel-miR-67 | UCACAACCUCCUAGAAAGAGUA | UACUCUUUCUAGGAGGUUGUGA |
| Cel-miR-68 | UCGAAGACUCAAAAGUGUAGAC | GUCUACACUUUUGAGUCUUCGA |
| Cel-miR-69 | UCGAAAAUUAAAAAGUGUAGAA | UUCUACACUUUUUAAUUUUCGA |
| Cel-miR-70 | UAAUACGUCGUUGGUGUUUCCA | UGGAAACACCAACGACGUAUUA |
| Cel-miR-71 | UGAAAGACAUGGGUAGUGAACG | CGUUCACUACCCAUGUCUUUCA |
| Cel-miR-72 | AGGCAAGAUGUUGGCAUAGCUG | CAGCUAUGCCAACAUCUUGCCU |
| Cel-miR-73 | UGGCAAGAUGUAGGCAGUUCAG | CUGAACUGCCUACAUCUUGCCA |
| Cel-miR-74 | UGGCAAGAAAUGGCAGUCUACA | UGUAGACUGCCAUUUCUUGCCA |
| Cel-miR-75 | UUAAAGCUACCAACCGGCUUCA | UGAAGCCGGUUGGUAGCUUUAA |
| Cel-miR-76 | UUCGUUGUUGAUGAAGCCUUGA | UCAAGGCUUCAUCAACAACGAA |
| Cel-miR-77 | UUCAUCAGGCCAUAGCUGUCCA | UGGACAGCUAUGGCCUGAUGAA |
| Cel-miR-78 | UGGAGGCCUGGUUGUUUGUGCU | AGCACAAACAACCAGGCCUCCA |
| Cel-miR-79 | AUAAAGCUAGGUUACCAAAGCU | AGCUUUGGUAACCUAGCUUUAU |
| Cel-miR-227 | AGCUUUCGACAUGAUUCUGAAC | GUUCAGAAUCAUGUCGAAAGCU |
| Cel-miR-80 | UGAGAUCAUUAGUUGAAAGCCG | CGGCUUUCAACUAAUGAUCUCA |
| Cel-miR-81 | UGAGAUCAUCGUGAAAGCUAGU | ACUAGCUUUCACGAUGAUCUCA |
| Cel-miR-82 | UGAGAUCAUCGUGAAAGCCAGU | ACUGGCUUUCACGAUGAUCUCA |
| Cel-miR-83 | UAGCACCAUAUAAAUUCAGUAA | UUACUGAAUUUAUAUGGUGCUA |
| Cel-miR-84 | UGAGGUAGUAUGUAAUAUUGUA | UACAAUAUUACAUACUACCUCA |
| Cel-miR-85 | UACAAAGUAUUUGAAAAGUCGU | ACGACUUUUCAAAUACUUUGUA |
| Cel-miR-86 | UAAGUGAAUGCUUUGCCACAGU | ACUGUGGCAAAGCAUUCACUUA |
| Cel-miR-87 | GUGAGCAAAGUUUCAGGUGUGC | GCACACCUGAAACUUUGCUCAC |
| Cel-miR-90 | UGAUAUGUUGUUUGAAUGCCCC | GGGGCAUUCAAACAACAUAUCA |
| Cel-miR-124 | UAAGGCACGCGGUGAAUGCCAC | GUGGCAUUCACCGCGUGCCUUA |
| Cel-miR-228 | AAUGGCACUGCAUGAAUUCACG | CGUGAAUUCAUGCAGUGCCAUU |
| Cel-miR-229 | AAUGACACUGGUUAUCUUUUCC | GGAAAAGAUAACCAGUGUCAUU |
| Cel-miR-230 | GUAUUAGUUGUGCGACCAGGAG | CUCCUGGUCGCACAACUAAUAC |
| Cel-miR-231 | UAAGCUCGUGAUCAACAGGCAG | CUGCCUGUUGAUCACGAGCUUA |
| Cel-miR-232 | UAAAUGCAUCUUAACUGCGGUG | CACCGCAGUUAAGAUGCAUUUA |
| Cel-miR-233 | UUGAGCAAUGCGCAUGUGCGGG | CCCGCACAUGCGCAUUGCUCAA |
| Cel-miR-234 | UUAUUGCUCGAGAAUACCCUUU | AAAGGGUAUUCUCGAGCAAUAA |
| Cel-miR-235 | UAUUGCACUCUCCCCGGCCUGA | UCAGGCCGGGGAGAGUGCAAUA |
| Cel-miR-236 | UAAUACUGUCAGGUAAUGACGC | GCGUCAUUACCUGACAGUAUUA |
| Cel-miR-237 | UCCCUGAGAAUUCUCGAACAGC | GCUGUUCGAGAAUUCUCAGGGA |
| Cel-miR-238 | UUUGUACUCCGAUGCCAUUCAG | CUGAAUGGCAUCGGAGUACAAA |
| Cel-miR-239a | UUUGUACUACACAUAGGUACUG | CAGUACCUAUGUGUAGUACAAA |
| Cel-miR-239b | UUUGUACUACACAAAAGUACUG | CAGUACUUUUGUGUAGUACAAA |
| Cel-miR-240 | UACUGGCCCCCAAAUCUUCGCU | AGCGAAGAUUUGGGGGCCAGUA |
| Cel-miR-241 | UGAGGUAGGUGCGAGAAAUGAC | GUCAUUUCUCGCACCUACCUCA |
| Cel-miR-242 | UUGCGUAGGCCUUUGCUUCGAG | CUCGAAGCAAAGGCCUACGCAA |
| Cel-miR-243 | CGGUACGAUCGCGGCGGGAUAU | AUAUCCCGCCGCGAUCGUACCG |
| Cel-miR-244 | UCUUUGGUUGUACAAAGUGGUA | UACCACUUUGUACAACCAAAGA |
| Cel-miR-245 | AUUGGUCCCCUCCAAGUAGCUC | GAGCUACUUGGAGGGGACCAAU |
| Cel-miR-246 | UUACAUGUUUCGGGUAGGAGCU | AGCUCCUACCCGAAACAUGUAA |
| Cel-miR-247 | UGACUAGAGCCUAUUCUCUUCU | AGAAGAGAAUAGGCUCUAGUCA |
| Cel-miR-248 | UACACGUGCACGGAUAACGCUC | GAGCGUUAUCCGUGCACGUGUA |
| Cel-miR-249 | UCACAGGACUUUUGAGCGUUGC | GCAACGCUCAAAAGUCCUGUGA |
| Cel-miR-250 | UCACAGUCAACUGUUGGCAUGG | CCAUGCCAACAGUUGACUGUGA |
| Cel-miR-251 | UUAAGUAGUGGUGCCGCUCUUA | UAAGAGCGGCACCACUACUUAA |
| Cel-miR-252 | UAAGUAGUAGUGCCGCAGGUAA | UUACCUGCGGCACUACUACUUA |
| Cel-miR-253 | CACACCUCACUAACACUGACCA | UGGUCAGUGWAGUGAGGUGUG |
| Cel-miR-254 | UGCAAAUCUUUCGCGACUGUAG | CUACAGUCGCGAAAGAUUUGCA |
| Cel-miR-256 | UGGAAUGCAUAGAAGACUGUAC | GUACAGUCUUCUAUGCAUUCCA |
| Cel-miR-257 | GAGUAUCAGGAGUACCCAGUGA | UCACUGGGUACUCCUGAUACUC |
| Cel-miR-258 | GGUUUUGAGAGGAAUCCUUUUA | UAAAAGGAUUCCUCUCAAAACC |
| Cel-miR-259 | AGUAAAUCUCAUCCUAAUCUGG | CCAGAUUAGGAUGAGAUUUACU |
| Cel-miR-260 | GUGAUGUCGAACUCUUGUAGGA | UCCUACAAGAGUUCGACAUCAC. |
| Cel-miR-261 | UAGCUUUUUAGUUUUCACGGUG | CACCGUGAAAACUAAAAAGCUA |
| Cel-miR-262 | GUUUCUCGAUGUUUUCUGAUAC | GUAUCAGAAAACAUCGAGAAAC |
| Cel-miR-264 | GGCGGGUGGUUGUUGUUAUGGG | CCCAUAACAACAACCACCCGCC |
| Cel-miR-265 | UGAGGGAGGAAGGGUGGUAUUU | AAAUACCACCCUUCCUCCCUCA |
| Cel-miR-266 | AGGCAAGACUUUGGCAAAGCUU | AAGCUUUGCCAAAGUCUUGCCU |
| Cel-miR-267 | CCCGUGAAGUGUCUGCUGCAAU | AUUGCAGCAGACACUUCACGGG |
| Cel-miR-268 | GGCAAGAAUUAGAAGCAGUUUG | CAAACUGCUUCUAAUUCUUGCC |
| Cel-miR-269 | GGCAAGACUCUGGCAAAACUUG | CAAGUUUUGCCAGAGUCUUGCC |
| Cel-miR-270 | GGCAUGAUGUAGCAGUGGAGAU | AUCUCCACUGCUACAUCAUGCC |
| Cel-miR-271 | UCGCCGGGUGGGAAAGCAUUCG | CGAAUGCUUUCCCACCCGGCGA |
| Cel-miR-272 | UGUAGGCAUGGGUGUUUGGAAG | CUUCCAAACACCCAUGCCUACA |
| Cel-miR-273 | UGCCCGUACUGUGUCGGCUGCU | AGCAGCCGACACAGUACGGGCA |

**Table 4: Drosophila microRNA and anti-microRNA sequences.**

| microRNA name | microRNA sequence (5' to 3') | Anti-microRNA molecule sequence (5' to 3') |
|---|---|---|
| Dme-miR-263a | GUUAAUGGCACUGGAAGAAUUC | GAAUUCUUCCAGUGCCAUUAAC |
| Dme-miR-184 | UGGACGGAGAACUGAUAAGGGC | GCCCUUAUCAGUUCUCCGUCCA |
| Dme-miR-274 | UUUUGUGACCGACACUAACGGG | CCCGUUAGUGUCGGUCACAAAA |
| Dme-miR-275 | UCAGGUACCUGAAGUAGCGCGC | GCGCGCUACUUCAGGUACCUGA |
| Dme-miR-92a | CAUUGCACUUGUCCCGGCCUAU | AUAGGCCGGGACAAGUGCAAUG |
| Dme-miR-219 | UGAUUGUCCAAACGCAAUUCUU | AAGAAUUGCGUUUGGACAAUCA |
| Dme-miR-276a | UAGGAACUUCAUACCGUGCUCU | AGAGCACGGUAUGAAGUUCCUA |
| Dme-miR-277 | UAAAUGCACUAUCUGGUACGAC | GUCGUACCAGAUAGUGCAUUUA |
| Dme-miR-278 | UCGGUGGGACUUUCGUCCGUUU | AAACGGACGAAAGUCCCACCGA |
| Dme-miR-133 | UUGGUCCCCUUCAACCAGCUGU | ACAGCUGGUUGAAGGGGACCAA |
| ,Dme-miR-279 | UGACUAGAUCCACACUCAUUAA | UUAAUGAGUGUGGAUCUAGUCA |
| Dme-miR-33 | AGGUGCAUUGUAGUCGCAUUGU | ACAAUGCGACUACAAUGCACCU |
| Dme-miR-280 | UGUAUUUACGUUGCAUAUGAAA | UUUCAUAUGCAACGUAAAUACA |
| Dme-miR-281 | UGUCAUGGAAUUGCUCUCUUUG | CAAAGAGAGCAAUUCCAUGACA |
| Dme-miR-282 | AAUCUAGCCUCUACUAGGCUUU | AAAGCCUAGUAGAGGCUAGAUU |
| Dme-miR-283 | UAAAUAUCAGCUGGUAAUUCUG | CAGAAUUACCAGCUGAUAUUUA |
| Dme-miR-284 | UGAAGUCAGCAACUUGAUUCCA | UGGAAUCAAGUUGCUGACUUCA |
| Dme-miR-34 | UGGCAGUGUGGUUAGCUGGUUG | CAACCAGCUAACCACACUGCCA |
| Dme-miR-124 | UAAGGCACGCGGUGAAUGCCAA | UUGGCAUUCACCGCGUGCCUUA |
| Dme-miR-79 | UAAAGCUAGAUUACCAAAGCAU | AUGCUUUGGUAAUCUAGCUUUA |
| Dme-miR-276b | UAGGAACUUAAUACCGUGCUCU | AGAGCACGGUAUUAAGUUCCUA |
| Dme-miR-210 | UUGUGCGUGUGACAGCGGCUAU | AUAGCCGCUGUCACACGCACAA |
| Dme-miR-285 | UAGCACCAUUCGAAAUCAGUGC | GCACUGAUUUCGAAUGGUGCUA |
| Dme-miR-100 | AACCCGUAAAUCCGAACUUGUG | CACAAGUUCGGAUUUACGGGUU |
| Dme-miR-92b | AAUUGCACUAGUCCCGGCCUGC | GCAGGCCGGGACUAGUGCAAUU |
| Dme-miR-286 | UGACUAGACCGAACACUCGUGC | GCACGAGUGUUCGGUCUAGUCA |
| Dme-miR-287 | UGUGUUGAAAAUCGUUUGCACG | CGUGCAAACGAUUUUCAACACA |
| Dme-miR-87 | UUGAGCAAAAUUUCAGGUGUGU | ACACACCUGAAAUUUUGCUCAA |
| Dme-miR-263b | CUUGGCACUGGGAGAAUUCACA | UGUGAAUUCUCCCAGUGCCAAG |
| Dme-miR-288 | UUUCAUGUCGAUUUCAUUUCAU | AUGAAAUGAAAUCGACAUGAAA |
| Dme-miR-289 | UAAAUAUUUAAGUGGAGCCUGC | GCAGGCUCCACUUAAAUAUUUA |
| Dme-bantam | UGAGAUCAUUUUGAAAGCUGAU | AUCAGCUUUCAAAAUGAUCUCA |
| Dme-miR-303 | UUUAGGUUUCACAGGAAACUGG | CCAGUUUCCUGUGAAACCUAAA |
| Dme-miR-31b | UGGCAAGAUGUCGGAAUAGCUG | CAGCUAUUCCGACAUCUUGCCA |
| Dme-miR-304 | UAAUCUCAAUUUGUAAAUGUGA | UCACAUUUACAAAUUGAGAUUA |
| Dme-miR-305 | AUUGUACUUCAUCAGGUGCUCU | AGAGCACCUGAUGAAGUACAAU |
| Dme-miR-9c | UCUUUGGUAUUCUAGCUGUAGA | UCUACAGCUAGAAUACCAAAGA |
| Dme-miR-306 | UCAGGUACUUAGUGACUCUCAA | UUGAGAGUCACUAAGUACCUGA |
| Dme-miR-9b | UCUUUGGUGAUUUUAGCUGUAU | AUACAGCUAAAAUCACCAAAGA |
| Dme-miR-125 | UCCCUGAGACCCUAACUUGUGA | UCACAAGUUAGGGUCUCAGGGA |
| Dme-miR-307 | UCACAACCUCCUUGAGUGAGCG | CGCUCACUCAAGGAGGUUGUGA |
| Dme-miR-308 | AAUCACAGGAUUAUACUGUGAG | CUCACAGUAUAAUCCUGUGAUU |
| dme-miR-31a | UGGCAAGAUGUCGGCAUAGCUG | CAGCUAUGCCGACAUCUUGCCA |
| dme-miR-309 | GCACUGGGUAAAGUUUGUCCUA | UAGGACAAACUUUACCCAGUGC |
| dme-miR-310 | UAUUGCACACUUCCCGGCCUUU | AAAGGCCGGGAAGUGUGCAAUA |
| dme-miR-311 | UAUUGCACAUUCACCGGCCUGA | UCAGGCCGGUGAAUGUGCAAUA |
| dme-miR-312 | UAUUGCACUUGAGACGGCCUGA | UCAGGCCGUCUCAAGUGCAAUA |
| dme-miR-313 | UAUUGCACUUUUCACAGCCCGA | UCGGGCUGUGAAAAGUGCAAUA |
| dme-miR-314 | UAUUCGAGCCAAUAAGUUCGG | CCGAACUUAUUGGCUCGAAUA |
| dme-miR-315 | UUUUGAUUGUUGCUCAGAAAGC | GCUUUCUGAGCAACAAUCAAAA |
| dme-miR-316 | UGUCUUUUUCCGCUUACUGGCG | CGCCAGUAAGCGGAAAA.AGACA |
| dme-miR-317 | UGAACACAGCUGGUGGUAUCCA | UGGAUACCACCAGCUGUGUUCA |
| dme-miR-318 | UCACUGGGCUUUGUUUAUCUCA | UGAGAUAAACAAAGCCCAGUGA |
| dme-miR-2c | UAUCACAGCCAGCUUUGAUGGG | CCCAUCAAAGCUGGCUGUGAUA |
| Dme-miR-iab45p | ACGUAUACUGAAUGUAUCCUGA | UCAGGAUACAUUCAGUAUACGU |
| Dme-miR-iab43p | CGGUAUACCUUCAGUAUACGUA | UACGUAUACUGAAGGUAUACCG |

### EXAMPLES

### Example 1: Materials and Methods

### Oligonucleotide synthesis

MiR-21 were synthesized using 5'-silyl, 2'-ACE phosphoramidites (Dharmacon, Lafayette, CO, USA) on 0.2 µmol synthesis columns using a modified ABI 394 synthesizer (Foster City, CA, USA) (Scaringe, Methods Enzymol. 317, 3-18 (2001) and Scaringe, Methods 23, 206-217 (2001)). The phosphate methyl group was removed by flushing the column with 2 ml of 0.2 M 2-carbamoyl-2-cyanoethylene-1,1-dithiolate trihydrate in DMF/water (98:2 v/v) for 30 min at room temperature. The reagent was removed and the column rinsed with 10 ml water followed by 10 ml acetonitrile. The oligonucleotide was cleaved and eluted from the solid support by flushing with 1.6 ml of 40% aqueous methylamine over 2 min, collected in a screwcap vial and incubated for 10 min at 55 °C. Subsequently, the base-treated oligonucleotide was dried down in an Eppendorf concentrator to remove methylamine and water. The residue was dissolved in sterile 2'-deprotection buffer (400 µl of 100 mM acetate-TEMED, pH 3.8, for a 0.2 µmol scale synthesis) and incubated for 30 minutes at 60 °C to remove the 2' ACE group. The oligoribonucleotide was precipitated from the acetate-TEMED solution by adding 24 µl 5 M NaCl and 1.2 ml of absolute ethanol.

2'-O-Methyl oligoribonucleotides were synthesized using 5'-DMT, 2'-Omethyl phosphoramidites (Proligo, Hamburg, Germany) on 1 µmol synthesis columns loaded with 3'-aminomodifier (TFA) C7 Icaa control pore glass support (Cherngenes, MA, USA). The aminolinker was added in order to also use the oligonucleotides for conjugation to amino group reactive reagents, such as biotin succinimidyl esters. The synthesis products were deprotected for 16 h at 55 °C in 30% aqueous ammonia and then precipitated by the addition of 12 ml absolute 1-butanol. The full-length product was then gel-purified using a denaturing 20% polyacrylamide gel. 2'-Deoxyoligonucleotides were prepared using 0.2 µmol scale synthesis and standard DNA synthesis reagents (Proligo, Hamburg, Germany).

The sequences of the 2'-O-methyl oligoribonucleotides were 5'-GUCAACAUCAGUCUGAUAAGCUAL (L, 3' aminolinker) for 2'-OMe miR-21, and 5'-AAGGCAAGCUGACCCUGAAGUL for EGFP 2'-OMe antisense, 5'-UGAAGUCCCAGUCGAACGGAAL for EGFP 2'-OMe reverse; the sequence of chimeric 2'-OMe/DNA oligonucleotides was 5'-GTCAACATCAGTCTGATAAGCTAGCGL for 2'-deoxy miR-21 (underlined, 2'-OMe residues), and 5'-AAGGCAAGCTGACCCTGAAGTGCGL for EGFP 2'-deoxy antisense.

The miR-21 cleavage substrate was prepared by PCR-based extension of the partially complementary synthetic DNA oligonucleotides 5'-GAACAATTGCTTTTACAGATGCACATATCGAGGTGAACATCACGTACGTCAACATCA GTCTGATAAGCTATCGGTTGGCAGAAGCTAT and 5'-GGCATAAAGAATTGAAGAGAGTTTTCACTGCATACGACGATTCTGTGATTTGTATTC AGCCCATATCGTTTCATAGCTTCTGCCAACCGA. The extended dsDNA was then used as template for a new PCR with primers 5'- . TAATACGACTCACTATAGAACAATTGCTTTTACAG and 5'-ATTTAGGTGACACTATAGGCATAAAGAATTGAAGA to introduce the T7 and SP6 promoter sequences for in vitro transcription. The PCR product was ligated into pCR2.1-TOPO (Invitrogen). Plasmids isolated from sequence-verified clones were used as templates for PCR to produce sufficient template for run-off in vitro transcription reactions using phage RNA polymerases (Elbashir et al., EMBO 20, 6877-6888 (2001)). ³²P-Cap-labelling was performed as reported (Martinez et al., Cell 110, 563-574 (2002)).

### Plasmids

Plasmids pEGFP-S-21 and pEGFP-A-21 were generated by T4 DNA ligation of preannealed oligodeoxynucleotides 5'-GGCCTCAACATCAGTCTGATAAGCTAGGTACCT and 5'-GGCCAGGTACCTAGCTTATCAGACTGATGTTGA into NotI digested pEGFP-N- 1 (Clontech). The plasmid pHcRed-C1 was from Clontech.

### HeLa extracts and miR-21 quantification

HeLa cell extracts were prepared as described (Dignam et al., Nucleic Acid Res. 11 1475-1489 (1983)). 5x10⁹ cells from HeLa suspension cultures were collected by centrifugation and washed with PBS (pH7.4). The cell pellet (approx. 15 ml) was re-suspended in two times of its volume with 10mM KCl/1.5 mM MgCl₂/0.5 mM dithiothreitol/10mM HEPES-KOH (pH 7.9) and homogenized by douncing. The nuclei were then removed by centrifugation of the cell lysate at 1000 g for 10 min. The supernatant was spun in an ultracentrifuge for 1 h at 10,5000 g to obtain the cytoplasmic S100 extract. The concentration of KCl of the S100 extract was subsequently raised to 100 mM by the addition of 1 M KCl. The extract was then supplemented with 10% glycerol and frozen in liquid nitrogen.

280 µg of total RNA was isolated from 1 ml of S100 extract using the acidic guanidinium thiocyanate-phenol-chloroform extraction method (Chomczynski et al., Anal. Biochem. 162, 156-159 (1987)). A calibration curve for miR-21 Northern signals was produced by loading increasing amounts (10 to 30000 pg) of synthetically made miR-21 (Lim et al. et al., Genes & Devel.17, 991-1008 (2003)). Northern blot analysis was performed as described using 30 µg of total RNA per well (Lagos-Quintana et al., Science 294, 853-858 (2001)).

### In vitro miRNA cleavage and inhibition assay

2'-O-Methyl oligoribonucleotides or 2'-deoxyoligonucleotides were pre-incubated with HeLa S100 at 30 °C for 20 min prior to the addition of the cap-labeled miR-21 target RNA. The concentration of the reaction components were 5 nM target RNA, 1 mM ATP, 0.2 mM GTP, 10 U/ml RNasin (Promega) and 50% HeLa S 100 extract in a final reaction volume of 25 µl. The reaction time was 1.5 h at 30 °C. The reaction was stopped by addition of 200 µl of 300 mM NaCl/25 mM EDTA/20% w/v SDS/200 mM Tris HCl (pH7.5). Subsequently, proteinase K was added to a final concentration of 0.6 mg/ml and the sample was incubated for 15 min at 65 °C. After phenol/chloroform extraction, the RNA was ethanol-precipitated and separated on a 6% denaturing polyacrylamide gel. Radioactivity was detected by phosphorimaging.

### Cell culture and transfection

HeLa S3 and HeLa S3/GFP were grown in 5% CO2 at 37 °C in Dulbecco's modified Eagle's medium (DMEM) supplemented with 10% fetal bovine serum (FBS), 100 unit/ml penicillin, and 100 µg/ml streptomycin. One day before transfection, 105 cells were plated in 500 µl DMEM containing 10% FBS per well of a 24-well plate. Plasmid and plasmid/oligonucleotide transfection was carried out with Lipofectamine2000 (Invitrogen). 0.2 µg pEGFP or its derivatives were cotransfected with 0.3 µg pHcRed with or without 10 pmol of 2'-O-methyl oligoribonucleotide or 10 pmol of 2'-deoxyoligonucleotide per well. Fluorescent cell images were recorded on a Zeiss Axiovert 200 inverted fluorescence microscope (Plan-Apochromat 10x/0.45) equipped with Chroma Technology Corp. filter sets 41001 (EGFP) and , 41002c (HcRed) and AxioVision 3.1 software.

### Example 2: MicroRNA-21 Cleavage of Target RNA

In order to assess the ability of modified oligonucleotides to specifically interfere with miRNA function, we used our previously described mammalian biochemical system developed for assaying RISC activity (Martinez et al., Cell 100, 563-574 (2002)). Zamore and colleagues (Hutvágner et al., Science 297, 2056-2050 (2002)) showed that crude cytoplasmic cell lysates and eIF2C2 immunoprecipitates prepared from these lysates contain let-7 RNPs that specifically cleave let-7-complementary target RNAs. We previously reported that in HeLa cells, numerous miRNAs are expressed including several let-7 miRNA variants (Lagos-Quintana et al., Science 294, 853-858 (2001)).

To assess if other HeLa cell miRNAs are also engaged in RISC like miRNPs we examined the cleavage of a 32P-cap-labelled substrate RNA with a complementary site to the highly expressed miR-21 (Lagos-Quintana et al., Science 294, 853-858 (2001); Mourelatos et al., Genes & Dev.16, 720-728 (2002)). Sequence-specific target RNA degradation was readily observed and appeared to be approximately 2- to 5-fold more effective than cleavage of a similar let-7 target RNA (Figure 2A, lane 1, and data not shown). We therefore decided to interfere with miR-21 guided target RNA cleavage.

### Examples 3: Anti MicroRNA-21 2'-O-methyl Oligoribonucleotide Inhibited MicroRNA-21-Induced Cleavage of Target RNA

A 24-nucleotide 2'-'O-methyl oligoribonucleotide that contained a 3' C7 aminolinker and was complementary to the longest form of the miR-21 was synthesized. The aminolinker was introduced in order to enable post-synthetic conjugation of non-nucleotidic residues such as biotin.

Increasing concentrations of anti miR-21 2'-O-methyl oligoribonucleotide and a control 2'-O-methyl oligoribonucleotide cognate to an EGFP sequence were added to the S100 extract 20 min prior to the addition of 32P-cap-labelled substrate. We determined the concentration of miR-21 in the S 100 extract by quantitative Northern blotting to be 50 pM (Lim et al., Genes & Devel. 17, 991-1008 (2003)).

The control EGFP oligonucleotide did not interfere with miR-21 cleavage even at the highest applied concentration (Figure 2A, lanes 2-3). In contrast, the activity of miR-21 was completely blocked at a concentration of only 3 nM (Figure 2A, lane 5), and a concentration of 0.3 nM showed a substantial 60%-70% reduction of cleavage activity (Figure 2, lane 6)- At a concentration of 0.03 nM, the cleavage activity of miR-21 was not affected when compared to the lysate alone (Figure 2, lane 1, 7).

Antisense 2'-deoxyoligonucleotides (approximately 90% DNA molecules) at concentrations identical to those of 2'-O-methyl oligoribonucleotides, we could not detect blockage of miR-21 induced cleavage (Figure 2A, lanes 8-10). The 2'-deoxynucleotides used in this study were protected against 3'-exonucleases by the addition of three 2'-O-methyl ribonucleotide residues.

### Example 4: Anti MicroRNA-21 2'-O-methyl Oligoribonucleotide Inhibited MicroRN'A-21-Induced Cleavage of Target RNA In Vitro

In order to monitor the activity of miR-21 in HeLa cells, we constructed reporter plasmids that express EGFP mRNA that contains in its 3' UTR a 22-nt sequence complementary to miR-21 (pEGFP-S-21) or in sense orientation to miR-21 (p-EGFP-A-21). Endogenous miRNAs have previously been shown to act like siRNAs by cleaving reporter mRNAs carrying sequences perfectly complementary to miRNA. To monitor transfection efficiency and specific interference with the EGFP indicator plasmids, the far-red fluorescent protein encoding plasmid pHcRed-C1 was cotransfected.

Expression of EGFP was observed in HeLa cells transfected with pEGFP and pEGFP-A-21 (Figure 3, rows 1 and 2)_{;} but not from those transfected with pEGFP-S-21 (Figure 3, row 3). However, expression of EGFP from pEGFP-S-21 was restored upon cotransfection with anti miR-21 2'-O-methyl oligoribonucleotide (Figure 3, row 4). Consistent with our above observation, the 2'-deoxy anti miR-21 oligonucleotide showed no effect (Figure 3, row 5). Similarly, cotransfection of the EGFP 2'-O-methyl oligoribonucleotide in sense orientation with respect to the EGFP mRNA (or antisense to EGFP guide siRNA) had no effect (Figure 3,row 6).

We have demonstrated that miRNP complexes can be effectively and sequence-specifically inhibited with 2'-O-methyl oligoribonucleotides antisense to the guide strand positioned in the RNA silencing .complex.

### Embodiments of the present invention include:

1. An isolated single stranded anti-microRNA molecule comprising a minimum of ten moieties and a maximum of fifty moieties on a molecular backbone, the molecular backbone comprising backbone units, each moiety comprising a base bonded to a backbone unit, each base forming a Watson-Crick base pair with a complementary base wherein:
   at least ten contiguous bases have the same sequence as a sequence of bases in any one of the anti-microRNA molecules shown in Tables 1-4, except that up to thirty percent of the bases pairs may be wobble base pairs, and up to 10% of the contiguous bases may be additions, deletions, mismatches, or combinations thereof;
   no more than fifty percent of the contiguous moieties contain deoxyribonuleotide backbone units;
   the moiety in the molecule at the position corresponding to position 11 of the microRNA is non-complementary; and
   the molecule is capable of inhibiting microRNP activity.
2. A molecule according to embodiment 1, wherein up to 5% of the contiguous moieties are additions, deletions, mismatches, or combinations thereof.
3. A molecule according to embodiment 1, wherein at least one of the moieties is a deoxyribonucleotide.
4. A molecule according to embodiment 3, wherein the deoxyribonucleotide is a modified deoxyribonucleotide moiety.
5. A molecule according to embodiment 4, wherein the modified deoxyribonucleotide is a phosphorothioate deoxyribonucleotide moiety.
6. A molecule according to embodiment 4, wherein the modified deoxyribonucleotide is N'3-N'5 phosphoroamidate deoxyribonucleotide moiety.
7. A molecule according to embodiment 1, wherein at least one of the moieties is a ribonucleotide moiety.
8. A molecule according to embodiment 7, wherein at least one of the moieties is a modified ribonucleotide moiety.
9. A molecule according to embodiment 8, wherein the modified ribonucleotide is substituted at the 2' position.
10. A molecule according to embodiment 9, wherein the substituent at the 2' position is a C₁ to C₄ alkyl group.
11. A molecule according to embodiment 10, wherein the alkyl group is methyl.
12. A molecule according to embodiment 10, wherein the alkyl group is allyl.
13. A molecule according to embodiment 9, wherein the substituent at the 2' position is a C₁ to C₄ alkoxy - C₁ to C₄ alkyl group.
14. A molecule according to embodiment 13, wherein the C₁ to C₄ alkoxy - C₁ to C₄ alkyl group is methoxyethyl.
15. A molecule according to embodiment 8, wherein the modified ribonucleotide has a methylene bridge between the 2'-oxygen atom and the 4'-carbon atom.
16. A molecule according to embodiment 1, wherein at least one of the moieties is a peptide nucleic acid moiety.
17. A molecule according to embodiment 1, wherein at least one of the moieties is a 2'-fluororibonucleotide moiety.
18. A molecule according to embodiment 1, wherein at least one of the moieties is a morpholino phosphoroamidate nucleotide moiety.
19. A molecule according to embodiment 1, wherein at least one of the moieties is a tricyclo nucleotide moiety.
20. A molecule according to embodiment 1, wherein at least one of the moieties is a cyclohexene nucleotide moiety.
21. A molecule according to embodiment 1, wherein the molecule comprises at least one modified moiety for increased nuclease resistance.
22. A molecule according to embodiment 21, wherein the nuclease is an exonuclease.
23. A molecule according to embodiment 22, wherein the molecule comprises at least one modified moiety at the 5' end.
24. A molecule according to embodiment 22, wherein the molecule comprises at least two modified moieties at the 5' end.
25. A molecule according to embodiment 22, wherein the molecule comprises at least one modified moiety at the 3' end.
26. A molecule according to embodiment 22, wherein the molecule comprises at least two modified moieties at the 3' end.
27. A molecule according to embodiment 22, wherein the molecule comprises at least one modified moiety at the 5' end and at least one modified moiety at the 3'end.
28. A molecule according to embodiment 22, wherein the molecule comprises at least two modified moieties at the 5' end and at least two modified moieties at the 3'end.
29. A molecule according to embodiment 22, wherein the molecule comprises a nucleotide cap at the 5' end, the 3' end or both.
30. A molecule according to embodiment 22, wherein the molecule comprises an ethylene glycol compound and/or amino linkers at the 5' end, the 3' end, or both.
31. A molecule according to embodiment 1, wherein the nuclease is an endonuclease.
32. A molecule according to embodiment 31, wherein the molecule comprises at least one modified moiety between the 5' and 3' end.
33. A molecule according to embodiment 31, wherein the molecule comprises an ethylene glycol compound and/or amino linker between the 5' end and 3' end.
34. A molecule according to embodiment 1, wherein all of the moieties are nuclease resistant.
35. A method for inhibiting microRNP activity in a cell, the microRNP comprising a microRNA molecule, the microRNA molecule comprising a sequences of bases complementary of the sequence of bases in a single stranded anti-microRNA molecule, the method comprising introducing into the cell the single-stranded anti-microRNA molecule comprising a sequence of a minimum of ten moieties and a maximum of fifty moieties on a molecular backbone, the molecular backbone comprising backbone units, each moiety comprising a base bonded to a backbone unit, each base forming a Watson-Crick base pair with a complementary base, wherein:
   at least ten contiguous bases of the anti-microRNA molecule are complementary to the microRNA, except that up to thirty percent of the bases may be substituted by wobble base pairs, and up to ten percent of the at least ten moieties are addition, deletions, mismatches, or combinations thereof;
   no more than fifty percent of the contiguous moieties contain deoxyribonuleotide backbone units; and
   the moiety in the molecule at the position corresponding to position 11 of the microRNA is non-complementary.
36. A method according to embodiment 35, wherein the anti-microRNA is a human anti-microRNA.
37. A method according to embodiment 35, wherein the anti-microRNA is a mouse anti-microRNA.
38. A method according to embodiment 35, wherein the anti-microRNA is a rat anti-microRNA.
39. A method according to embodiment 35, wherein the ant-microRNA is a drosophila microRNA.
40. A method according to embodiment 35, wherein the anti-microRNA is a C. elegans microRNA.
41. An isolated microRNA molecule comprising a minimum of ten moieties and a maximum of fifty moieties on a molecular backbone, the molecular backbone comprising backbone units, each moiety comprising a base bonded to a backbone unit wherein:
   at least ten contiguous bases have the same sequence as a sequence of bases in any one of the microRNA molecules shown in Table 2, except that up to thirty percent of the bases pairs may be wobble base pairs, and up to 10% of the contiguous bases are additions, deletions, mismatches, or combinations thereof; and
   no more than fifty percent of the contiguous moieties contain deoxyribonuleotide backbone units.
42. A molecule according to embodiment 41 having the sequence shown in Table 2.
43. A molecule according to embodiment 41, wherein the molecule is modified for increased nuclease resistance.
44. A molecule according to embodiment 41, wherein the moiety at position 11 is an addition , deletion or substitution.
45. An isolated microRNA molecule comprising a minimum of ten moieties and a maximum of fifty moieties on a molecular backbone, the molecular backbone comprising backbone units, each moiety comprising a base bonded to a backbone unit wherein:
   at least ten contiguous bases have any one of the microRNA sequences shown in Tables 1, 3 and 4, except that up to thirty percent of the bases pairs may be wobble base pairs, and up to 10% of the contiguous bases are additions, deletions, mismatches, or combinations thereof;
   no more than fifty percent of the contiguous moieties contain deoxyribonuleotide backbone units; and
   is modified for increased nuclease resistance.
46. A molecule according to embodiment 45, wherein the molecule is modified for increased nuclease resistance.
47. A molecule according to embodiment 45, wherein the moiety at position 11 is an addition, deletion, or substitution.
48. An isolated single stranded anti-microRNA molecule comprising a minimum of ten moieties and a maximum of fifty moieties on a molecular backbone, the molecular backbone comprising backbone units, each moiety comprising a base bonded to a backbone unit, each base forming a Watson-Crick base pair with a complementary base wherein:
   at least ten contiguous bases have the same sequence as a sequence of bases in any one of the anti-microRNA molecules shown in Tables 1-4, except that up to thirty percent of the bases pairs may be wobble base pairs, and up to 10% of the contiguous bases may be additions, deletions, mismatches, or combinations thereof;
   no more than fifty percent of the contiguous moieties contain deoxyribonuleotide backbone units; and
   the molecule is capable of inhibiting microRNP activity.
49. A method for inhibiting microRNP activity in a cell, the microRNP comprising a microRNA molecule, the microRNA molecule comprising a sequences of bases complementary of the sequence of bases in a single stranded anti-microRNA molecule, the method comprising introducing into the cell the single-stranded anti-microRNA molecule comprising a sequence of a minimum of ten moieties and a maximum of fifty moieties on a molecular backbone, the molecular backbone comprising backbone units, each moiety comprising a base bonded to a backbone unit, each base forming a Watson-Crick base pair with a complementary base, wherein:
   at least ten contiguous bases of the anti-microRNA molecule are complementary to the microRNA, except that up to thirty percent of the bases may be substituted by wobble base pairs, and up to ten percent of the at least ten moieties may be additions, deletions, mismatches, or combinations thereof; and
   no more than fifty percent of the contiguous moieties contain deoxyribonuleotide backbone units.

## Claims

1. An isolated single stranded anti-microRNA molecule comprising the sequence of SEQ ID NO: 445 and having a maximum of fifty moieties .

2. A molecule according to claim 1, wherein at least one of the moieties is a deoxyribonucleotide, a ribonucleotide moiety, a peptide nucleic acid moiety, a 2'-fluororibonucleotide moiety, a morpholino phosphoroamidate nucleotide moiety, a tricycle nucleotide moiety, a cyclohexene moiety, or a moiety modified for increased nuclease resistance.

3. A molecule according to claim 3, wherein the deoxyribonucleotide is a modified deoxyribonucleotide moiety, such as a phosphorothioate deoxyribonucleotide moiety or a N'3-N'5 phosphoroamidate deoxyribonucleotide moiety.

4. A molecule according to claim 2, wherein at least one of the moieties is a modified ribonucleotide moiety.

5. A molecule according to claim 4, wherein the modified ribonucleotide is substituted at the 2' position.

6. A molecule according to claim 5, wherein the substituent at the 2' position is a C₁ to C₄ alkyl group, preferably methyl or allyl.

7. A molecule according to claim 5, wherein the substituent at the 2' position is a C₁ to C₄ alkoxy - C₁ to C₄ alkyl group, preferably methoxyethyl.

8. A molecule according to claim 4, wherein the modified ribonucleotide has a methylene bridge between the 2'-oxygen atom and the 4'-carbon atom.

9. A molecule according to claim 2, wherein at least one of the moieties is a moiety modified for increased nuclease resistance, and wherein the nuclease is an exonuclease.

10. A molecule according to claim 9, wherein the molecule comprises
(a) at least one modified moiety at the 5' end;
(b) at least two modified moieties at the 5' end;
(c) at least one modified moiety at the 3' end;
(d) at least two modified moieties at the 3' end;
(e) at least one modified moiety at the 5' end and at least one modified moiety at the 3'end;
(f) at least two modified moieties at the 5' end and at least two modified moieties at the 3'end;
(g) a nucleotide cap at the 5' end, the 3' end or both.
(h) an ethylene glycol compound and/or amino linkers at the 5' end, the 3' end, or both.

11. A molecule according to claim 2, wherein at least one of the moieties is a moiety modified for increased nuclease resistance, and wherein the nuclease is an endonuclease.

12. A molecule according to claim 11, wherein the molecule comprises at least one modified moiety between the 5' and 3' end and/or an ethylene glycol compound and/or amino linker between the 5' end and 3' end.

13. A molecule according to claim 1, wherein all of the moieties are nuclease resistant.

14. An *in vitro* method for inhibiting microRNP activity in a cell, the microRNP comprising a microRNA molecule, the microRNA molecule comprising a sequences of bases complementary to the sequence of bases in a single stranded anti-microRNA molecule, the method comprising introducing into the cell the single-stranded anti-microRNA molecule of claim 1.

15. A single stranded anti-microRNA molecule for use in inhibiting microRNP activity in a cell, the microRNP comprising a microRNA molecule, the microRNA molecule comprising a sequence of bases complementary to the sequence of bases in the single stranded anti-microRNA molecule of claim 1.

16. An isolated microRNA molecule comprising the sequence of SEQ ID NO: 139 and having a maximum of fifty moieties.

17. A molecule according to claim 16, wherein the molecule is modified for increased nuclease resistance.
